(19) Europäisches Patentamt
European Patent Office
Office européen des brevets

(11) **EP 4 431 115 A1**

(12) **EUROPEAN PATENT APPLICATION**
published in accordance with Art. 153(4) EPC

(43) Date of publication:
**18.09.2024 Bulletin 2024/38**

(21) Application number: **23780293.9**

(22) Date of filing: **27.03.2023**

(51) International Patent Classification (IPC):
***A61K 47/34** (2017.01)* ***A61K 47/44** (2017.01)*
***A61K 38/08** (2019.01)* ***G01N 33/15** (2006.01)*
***G01N 33/50** (2006.01)*

(52) Cooperative Patent Classification (CPC):
**A61K 38/08; A61K 47/34; A61K 47/44; G01N 33/15; G01N 33/50**

(86) International application number:
**PCT/JP2023/012087**

(87) International publication number:
**WO 2023/190283 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.03.2022 JP 2022051944**

(71) Applicant: **CHUGAI SEIYAKU KABUSHIKI KAISHA**
**Tokyo 115-8543 (JP)**

(72) Inventors:
- **TAKANASHI, Kenji**
  **Gotemba-shi, Shizuoka 412-8513 (JP)**
- **TAKANO, Ryusuke**
  **Gotemba-shi, Shizuoka 412-8513 (JP)**
- **TANIDA, Satoshi**
  **Gotemba-shi, Shizuoka 412-8513 (JP)**
- **NAKAE, Shinichi**
  **Gotemba-shi, Shizuoka 412-8513 (JP)**
- **IIKURA, Hitoshi**
  **Gotemba-shi, Shizuoka 412-8513 (JP)**
- **NISHIMURA, Yoshikazu**
  **Gotemba-shi, Shizuoka 412-8513 (JP)**
- **KURAMOTO, Shino**
  **Kamakura-shi, Kanagawa 247-8530 (JP)**

(74) Representative: **Vossius & Partner**
**Patentanwälte Rechtsanwälte mbB**
**Siebertstrasse 3**
**81675 München (DE)**

(54) **COMPOUND PHARMACEUTICAL PREPARATION CONTAINING OIL COMPONENT COMPRISING POLYOXYETHYLENE STRUCTURE**

(57) The present invention relates to a formulation comprising a compound and an oily component having a polyoxyethylene structure, wherein the content of the oily component having a polyoxyethylene structure is 2.0% by volume or more and 7.5% by volume or less based on 100% by volume of the formulation.

***Fig.1***

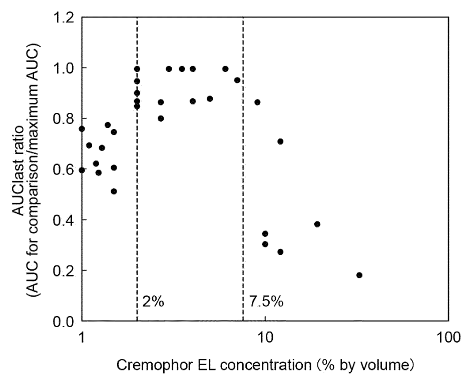


EP 4 431 115 A1

## Description

### Technical Field

The present invention relates to a compound formulation comprising an oily component having a polyoxyethylene structure. The present invention also relates to a method for screening a candidate compound using the formulation.

### Background Art

It is generally believed that compounds having a molecular weight of 500 or more have low membrane permeability when administered orally, posing a problem in oral absorbability (for example, Non Patent Literatures 1 and 2). Also, compounds whose solubility and membrane permeability are both low tend to have low oral absorbability. In many cases, these compounds do not have sufficient absorbability even when the dosage is increased, and proper drug efficacy evaluation and safety evaluation are not possible. For this reason, the selection of compounds having such characteristics has been avoided as much as possible at the screening stage in pharmaceutical development.
Meanwhile, when a compound with low oral absorbability is selected as a candidate compound for a pharmaceutical, efforts have been made to enhance oral absorbability through formulation technology or drug delivery technology (for example, Non Patent Literature 3).

### Citation List

### Non Patent Literature

Non Patent Literature 1: Donovan, M. D. et al., Pharm. Res., 1990, 7, pp. 863-868.
Non Patent Literature 2: Lipinski, C. A., Adv. Drug Del. Rev. 1997, 23, pp3-25. (Lipinski, rule of 5)
Non Patent Literature 3: Ghadi, R. et al., J. Control. Release, 2017, 248, pp. 71-95.

### Summary of Invention

### Technical Problem

However, the optimal additive and/or prescription for each compound may vary. In an early stage of screening for a candidate compound where there are many test compounds, it is not practical to select the optimal additive and/or prescription, etc. for each test compound and administer the test compounds each with a different additive and/or prescription, since it is necessary to optimize the respective different additives and/or prescriptions depending on the number of test compounds.
Also, in the case where an animal study by oral administration is performed in an early stage of screening for a medicament, evaluation of test compounds is made with formulations prepared by using a certain common additive, but depending on the compatibility between the test compounds and the additive, sufficient oral absorbability may not be obtained and the test compounds may not be evaluated properly. As a result, the process of screening a candidate compound was subject to chance elements, and there has been a possibility that an originally useful test compound could be overlooked as a false negative.
The present invention has been made in view of such circumstances, and an object thereof is to provide a formulation with oral absorbability enhanced in a versatile manner in order to enable evaluation of compounds under the same formulation conditions. Another object of the present invention is to provide a method for screening a candidate compound using the formulation.

### Solution to Problem

One aspect of the present invention relates to a formulation. Specifically, for example, the present invention encompasses each of the following inventions.

[A1] A formulation comprising:
a compound and an oily component having a polyoxyethylene structure, wherein the content of the oily component is 2.0% by volume or more and 7.5% by volume or less based on 100% by volume of the formulation.
[A2] An agent for promoting absorption of a compound, comprising:

an oily component having a polyoxyethylene structure as an active component,
wherein the agent is used such that the content of the oily component is 2.0% by volume or more and 7.5% by volume or less based on 100% by volume of a formulation containing the compound.

[A3] An agent for improving solubility of a compound, comprising:

an oily component having a polyoxyethylene structure as an active component,
wherein the agent is used such that the content of the oily component is 2.0% by volume or more and 7.5% by volume or less based on 100% by volume of a formulation containing the compound.

[A4] The formulation or agent according to any of [A1] to [A3], wherein the compound has a ClogP of 4 or more and 25 or less.
[A5] The formulation or agent according to any of [A1] to [A4], wherein the compound has a ClogP of 8 or more and 21 or less.
[A6] The formulation or agent according to any of [A1] to [A5], wherein the compound has a ClogP of 9 or more and 20 or less.
[A7] The formulation or agent according to any of [A1] to [A6], wherein the compound has a ClogP of 13 or more and 17 or less.
[A8] The formulation or agent according to any of [A1] to [A7], wherein the compound has a ClogP of 13 or more.
[A9] The formulation or agent according to any of [A1] to [A8], wherein the compound has a ClogP more than or equal to the ClogP of the compound 2.
[A10] The formulation or agent according to any of [A1] to [A9], wherein the compound has a ClogP more than or equal to the ClogP of the compound 2 and less than or equal to the ClogP of the compound 1.
[A11] The formulation or agent according to any of [A1] to [A10], wherein the compound has a value of Caco-2 Papp (cm/sec) of 1.0E-9 or more.
[A12] The formulation or agent according to any of [A1] to [A11], wherein the compound has a value of Caco-2 Papp (cm/sec) of 1.0E-7 or more.
[A13] The formulation or agent according to any of [A1] to [A12], wherein the compound has a value of Caco-2 Papp (cm/sec) of 1.0E-4 or less.
[A14] The formulation or agent according to any of [A1] to [A13], wherein the compound has a value of Caco-2 Papp (cm/sec) of 1.0E-5 or less.
[A15] The formulation or agent according to any of [A1] to [A14], wherein the compound has a value of Caco-2 Papp (cm/sec) of 6.0E-6 or less.
[A16] The formulation or agent according to any of [A1] to [A15], wherein the compound has a value of Caco-2 Papp (cm/sec) of 1.0E-9 or more and 1.0E-4 or less.
[A17] The formulation or agent according to any of [A1] to [A16], wherein the compound has a value of Caco-2 Papp (cm/sec) of 1.0E-8 or more and 1.0E-5 or less.
[A18] The formulation or agent according to any of [A1] to [A17], wherein the compound has a value of Caco-2 Papp (cm/sec) of 1.0E-7 or more and 1.0E-5 or less.
[A19] The formulation or agent according to any of [A1] to [A18], wherein the compound has a value of Caco-2 Papp (cm/sec) more than or equal to the value of Papp (cm/sec) of the compound 9.
[A20] The formulation or agent according to any of [A1] to [A19], wherein the compound has a value of Caco-2 Papp (cm/sec) less than or equal to the value of Papp (cm/sec) of the compound 2.
[A21] The formulation or agent according to any of [A1] to [A20], wherein the compound has a value of Caco-2 Papp (cm/sec) more than or equal to the value of Papp (cm/sec) of the compound 9 and less than or equal to the value of Papp (cm/sec) of the compound 2.
[A22] The formulation or agent according to any of [A11] to [A21], wherein the value of Caco-2 Papp (cm/sec) is calculated by membrane permeability evaluation under the following conditions:

(a) Caco-2 cells are cultured on 96-well Transwell for 3 weeks, then a compound to be evaluated, a DMEM medium, and FaSSIF (1% DMSO) are added to the Apical side, a DMEM medium is added to the Basal side, and the cells are preincubated under a 5% $CO_2$ atmosphere at 37°C with shaking at 80 rpm for 24 hours;
(b) after the preincubation, the preincubation solutions on the Apical side and Basal side are sucked off and washed, and a FaSSIF/HBSS buffer solution (1% DMSO) (pH 6.0) containing the compound is added to the Apical side, and a HBSS buffer solution (4% BSA) (pH 7.4) is added to the Basal side;
(c) each well is shaken under a 5% $CO_2$ atmosphere at 37°C and 80 rpm, and at 180 minutes after the start of shaking, a sample on the Basal side is collected, and the permeation amount of the compound is measured by liquid chromatography-mass spectrometry (LC/MS); and

(d) from the measured permeation amount, the permeability coefficient (Caco-2 Papp (cm/sec)) is calculated.

[A23] The formulation or agent according to any of [A1] to [A22], wherein the compound has a solubility of 10 mg/mL or less in a 50 mM phosphate buffer solution (pH 6.5) at 25°C.
[A24] The formulation or agent according to any of [A1] to [A23], wherein the compound has a solubility of 5 mg/mL or less in a 50 mM phosphate buffer solution (pH 6.5) at 25°C.
[A25] The formulation or agent according to any of [A1] to [A24], wherein the compound has a solubility of 2.5 mg/mL or less in a 50 mM phosphate buffer solution (pH 6.5) at 25°C.
[A26] The formulation or agent according to any of [A1] to [A25], wherein the compound has a solubility of 2 mg/mL or less in a 50 mM phosphate buffer solution (pH 6.5) at 25°C.
[A27] The formulation or agent according to any of [A1] to [A26], wherein the compound has a molecular weight of 5000 or less.
[A28] The formulation or agent according to any of [A1] to [A27], wherein the compound has a molecular weight of 2000 or less.
[A29] The formulation or agent according to any of [A1] to [A28], wherein the compound has a molecular weight of 500 or more.
[A30] The formulation or agent according to any of [A1] to [A29], wherein the compound has a molecular weight of 1000 or more.
[A31] The formulation or agent according to any of [A1] to [A30], wherein the compound has a molecular weight of 1100 or more.
[A32] The formulation or agent according to any of [A1] to [A31], wherein the compound has a molecular weight of 1200 or more.
[A33] The formulation or agent according to any of [A1] to [A32], wherein the compound has the following features (i) and (ii):

(i) the compound has a ClogP of 9 or more; and
(ii) the compound has a value of Caco-2 Papp (cm/sec) of 1.0E-5 or less.

[A34] The formulation or agent according to any of [A1] to [A32], wherein the compound has the following features (i) and (ii):

(i) the compound has a ClogP of 10 or more; and
(ii) the compound has a value of Caco-2 Papp (cm/sec) of 1.0E-7 or more and 1.0E-5 or less.

[A35] The formulation or agent according to any of [A1] to [A32], wherein the compound has the following features (i), (ii), and (iii):

(i) the compound has a ClogP of 13 or more;
(ii) the compound has a value of Caco-2 Papp (cm/sec) of 1.4E-7 or more and 6.0E-6 or less; and
(iii) the compound has a molecular weight of 1000 or more.

[A36] The formulation or agent according to any of [A1] to [A35], wherein the compound is a peptide compound.
[A37] The formulation or agent according to any of [A1] to [A36], wherein the compound is a peptide compound containing a non-natural amino acid residue.
[A38] The formulation or agent according to any of [A1] to [A37], wherein the compound is a peptide compound containing 5 or more and 30 or less amino acid residues.
[A39] The formulation or agent according to any of [A1] to [A38], wherein the compound is a peptide compound containing 7 or more and 25 or less amino acid residues.
[A40] The formulation or agent according to any of [A1] to [A39], wherein the compound is a peptide compound containing 8 or more and 15 or less amino acid residues.
[A41] The formulation or agent according to any of [A1] to [A40], wherein the compound is a peptide compound containing 9 or more and 13 or less amino acid residues.
[A42] The formulation or agent according to any of [A1] to [A41], wherein the compound is a peptide compound containing 11 amino acid residues.
[A43] The formulation or agent according to any of [A36] to [A42], wherein the peptide compound contains 2 or more N-substituted amino acid residues.
[A44] The formulation or agent according to any of [A36] to [A43], wherein the peptide compound contains 3 or more N-substituted amino acid residues.

[A45] The formulation or agent according to any of [A36] to [A44], wherein the peptide compound contains 4 or more N-substituted amino acid residues.

[A46] The formulation or agent according to any of [A36] to [A45], wherein the peptide compound contains 5 or more N-substituted amino acid residues.

[A47] The formulation or agent according to any of [A43] to [A46], wherein the N-substituted amino acid residue is a N-$C_1$-$C_6$ alkylamino acid residue.

[A48] The formulation or agent according to any of [A43] to [A47], wherein the N-substituted amino acid residue is a N-methylamino acid or N-ethylamino acid residue.

[A49] The formulation or agent according to any of [A43] to [A48], wherein the N-substituted amino acid residue is a N-methylamino acid residue.

[A50] The formulation or agent according to any of [A36] to [A49], wherein the peptide compound is a peptide compound having a cyclic moiety.

[A51] The formulation or agent according to [A50], wherein the number of amino acid residues constituting the cyclic moiety of the peptide compound having a cyclic moiety is 5 or more and 15 or less.

[A52] The formulation or agent according to [A50] or [A51], wherein the number of amino acid residues constituting the cyclic moiety of the peptide compound having a cyclic moiety is 6 or more and 14 or less.

[A53] The formulation or agent according to any of [A50] to [A52], wherein the number of amino acid residues constituting the cyclic moiety of the peptide compound having a cyclic moiety is 7 or more and 14 or less.

[A54] The formulation or agent according to any of [A50] to [A53], wherein the number of amino acid residues constituting the cyclic moiety of the peptide compound having a cyclic moiety is 8 or more and 12 or less.

[A55] The formulation or agent according to any of [A50] to [A54], wherein the number of amino acid residues constituting the cyclic moiety of the peptide compound having a cyclic moiety is 9 or more and 11 or less.

[A56] The formulation or agent according to any of [A50] to [A55], wherein the number of amino acid residues constituting the cyclic moiety of the peptide compound having a cyclic moiety is 11.

[A57] The formulation or agent according to any of [A50] to [A56], wherein the number of ring atoms of the peptide compound having a cyclic moiety is 34 or more and 46 or less.

[A58] The formulation or agent according to any of [A50] to [A57], wherein the number of ring atoms of the peptide compound having a cyclic moiety is 34 or more and 43 or less.

[A59] The formulation or agent according to any of [A50] to [A58], wherein the number of ring atoms of the peptide compound having a cyclic moiety is 34 or more and 40 or less.

[A60] The formulation or agent according to any of [A50] to [A59], wherein the number of ring atoms of the peptide compound having a cyclic moiety is 34 or more and 37 or less.

[A61] The formulation or agent according to any of [A50] to [A60], wherein the number of ring atoms of the peptide compound having a cyclic moiety is 34 or more and 36 or less.

[A62] The formulation or agent according to any of [A50] to [A61], wherein the number of ring atoms of the peptide compound having a cyclic moiety is 34.

[A63] The formulation or agent according to any of [A1] to [A62], wherein the compound is a peptide compound having a cyclic moiety, and
the peptide compound having a cyclic moiety has the following features (i) and (ii):

(i) the peptide compound has a cyclic moiety composed of 5 or more and 12 or less amino acid residues, and the number of amino acid residues is 9 or more and 13 or less; and
(ii) the peptide compound contains at least 2 N-substituted amino acid residues and at least 1 non-N-substituted amino acid residue.

[A64] The formulation or agent according to any of [A1] to [A62], wherein the compound is a peptide compound having a cyclic moiety, and
the peptide compound having a cyclic moiety has the following features (i) and (ii):

(i) the peptide compound has a cyclic moiety composed of 9 or more and 12 or less amino acid residues, and the number of amino acid residues is 9 or more and 13 or less; and
(ii) the peptide compound contains at least 3 N-substituted amino acid residues and at least 1 non-N-substituted amino acid residue.

[A65] The formulation or agent according to any of [A1] to [A62], wherein the compound is a peptide compound having a cyclic moiety, and
the peptide compound having a cyclic moiety has the following features (i) and (ii):

(i) the peptide compound has a cyclic moiety composed of 11 amino acid residues, and the number of amino acid residues is 11; and
(ii) the peptide compound contains at least 4 N-substituted amino acid residues and at least 1 non-N-substituted amino acid residue.

[A66] The formulation or agent according to any of [A1] to [A62], wherein the compound is a peptide compound having a cyclic moiety, and
the peptide compound having a cyclic moiety has the following features (i), (ii), (iii), and (iv):

(i) the peptide compound has a cyclic moiety composed of 5 or more and 12 or less amino acid residues, and the number of amino acid residues is 9 or more and 13 or less;
(ii) the peptide compound contains at least 2 N-substituted amino acid residues and at least 1 non-N-substituted amino acid residue;
(iii) the peptide compound has a ClogP of 9 or more; and
(iv) the peptide compound has a value of Caco-2 Papp (cm/sec) of 1.0E-5 or less.

[A67] The formulation or agent according to any of [A1] to [A62], wherein the compound is a peptide compound having a cyclic moiety, and
the peptide compound having a cyclic moiety has the following features (i), (ii), (iii), and (iv):

(i) the peptide compound has a cyclic moiety composed of 9 or more and 12 or less amino acid residues, and the number of amino acid residues is 9 or more and 13 or less;
(ii) the peptide compound contains at least 3 N-substituted amino acid residues and at least 1 non-N-substituted amino acid residue;
(iii) the peptide compound has a ClogP of 9 or more; and
(iv) the peptide compound has a value of Caco-2 Papp (cm/sec) of 1.0E-5 or less.

[A68] The formulation or agent according to any of [A1] to [A62], wherein the compound is a peptide compound having a cyclic moiety, and
the peptide compound having a cyclic moiety has the following features (i), (ii), (iii), and (iv):

(i) the peptide compound has a cyclic moiety composed of 11 amino acid residues, and the number of amino acid residues is 11;
(ii) the peptide compound contains at least 4 N-substituted amino acid residues and at least 1 non-N-substituted amino acid residue;
(iii) the peptide compound has a ClogP of 11 or more; and
(iv) the peptide compound has a value of Caco-2 Papp (cm/sec) of 6.0E-6 or less.

[A69] The formulation or agent according to any of [A1] to [A62], wherein the compound is a peptide compound having a cyclic moiety, and
the peptide compound having a cyclic moiety has the following features (i) and (ii):

(i) the number of amino acid residues is 9 or more and 13 or less, and the number of ring atoms of the cyclic moiety is 34; and
(ii) the peptide compound contains at least 2 N-substituted amino acid residues and at least 1 non-N-substituted amino acid residue.

[A70] The formulation or agent according to any of [A1] to [A62], wherein the compound is a peptide compound having a cyclic moiety, and
the peptide compound having a cyclic moiety has the following features (i), (ii), (iii), (iv), and (v):

(i) the number of amino acid residues is 9 or more and 13 or less, and the number of ring atoms of the cyclic moiety is 34;
(ii) the peptide compound contains at least 2 N-substituted amino acid residues and at least 1 non-N-substituted amino acid residue;
(iii) the peptide compound has a ClogP of 9 or more;
(iv) the peptide compound has a value of Caco-2 Papp (cm/sec) of 1.0E-5 or less; and
(v) the peptide compound has a molecular weight of 1000 or more.

[A71] The formulation or agent according to any of [A1] to [A62], wherein the compound is a peptide compound having a cyclic moiety, and
the peptide compound having a cyclic moiety has the following features (i), (ii), (iii), (iv), and (v):

(i) the number of amino acid residues is 11, and the number of ring atoms of the cyclic moiety is 34;
(ii) the peptide compound contains at least 4 N-substituted amino acid residues and at least 1 non-N-substituted amino acid residue;
(iii) the peptide compound has a ClogP of 13 or more;
(iv) the peptide compound has a value of Caco-2 Papp (cm/sec) of 1.4E-7 or more and 6.0E-6 or less; and
(v) the peptide compound has a molecular weight of 1000 or more.

[A72] The formulation or agent according to any of [A36] to [A71], wherein the peptide compound has a ClogP/number of amino acid residues of 1.0 or more.
[A73] The formulation or agent according to any of [A36] to [A72], wherein the peptide compound has a ClogP/number of amino acid residues of 1.0 or more and 1.8 or less.
[A74] The formulation or agent according to any of [A36] to [A73], wherein the peptide compound has a ClogP/number of amino acid residues of 1.1 or more and 1.6 or less.
[A75] The formulation or agent according to any of [A1] to [A74], which is a liquid formulation.
[A76] The formulation or agent according to any of [A1] to [A75], further comprising dimethyl sulfoxide (DMSO).
[A77] The formulation or agent according to [A76], wherein the content of the DMSO is 1% by volume or more and 30% by volume or less based on 100% by volume of the formulation.
[A78] The formulation or agent according to [A76] or [A77], wherein the content of the DMSO is 5% by volume or more and 20% by volume or less based on 100% by volume of the formulation.
[A79] The formulation or agent according to any of [A76] to [A78], wherein the content of the DMSO is 10% by volume based on 100% by volume of the formulation.
[A80] The formulation or agent according to any of [A1] to [A79], wherein the oily component having a polyoxyethylene structure is at least one selected from the group consisting of polyoxyethylene castor oil, polyoxyethylene hydrogenated castor oil, and polyoxyethylene sorbitan fatty acid esters.
[A81] The formulation or agent according to any of [A1] to [A80], wherein the oily component having a polyoxyethylene structure is polyoxyethylene castor oil.
[A82] The formulation or agent according to any of [A1] to [A81], wherein the oily component having a polyoxyethylene structure has an average number of moles of ethylene oxide added of 10 or more and 90 or less.
[A83] The formulation or agent according to any of [A1] to [A82], wherein the oily component having a polyoxyethylene structure has an average number of moles of ethylene oxide added of 15 or more and 60 or less.
[A84] The formulation or agent according to any of [A1] to [A83], wherein the oily component having a polyoxyethylene structure has an average number of moles of ethylene oxide added of 20 or more and 50 or less.
[A85] The formulation or agent according to any of [A1] to [A84], wherein the oily component having a polyoxyethylene structure has an average number of moles of ethylene oxide added of 25 or more and 40 or less.
[A86] The formulation or agent according to any of [A1] to [A85], wherein the oily component having a polyoxyethylene structure has an average number of moles of ethylene oxide added of 30 or more and 39 or less.
[A87] The formulation or agent according to any of [A1] to [A86], wherein the oily component having a polyoxyethylene structure has an average number of moles of ethylene oxide added of 35.
[A88] The formulation or agent according to any of [A1] to [A87], wherein the components contained in the formulation are substantially composed of water, the compound, the oily component having a polyoxyethylene structure, and the DMSO.
[A89] The formulation or agent according to any of [A1] to [A88], wherein the formulation does not comprise a surfactant other than the oily component having a polyoxyethylene structure.
[A90] The formulation or agent according to any of [A1] to [A89], wherein the formulation does not comprise lauroyl-L-carnitine.
[A91] The formulation or agent according to any of [A1] to [A90], wherein cyclosporin and analogues thereof are not included in the compound.
[A92] The formulation or agent according to any of [A1] to [A91], wherein dihydrocyclosporin D is not included in the compound.
[A93] The formulation or agent according to any of [A1] to [A92], wherein (5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2, 1-u]pyrrolo[2,1-i][1,4,7,10,13,16,19,22,25,28,31]undecaazacyclotetratriacontine-21,1'-cyclopentane]-15-carboxamide, (5S,8S,11S,15S,18S,23aS,25R,29S,35S,37aS)-8-((S)-sec-butyl)-35-(cyclohexylmethyl)-18-cyclopentyl-29-(3,5-difluoro-4-(trifluoromethyl)phenethyl)-25-ethoxy-11-isobutyl-N,N,5,6,12,16,19,33,36-nonamethyl-4,7,10,13,17,20,23,28,31,34,37-undecaoxotetratri acontahydro-2H,4H-spiro[azeto[2,1 -u]pyrrolo[2,1 - i][1,4,7,10,13,16,19,22,25,28,31]undecaazacyclotetratriacontine-21,1'-cyclopentane]-15-carboxamide, (3S,9S,18S,21S,25S,28S,34S)-3-[2-[3-chloro-4-(trifluoromethyl)phenyl]ethyl]-28-cyclohexyl-9-(cyclohexylmethyl)-21-isobutyl-7,10,13,16,22,26,29-heptamethyl-18-[(1S)-1-methylpropyl]-25-(piperidine-1-carbonyl)spiro[1,4,7,10,13,16,19,22,26,29,32-undecazabicyclo[32.3.0]heptatriacontane-31, 1 '-cyclopentane]-2,5,8, 11, 14, 17,20,23,27,30,33-undecaone, (5S,8S, 11 S, 15S, 18S,23aS,29S,35S,37aS)-8-((S)-sec-butyl)-29-(3-chloro-4-(trifluoromethyl)phenethyl)-35-(cyclohexylmethyl)-18-cyclopentyl-11-isobutyl-5,6,12,16,19,33,36-heptamethyl-15-(morpholine-4-carbonyl)docosahydro-2H,4H-spiro[azeto[2,1-u]pyrrolo[2,1-i][1,4,7,10,13,16,19,22,25,28,31]undecaazacyclotetratriacontine-21,1'-cyclopentane]-4,7,10,13,17,20,23,28,31,34,37(14H,22H)-undecaone, and (5S,8S,11S,15S,18S,23aS,29S,35S,37aS)-8-((S)-sec-butyl)-29-(3-chloro-4-(trifluoromethyl)phenethyl)-18-cyclopentyl-36-ethyl-11-isobutyl-5,6,12,16,19,33-hexamethyl-35-(4-methylbenzyl)-15-(morpholine-4-carbonyl)docosahydro-2H,4H-spiro[azeto[2,1-u]pyrrolo[2,1-i][1,4,7,10,13,16,19,22,25,28,31]undecaazacyclotetratriacontine-21,1'-cyclopentane]-4,7,10,13,17,20,23,28,31,34,37(14H,22H)-undecaone are not included in the compound.

[A94] The formulation or agent according to any of [A1] to [A93], wherein the formulation provides a promoted absorption of the compound compared to the formulation not comprising the oily component having a polyoxyethylene structure.

[A95] The formulation or agent according to any of [A1] to [A94], wherein the formulation is an oral formulation.

[A96] The formulation or agent according to any of [A1] to [A95], wherein the formulation is for screening a candidate compound.

[A97] The formulation or agent according to any of [A1] to [A96], wherein the content of the oily component is 2.0% by volume or more and less than 7.5% by volume based on 100% by volume of the formulation.

[A98] The formulation or agent according to any of [A1] to [A96], wherein the content of the oily component is 2.0% by volume or more and 7.0% by volume or less based on 100% by volume of the formulation.

[A99] The formulation or agent according to any of [A1] to [A96], wherein the content of the oily component is 2.0% by volume or more and 5.0% by volume or less based on 100% by volume of the formulation.

One aspect of the present invention relates to a method for screening a candidate compound. Specifically, for example, the present invention encompasses each of the following inventions.

[B1] A method for screening a candidate compound, comprising:
administering to a subject the formulation according to any of [A1] to [A99].

[B2] The method according to [B 1], comprising selecting the candidate compound using at least one selected from the group consisting of the following (1), (2), and (3) as an indicator:

(1) drug efficacy;
(2) toxicity; and
(3) pharmacokinetic characteristics.

[B3] The method according to [B2], wherein the indicator is (3) pharmacokinetic characteristics.

[B4] The method according to any of [B1] to [B3], wherein the candidate compound is selected from multiple test compounds.

[B5] The method according to any of [B1] to [B4], wherein the candidate compound is selected from 3 or more test compounds.

[B6] The method according to any of [B1] to [B5], wherein the candidate compound is a candidate compound for a medicament.

[B7] The method according to any of [B1] to [B6], wherein the subject is a non-human animal.

[B8] The method according to any of [B1] to [B7], wherein the subject is at least one selected from the group consisting of dog, monkey, mini-pig, rabbit, rat, and mouse.

[B9] The method according to any of [B1] to [B8], wherein the subject is at least one selected from the group consisting of rat and mouse.

[B10] The method according to any of [B1] to [B9], wherein the subject is a mouse.

[B11] The method according to any of [B1] to [B10], wherein the administration is oral administration.

[B12] The method according to any of [B1] to [B1 1], wherein the dose of the test compounds to be administered is 0.1 mg/kg or more and 1000 mg/kg or less.

[B13] The method according to any of [B1] to [B11], wherein the dose of the test compounds to be administered is 1 mg/kg or more and 500 mg/kg or less.

[B14] The method according to any of [B1] to [B11], wherein the dose of the test compounds to be administered is

1 mg/kg or more and 100 mg/kg or less.
[B15] The method according to any of [B1] to [B11], wherein the dose of the test compounds to be administered is 1 mg/kg or more and 50 mg/kg or less.
[B16] The method according to any of [B1] to [B11], wherein the dose of the test compounds to be administered is 3 mg/kg or more and 30 mg/kg or less.
[B17] The method according to any of [B1] to [B1 1], wherein the dose of the test compounds to be administered is 10 mg/kg or more and 30 mg/kg or less.
[B18] The method according to any of [B1] to [B11], wherein the dose of the test compounds to be administered is 0.1 mg/kg or more and 10 mg/kg or less.
[B19] The method according to any of [B1] to [B11], wherein the dose of the test compounds to be administered is 1 mg/kg or more and 5 mg/kg or less.
[B20] The method according to any of [B1] to [B11], wherein the dose of the test compounds to be administered is 10 mg/kg or more and 100 mg/kg or less.
[B21] The method according to any of [B1] to [B11], wherein the dose of the test compounds to be administered is 15 mg/kg or more and 50 mg/kg or less.
[B22] The method according to any of [B1] to [B1 1], wherein the dose of the test compounds to be administered is 20 mg/kg or more and 40 mg/kg or less.
[B23] The method according to any of [B1] to [B 1 1], wherein the dose of the test compounds to be administered is 25 mg/kg or more and 35 mg/kg or less.
[B24] The method according to any of [B1] to [B11], wherein the dose of the test compounds to be administered is 3 mg/kg.
[B25] The method according to any of [B1] to [B11], wherein the dose of the test compounds to be administered is 30 mg/kg.

One aspect of the present invention relates to a method for comparing test compounds. Specifically, for example, the present invention encompasses each of the following inventions.

[C1] A method for comparing multiple test compounds, comprising:

administering to a subject the formulation according to any of [A1] to [A99]; and
comparing multiple test compounds using at least one selected from the group consisting of the following (1), (2), and (3) as an indicator:

(1) drug efficacy;
(2) toxicity; and
(3) pharmacokinetic characteristics,

wherein the formulation or agent contains one test compound.

[C2] The method according to [C1], wherein the indicator is (3) pharmacokinetic characteristics.
[C3] The method according to [C1] or [C2], wherein the subject is a non-human animal.
[C4] The method according to any of [C1] to [C3], wherein the subject is at least one selected from the group consisting of dog, monkey, mini-pig, rabbit, rat, and mouse.
[C5] The method according to any of [C1] to [C4], wherein the subject is at least one selected from the group consisting of rat and mouse.
[C6] The method according to any of [C1] to [C5], wherein the subject is a mouse.
[C7] The method according to any of [C1] to [C6], wherein the administration is oral administration.
[C8] The method according to any of [C1] to [C7], wherein the dose of the test compounds to be administered is 0.1 mg/kg or more and 1000 mg/kg or less.
[C9] The method according to any of [C1] to [C7], wherein the dose of the test compounds to be administered is 1 mg/kg or more and 500 mg/kg or less.
[C10] The method according to any of [C1] to [C7], wherein the dose of the test compounds to be administered is 1 mg/kg or more and 100 mg/kg or less.
[C11] The method according to any of [C1] to [C7], wherein the dose of the test compounds to be administered is 1 mg/kg or more and 50 mg/kg or less.
[C12] The method according to any of [C1] to [C7], wherein the dose of the test compounds to be administered is 3 mg/kg or more and 30 mg/kg or less.
[C13] The method according to any of [C1] to [C7], wherein the dose of the test compounds to be administered is

10 mg/kg or more and 30 mg/kg or less.

[C14] The method according to any of [C1] to [C7], wherein the dose of the test compounds to be administered is 0.1 mg/kg or more and 10 mg/kg or less.

[C15] The method according to any of [C1] to [C7], wherein the dose of the test compounds to be administered is 1 mg/kg or more and 5 mg/kg or less.

[C16] The method according to any of [C1] to [C7], wherein the dose of the test compounds to be administered is 10 mg/kg or more and 100 mg/kg or less.

[C17] The method according to any of [C1] to [C7], wherein the dose of the test compounds to be administered is 15 mg/kg or more and 50 mg/kg or less.

[C18] The method according to any of [C1] to [C7], wherein the dose of the test compounds to be administered is 20 mg/kg or more and 40 mg/kg or less.

[C19] The method according to any of [C1] to [C7], wherein the dose of the test compounds to be administered is 25 mg/kg or more and 35 mg/kg or less.

[C20] The method according to any of [C1] to [C7], wherein the dose of the test compounds to be administered is 3 mg/kg.

[C21] The method according to any of [C1] to [C7], wherein the dose of the test compounds to be administered is 30 mg/kg.

One aspect of the present invention relates to a method for enhancing internal absorption of test compounds. Specifically, for example, the present invention encompasses each of the following inventions.

[D1] A method for enhancing internal absorption of test compounds in a subject, comprising:
administering to the subject the formulation according to any of [A1] to [A96].

[D2] The method according to [D1], wherein the internal absorption is digestive absorption.

[D3] The method according to [D1] or [D2], wherein the subject is a non-human animal.

[D4] The method according to any of [D1] to [D3], wherein the subject is at least one selected from the group consisting of dog, monkey, mini-pig, rabbit, rat, and mouse.

[D5] The method according to any of [D1] to [D4], wherein the subject is at least one selected from the group consisting of rat and mouse.

[D6] The method according to any of [D1] to [D5], wherein the subject is a mouse.

[D7] The method according to any of [D1] to [D6], wherein the administration is oral administration.

[D8] The method according to any of [D 1] to [D7], wherein the dose of the test compounds to be administered is 0.1 mg/kg or more and 1000 mg/kg or less.

[D9] The method according to any of [D1] to [D7], wherein the dose of the test compounds to be administered is 1 mg/kg or more and 500 mg/kg or less.

[D10] The method according to any of [D1] to [D7], wherein the dose of the test compounds to be administered is 1 mg/kg or more and 100 mg/kg or less.

[D11] The method according to any of [D1] to [D7], wherein the dose of the test compounds to be administered is 1 mg/kg or more and 50 mg/kg or less.

[D12] The method according to any of [D1] to [D7], wherein the dose of the test compounds to be administered is 3 mg/kg or more and 30 mg/kg or less.

[D13] The method according to any of [D1] to [D7], wherein the dose of the test compounds to be administered is 10 mg/kg or more and 30 mg/kg or less.

[D14] The method according to any of [D1] to [D7], wherein the dose of the test compounds to be administered is 0.1 mg/kg or more and 10 mg/kg or less.

[D15] The method according to any of [D1] to [D7], wherein the dose of the test compounds to be administered is 1 mg/kg or more and 5 mg/kg or less.

[D16] The method according to any of [D1] to [D7], wherein the dose of the test compounds to be administered is 10 mg/kg or more and 100 mg/kg or less.

[D17] The method according to any of [D1] to [D7], wherein the dose of the test compounds to be administered is 15 mg/kg or more and 50 mg/kg or less.

[D18] The method according to any of [D1] to [D7], wherein the dose of the test compounds to be administered is 20 mg/kg or more and 40 mg/kg or less.

[D19] The method according to any of [D1] to [D7], wherein the dose of the test compounds to be administered is 25 mg/kg or more and 35 mg/kg or less.

[D20] The method according to any of [D1] to [D7], wherein the dose of the test compounds to be administered is 3 mg/kg.

[D21] The method according to any of [D 1] to [D7], wherein the dose of the test compounds to be administered is

30 mg/kg.

One aspect of the present invention relates to a method for producing a formulation used for screening a candidate compound. Specifically, for example, the present invention encompasses each of the following inventions.

[E1] A method for producing a formulation used for screening a candidate compound, comprising:

compounding a test compound and an oily component having a polyoxyethylene structure,
wherein the oily component is compounded such that the content is 2.0% by volume or more and 7.5% by volume or less based on 100% by volume of the formulation,
the test compound is the compound according to any of [A1] to [A96], and
the formulation is the formulation according to any of [A1] to [A96].

[E2] The production method according to [E1], wherein the components contained in the formulation are substantially composed of water, the test compound, the oily component having a polyoxyethylene structure, and DMSO.
[E3] The production method according to [E1] or [E2], wherein the formulation is a liquid formulation.
[E4] The production method according to any of [E1] to [E3], wherein the formulation is an oral formulation.

Advantageous Effects of Invention

According to the present invention, oral absorbability can be enhanced in a versatile manner by applying to a compound a formulation containing an oily component having a polyoxyethylene structure in the content range of 2.0% by volume or more and 7.5% by volume or less based on 100% by volume of the formulation. This allows evaluation of different compounds under the same formulation conditions. Then, by using the formulation, it is possible to screen a candidate compound while reducing the risk of overlooking an originally useful compound as a false negative.

Brief Description of Drawings

[Figure 1] Figure 1 is a graph showing the relationship between the concentration of polyoxyethylene castor oil (average number of moles of ethylene oxide added: 35) and the AUC ratio (30 mg/kg). The dashed lines in the graph represent the concentrations of polyoxyethylene castor oil (average number of moles of ethylene oxide added: 35) at 2.0% by volume and 7.5% by volume.
[Figure 2] Figure 2 is a graph showing the relationship between the concentration of polyoxyethylene castor oil (average number of moles of ethylene oxide added: 35) and the AUC ratio (3 mg/kg). The dashed lines in the graph represent the concentrations of polyoxyethylene castor oil (average number of moles of ethylene oxide added: 35) at 2.0% by volume and 7.5% by volume.

Description of Embodiments

Hereinafter, the mode for carrying out the present invention will be described in detail. However, the present invention is not limited by the embodiments given below.
As used herein, the term "one or more" means a number of 1 or 2 or more. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.
As used herein, "to" indicating a range means that values at both ends thereof are included, and for example, "A to B" means a range of A or more and B or less.
The term "about" as used herein, when used in combination with a numeric value, means the value range of +10% and -10% of the numeric value.
In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

[Formulation]

The formulation according to the present embodiment comprises a compound and an oily component having

a polyoxyethylene structure, in which the content of the oily component having a polyoxyethylene structure is 2.0% by volume or more and 7.5% by volume or less based on 100% by volume of the formulation.

(Compound)

There are no particular restrictions on the compound contained in the formulation according to the present embodiment, and any compound can be used. Meanwhile, since the formulation according to the present embodiment can enhance the oral absorbability of the compound in a versatile manner, compounds with low solubility and/or membrane permeability can be suitably used as the compound contained in the formulation according to the present embodiment.

The compound according to one embodiment may have a ClogP of 4 or more and 25 or less. The ClogP is a partition coefficient calculated by a computer, and can be determined in accordance with the principle described in "CLOGP Reference Manual Daylight Version 4.9 (release date: August 1, 2011, https://www.daylight.com/dayhtml/doc/clogp/)". Examples of the method for calculating the ClogP include calculating using Daylight Version 4.95 (release date: August 1, 2011, ClogP algorithm version 5.4, database version 28, https://www.daylight.com/dayhtml/doc/release_notes/index.html/) of Daylight Chemical Information Systems, Inc.

The compound according to the present embodiment may have a ClogP of, for example, 24 or less, 23 or less, 22 or less, 21 or less, 20 or less, 19 or less, 18 or less, 17 or less, or 16.1 or less. The lower limit of the ClogP of the compound according to the present embodiment may be, for example, 5 or more, 6 or more, 7 or more, 8 or more, 9 or more, 10 or more, 11 or more, 12 or more, 13 or more, or 13.2 or more. Examples of the range of the ClogP of the compound according to the present embodiment include 5 or more and 24 or less, 6 or more and 23 or less, 7 or more and 22 or less, 8 or more and 21 or less, 9 or more and 20 or less, 10 or more and 20 or less, 11 or more and 19 or less, 12 or more and 18 or less, 13 or more and 17 or less, and 13.2 or more and 16.1 or less. Also, for example, the ClogP of the compound according to the present embodiment may be more than or equal to the ClogP of the compound 2, may be less than or equal to the ClogP of the compound 1, or may be more than or equal to the ClogP of the compound 2 and less than or equal to the ClogP of the compound 1.

The compound according to one embodiment may have a value of Caco-2 Papp (cm/sec) of 1.0E-9 or more.

The value of Caco-2 Papp (cm/sec) is a value that serves as an indicator of the membrane permeability in the cell membrane, and can be measured by the method below.

(a) Caco-2 cells are cultured on 96-well Transwell for 3 weeks, then a compound to be evaluated, a DMEM medium, and FaSSIF (1% DMSO) are added to the Apical side, a DMEM medium is added to the Basal side, and the cells are preincubated under a 5% $CO_2$ atmosphere at 37°C with shaking at 80 rpm for 18 to 24 hours.
(b) After the preincubation, the preincubation solutions on the Apical side and Basal side are sucked off and washed, and a FaSSIF/HBSS buffer solution (1% DMSO) (pH 6.0) containing the compound is added to the Apical side, and a HBSS buffer solution (4% BSA) (pH 7.4) is added to the Basal side.
(c) Each well is shaken under a 5% $CO_2$ atmosphere at 37°C and 80 rpm, and at 180 minutes after the start of shaking, a sample on the Basal side is collected, and the permeation amount of the compound is measured by liquid chromatography-mass spectrometry (LC/MS).
(d) From the measured permeation amount, the permeability coefficient (Caco-2 Papp (cm/sec)) is calculated.

The permeability coefficient (Caco-2 Papp (cm/sec)) is calculated using a numerical expression 1.
[Expression 1]

$$P_{app} = \frac{1}{C_1(0) \times S} \times \frac{dQ}{dt} \qquad \text{(Numerical Expression 1)}$$

where dQ/dt is the permeation rate of the drug (amount of drug appearing on the Basal side per unit time), S is the total cell surface area, and Ci(0) is the concentration of drug added on the Apical side.

Note that, in the above measurement, a Pgp inhibitor, such as Zosquidar, can be added to each of the FaSSIF/HBSS buffer solution (1% DMSO) (pH 6.0) and the HBSS buffer solution (4% BSA) (pH 7.4).

In addition, as the concentration of the compound to be evaluated on the donor side used in the calculation of the permeability coefficient in the above step (d), the concentration of the compound when initially added can be used, or the concentration of the compound measured by collecting the solution on the Apical side before the start of preincubation or the start of shaking in the above step (c) can be used. In particular, when the preincubation is performed, it is preferable to use the concentration of the compound on the Apical side collected before pre-incubation.

The value of Caco-2 Papp (cm/sec) of the compound according to the present embodiment may be, for example, 1.0E-4 or less, 9.0E-5 or less, 8.0E-5 or less, 7.0E-5 or less, 6.0E-5 or less, 5.0E-5 or less, 4.0E-5 or less, 3.0E-5 or

less, 2.0E-5 or less, 1.0E-5 or less, 9.0E-6 or less, 8.0E-6 or less, 7.0E-6 or less, 6.0E-6 or less, or 5.99E-6 or less. The lower limit of the value of Caco-2 Papp (cm/sec) of the compound according to the present embodiment may be, for example, 1.0E-9 or more, 1.0E-8 or more, 2.0E-8 or more, 3.0E-8 or more, 4.0E-8 or more, 5.0E-8 or more, 6.0E-8 or more, 7.0E-8 or more, 8.0E-8 or more, 9.0E-8 or more, 1.0E-7 or more, 1.1E-7 or more, 1.2E-7 or more, 1.3E-7 or more, or 1.4E-7 or more. Examples of the range of the value of Caco-2 Papp (cm/sec) of the compound according to the present embodiment include 1.0E-9 or more and 1.0E-4 or less, 1.0E-8 or more and 1.0E-5 or less, 1.0E-7 or more and 1.0E-5 or less, and 1.4E-7 or more and 5.99E-6 or less. It should be noted that E-n (n is a natural number) means $10^{-n}$ (for example, 1.0E-5 = $1.0 \times 10^{-5}$). Also, for example, the value of Caco-2 Papp (cm/sec) of the compound according to the present embodiment may be more than or equal to the value of Papp (cm/sec) of the compound 9, may be less than or equal to the value of Papp (cm/sec) of the compound 2, or may be more than or equal to the value of Papp (cm/sec) of the compound 9 and less than or equal to the value of Papp (cm/sec) of the compound 2.

The compound according to one embodiment may have a solubility of 10 mg/mL or less in a 50 mM phosphate buffer solution (pH 6.5) at 25°C. The compound according to the present embodiment may have a solubility of, for example, 5 mg/mL or less, 2.5 mg/mL or less, 2 mg/mL or less, 1 mg/mL or less, 0.5 mg/mL or less, 0.25 mg/mL or less, 0.1 mg/mL or less, 0.05 mg/mL or less, 0.025 mg/mL or less, 0.01 mg/mL or less, 0.005 mg/mL or less, 0.0025 mg/mL or less, or 0.001 mg/mL or less in a 50 mM phosphate buffer solution (pH 6.5) at 25°C.

The compound according to one embodiment may be a compound classified into class IV by the Biopharmaceutics Classification System (BCS). The BCS is a guideline for predicting the digestive absorption characteristics of a pharmaceutical by classifying the pharmaceutical into four classes (classes I to IV) based on its solubility and absorption rate.

The solubility in the BCS ($D_0$: Dose Number) is determined with $D_0$ = 1 as the boundary, and the solubility is determined to be high (high solubility) when $D_0 \leq 1$ and determined to be low (low solubility) when $D_0 \geq 1$. The absorption rate in the BCS ($F_a$: a rate of absorption from the digestive tract) is determined with an absorption rate of 90% as the boundary, and the absorption rate is determined to be low (low absorption rate) when $F_a \leq 0.9$, and determined to be high (high absorption rate) when $F_a \geq 0.9$. Classes I to IV of the BCS are defined as follows:

Class I: high solubility ($D_0 \leq 1$) and high absorption rate ($F_a \geq 0.9$);
Class II: low solubility ($D_0 \geq 1$) and high absorption rate ($F_a \geq 0.9$);
Class III: high solubility ($D_0 \leq 1$) and low absorption rate ($F_a \leq 0.9$); and
Class IV: low solubility ($D_0 \geq 1$) and low absorption rate ($F_a \leq 0.9$).

The compound according to one embodiment may have a molecular weight of 5000 or less. The molecular weight as used herein means the sum of the atomic weights of the atoms constituting the compound molecule (unit: "g/mol"), and is obtained by calculating the sum of the atomic weights of the atoms included in the molecular structure formula (unit: "g/mol"). Below, the unit of molecular weight will sometimes be omitted herein.

There are no particular limitations on the molecular weight of the compound according to the present embodiment, and it may be, for example, 500 or more, 550 or more, 600 or more, 650 or more, 700 or more, 750 or more, 800 or more, 850 or more, 900 or more, or 950 or more, or may be 1000 or more, 1100 or more, 1200 or more, 1300 or more, or 1400 or more. There are no particular limitations on the upper limit of the molecular weight of the compound according to the present embodiment, and it may be 5000 or less, 4000 or less, 3000 or less, 2500 or less, 2000 or less, 1900 or less, 1800 or less, 1700 or less, or 1600 or less. Examples of the range of the molecular weight of the compound according to the present embodiment include 500 or more and 5000 or less, 750 or more and 4000 or less, 1000 or more and 3000 or less, 1100 or more and 2000 or less, 1200 or more and 1800 or less, and 1400 or more and 1600 or less.

The compound according to one embodiment may be a peptide compound. As used herein, there are no particular limitations on the "peptide compound" as long as it is a peptide compound in which amino acid residues are linked by an amide bond or an ester bond, and it is preferable that they are linked by an amide bond. There are no particular restrictions on the number of amino acid residues in the peptide compound, and it is preferably 5 or more, more preferably 7 or more, still more preferably 8 or more, and even more preferably 9 or more. Also, the number of amino acid residues in the peptide compound is preferably 30 or less, more preferably 25 or less, still more preferably 15 or less, and even more preferably 13 or less. The number of amino acid residues in the peptide compound may be, for example, 5 or more and 30 or less, 7 or more and 25 or less, 8 or more and 15 or less, 9 or more and 13 or less, or 11. The peptide compound may have a branched structure.

As used herein, the "amino acid residue" constituting a peptide compound is sometimes referred to simply as an "amino acid".

The term "amino acid" as used herein includes a natural amino acid and a non-natural amino acid. The term "amino acid" as used herein may mean an amino acid residue. Furthermore, as used herein, the term "amino acid residue" includes a natural amino acid residue and a non-natural amino acid residue.

Natural amino acids refer to glycine (Gly), alanine (Ala), serine (Ser), threonine (Thr), valine (Val), leucine (Leu),

isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro).

Examples of the non-natural amino acid include, but are not particularly limited to, β-amino acids, D-amino acids, N-substituted amino acids (excluding Pro), α,α-disubstituted amino acids, amino acids having a side chain different from that of a natural amino acid, and hydroxycarboxylic acids. As used herein, the non-natural N-substituted amino acid means a N-substituted amino acid other than Pro.

As the amino acid as used herein, any steric configuration is acceptable. The selection of a side chain of the amino acid is not particularly restricted, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, and a spiro-bonded cycloalkyl group. Each of the side chains may have a substituent. The substituent is not restricted either, and one or two or more substituents may be freely selected independently from any substituents including, for example, a halogen atom, an O atom, a S atom, a N atom, a B atom, a Si atom, or a P atom. That is, examples of the side chain include an alkyl group, an alkoxy group, an alkoxyalkyl group, an alkenyl group, an alkynyl group, an aryl group, a heteroaryl group, an aralkyl group, or a cycloalkyl group which may be substituted, or oxo, aminocarbonyl, and a halogen atom. In one non-limiting aspect, the amino acid as used herein may be a compound having a carboxyl group and an amino group in the same molecule (even in this case, the amino acid also includes imino acids such as proline and hydroxyproline).

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C(=O)-R), carboxy ($-CO_2H$), oxycarbonyl(-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxylcarbonyl ($-C(=O)-CO_2H$).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkyl carbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. In addition to these, examples include compounds in which a H atom bonded to the N atom in -C(=O)-NHR is further substituted with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition to these, examples include compounds in which a H atom bonded to the N atom in -NH-C(=O)-R is further substituted with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. In addition to these, examples include compounds in which a H atom bonded to the N atom in -NH-C(=O)-OR is further substituted with alkyl, cycloalkyl, alkenyl, alkynyl, aryl, heteroaryl, or aralkyl.

Examples of sulfonylamino ($-NH-SO_2-R$) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition to these, examples include compounds in which a H atom bonded to the N atom in $-NH-SO_2-R$ is further substituted with alkyl, cycloalkyl, alkenyl, alkynyl, aryl, heteroaryl, or aralkyl.

Examples of aminosulfonyl ($-SO_2-NHR$) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition to these, examples include compounds in which a H atom bonded to the N atom in $-SO_2-NHR$ is further substituted with alkyl, cycloalkyl, alkenyl, alkynyl, aryl, heteroaryl, or aralkyl.

Examples of sulfamoylamino ($-NH-SO_2-NHR$) include alkylsulfamoylamino, cycloalkylsulfamoylamino, alkenylsulfamoylamino, alkynylsulfamoylamino, arylsulfamoylamino, heteroaryl sulfamoyl amino, and aralkylsulfamoylamino. Furthermore, the two H atoms bonded to the N atoms in $-NHSO_2-NHR$ may be substituted with substituents independently

selected from the group consisting of alkyl, cycloalkyl, alkenyl, alkynyl, aryl, heteroaryl, and aralkyl, and the two substituents may form a ring.

Examples of S atom-derived substituents include thiol (-SH), thio (-S-R), sulfinyl (-S(=O)-R), sulfonyl ($-S(O)_2-R$), sulfo ($-SO_3H$), and pentafluorosulfanyl ($-SF_5$).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl ($-S(O)_2-R$) include alkylsulfonyl, cycloalkylsulfonyl, alkenylsulfonyl, alkynylsulfonyl, arylsulfonyl, heteroarylsulfonyl, and aralkylsulfonyl.

Examples of N atom-derived substituents include azido ($-N_3$, also referred to as "azide group"), cyano (-CN), primary amino ($-NH_2$), secondary amino (-NH-R), tertiary amino (-NR(R')), amidino ($-C(=NH)-NH_2$), substituted amidino (-C(=NR)-NR'R"), guanidino ($-NH-C(=NH)-NH_2$), substituted guanidino (-NR-C(=NR"')-NR'R"), and aminocarbonylamino (-NR-CO-NR'R").

Examples of secondary amino (-NH-R) include alkylamino, cycloalkylamino, alkenylamino, alkynylamino, arylamino, heteroaryl amino, and aralkylamino.

Examples of tertiary amino (-NR(R')) include amino groups having any two substituents each independently selected from alkyl, cycloalkyl, alkenyl, alkynyl, aryl, heteroaryl, aralkyl, and the like, such as alkyl(aralkyl)amino, and the any two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR"')-NR'R") include groups in which R, R', R", and R"' are each independently selected from alkyl, cycloalkyl, alkenyl, alkynyl, aryl, heteroaryl, and aralkyl, and groups in which these substituents form a ring.

Examples of aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of B atom-derived substituents include boryl (-BR(R')) and dioxyboryl (-B(OR)(OR')). The two substituents R and R' may be groups each independently selected from alkyl, cycloalkyl, alkenyl, alkynyl, aryl, heteroaryl, aralkyl, or the like, or may be a group in which they form a ring. Specific examples include a cyclic boryl group, and more specifically include a pinacolate boryl group, a neopentanediolate boryl group, and a catecholate boryl group.

The amino group of the main chain of the amino acid may be unsubstituted ($-NH_2$) or substituted (that is, -NHR). R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position $\alpha$ may form a ring, like proline.

As used herein, an amino acid in which the amino group of the main chain is substituted is referred to as a "N-substituted amino acid". Examples of the "N-substituted amino acid" as used herein include preferably, but are not limited to, N-alkylamino acid, N-$C_1$-$C_6$ alkylamino acid, N-$C_1$-$C_5$ alkylamino acid, N-$C_1$-$C_4$ alkylamino acid, N-$C_1$-$C_3$ alkylamino acid, N-ethylamino acid, N-methylamino acid, N-$C_7$-$C_{14}$ aralkylamino acid, N-benzylamino acid, and N-phenethylamino acid.

Specific examples of the substituent on the nitrogen atom of the N-substituted amino acid (R of -NHR described above) herein include an alkyl group (preferably a $C_1$-$C_6$ alkyl group, more preferably a $C_1$-$C_4$ alkyl group, more preferably a $C_1$-$C_3$ alkyl group, and still more preferably an ethyl group or a methyl group), a $C_7$-$C_{14}$ aralkyl group, a benzyl group, and a phenethyl group. As the substituent on the nitrogen atom of the N-substituted amino acid, an ethyl group or a methyl group is more preferred, and a methyl group is particularly preferred (that is, N-methylamino acid is particularly preferred as the N-substituted amino acid).

The "amino acid" as used herein includes all isotopes corresponding to each. The isotope of the "amino acid" is a form in which at least one atom is replaced at a ratio different from that in a natural amino acid with an atom identical thereto in atomic number (proton number) and different therefrom in mass number (total number of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom, which respectively include $^{2}H$, $^{3}H$, $^{13}C$, $^{14}C$, $^{15}N$, $^{17}O$, $^{18}O$, $^{32}P$, $^{35}S$, $^{18}F$, and $^{36}Cl$. For the compounds as used herein, all the compounds containing any proportions of radioactive or non-radioactive isotopic element are encompassed within the scope of the present invention.

The peptide compound according to the present embodiment may contain 2 or more N-substituted amino acid residues. The number of N-substituted amino acid residues contained in the peptide compound according to the present embodiment may be, for example, 3 or more, 4 or more, or 5 or more.

The peptide compound according to the present embodiment may be a peptide compound having a cyclic moiety (sometimes referred to as "cyclic peptide compound" herein). As used herein, the term "cyclic moiety" of a peptide compound means a cyclic portion formed by linkage of two or more amino acid residues. The number of amino acid residues constituting the cyclic moiety of a cyclic peptide compound may be 5 or more and 15 or less, 6 or more and 15 or less, 7 or more and 15 or less, 8 or more and 15 or less, 9 or more and 15 or less, 10 or more and 15 or less, 5 or more and 14 or less, 6 or more and 14 or less, 7 or more and 14 or less, 8 or more and 14 or less, 9 or more and 14 or less, 10 or more and 14 or less, 5 or more and 13 or less, 6 or more and 13 or less, 7 or more and 13 or less, 8 or more and 13 or less, 9 or more and 13 or less, 10 or more and 13 or less, 5 or more and 12 or less, 6 or more and 12 or less, 7 or more and 12 or less, 8 or more and 12 or less, 9 or more and 12 or less, 10 or more and 12 or less, 5 or more and 11 or less, 6 or more and 11 or less, 7 or more and 11 or less, 8 or more and 11 or less, 9 or more and 11 or less, 10 or more and 11 or less, or 11. The cyclic moiety is preferably formed via a covalent bond such as an amide bond, a carbon-carbon bond forming reaction, a S-S bond, a thioether bond, and a triazole bond, and can be formed by allowing a group on the N-terminal side of a linear peptide compound in which amino acid residues are linked and a group on the C-terminal side to be bonded to each other. Specifically, for example, the cyclic moiety can be formed by allowing an amino group on the N-terminal side of a linear peptide compound in which amino acid residues are linked and a carboxyl group on the C-terminal side to be bonded to each other. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, cyclization with a sulfur-sulfur bond, or cyclization by heterocyclic construction. Among these, cyclization via a covalent bond such as an amide bond and a carbon-carbon bond is preferred, and cyclization via an amide bond by a carboxy group of a side chain and an amino group of the main chain is more preferred. The positions of the carboxy group, the amino group, and the like used for cyclization may be on the main chain or the side chain, and are not particularly restricted as long as the positions allow the groups to be cyclized.

In the cyclic peptide compound according to the present embodiment, the number of ring atoms may be 15 or more and 46 or less. As used herein, the term "number of ring atoms" means the number of atoms (ring atoms) of a cyclic compound including the innermost portion of the ring, and is defined as the number of such atoms of the ring with the highest number of such atoms when the compound has multiple rings. Note that, when two rings exist sharing some atoms, the smaller number of shared atoms is used to calculate the number of ring atoms in each ring. Further describing the "number of ring atoms" by means of specific examples, for example, using this method, the number of ring atoms of tetrahydrofuran (THF) is 5, the number of ring atoms of tacrolimus (FK506) is 21, and the number of ring atoms of the compound 1 and the compound 9, described in Examples, is 34.

The number of ring atoms of the cyclic peptide compound according to the present embodiment may be, for example, 34 or more and 46 or less, 34 or more and 43 or less, 34 or more and 40 or less, 34 or more and 37 or less, 34 or more and 36 or less, or 34. The ring atoms used to calculate the number of ring atoms may be selected from the group consisting of carbon atom, hydrogen atom, nitrogen atom, oxygen atom, sulfur atom, phosphorus atom, and silicon atom, and may be selected from the group consisting of carbon atom, hydrogen atom, nitrogen atom, and oxygen atom.

The peptide compound according to one embodiment may have a ClogP/number of amino acid residues of 1.0 or more. As used herein, the term "number of amino acid residues" means a total number of amino acid residues constituting a peptide compound. For example, the number of amino acid residues of a cyclic peptide compound in which the cyclic moiety is composed of 10 amino acid residues and the linear moiety is composed of 1 amino acid residue is 11. The ClogP/number of amino acid residues is a value calculated by dividing the ClogP of a peptide compound by the number of amino acid residues contained in the peptide compound. For example, when the ClogP of a peptide compound is 14.0 and the number of amino acid residues contained in the peptide compound is 7, the ClogP/number of amino acid residues of the peptide compound is calculated to be 2.0.

The ClogP/number of amino acid residues of the peptide compound according to the present embodiment may be, for example, 1.1 or more or 1.2 or more. The upper limit of the ClogP/number of amino acid residues of the peptide compound according to the present embodiment may be, for example, 1.8 or less, 1.7 or less, 1.6 or less, or 1.5 or less. Examples of the range of the ClogP/number of amino acid residues of the peptide compound according to the present embodiment include 1.0 or more and 1.8 or less, 1.0 or more and 1.7 or less, 1.1 or more and 1.6 or less, and 1.1 or more and 1.5 or less.

The compound according to the present embodiment may have one of the features such as physical properties, characteristics, and structure described above, or it may have two or more the features in any combination. Further specific examples of the compound according to the present embodiment will be described below.

The compound according to one embodiment has the following features (i) and (ii):

(i) the compound has a ClogP of 9 or more; and
(ii) the compound has a value of Caco-2 Papp (cm/sec) of 1.0E-5 or less.

The compound according to one embodiment has the following features (i) and (ii):

(i) the compound has a ClogP of 10 or more; and
(ii) the compound has a value of Caco-2 Papp (cm/sec) of 1.0E-7 or more and 1.0E-5 or less.

The compound according to one embodiment has the following features (i), (ii), and (iii):

(i) the compound has a ClogP of 13 or more;
(ii) the compound has a value of Caco-2 Papp (cm/sec) of 1.4E-7 or more and 6.0E-6 or less; and
(iii) the compound has a molecular weight of 1000 or more.

The compound according to one embodiment is a peptide compound having a cyclic moiety and has the following features (i) and (ii):

(i) the compound has a cyclic moiety composed of 5 or more and 12 or less amino acid residues, and the number of amino acid residues is 9 or more and 13 or less; and
(ii) the compound contains at least 2 N-substituted amino acid residues and at least 1 non-N-substituted amino acid residue.

The compound according to one embodiment is a peptide compound having a cyclic moiety and has the following features (i) and (ii):

(i) the compound has a cyclic moiety composed of 9 or more and 12 or less amino acid residues, and the number of amino acid residues is 9 or more and 13 or less; and
(ii) the compound contains at least 3 N-substituted amino acid residues and at least 1 non-N-substituted amino acid residue.

The compound according to one embodiment is a peptide compound having a cyclic moiety and has the following features (i) and (ii):

(i) the compound has a cyclic moiety composed of 11 amino acid residues, and the number of amino acid residues is 11; and
(ii) the compound contains at least 4 N-substituted amino acid residues and at least 1 non-N-substituted amino acid residue.

The compound according to one embodiment is a peptide compound having a cyclic moiety and has the following features (i), (ii), (iii), and (iv):

(i) the compound has a cyclic moiety composed of 5 or more and 12 or less amino acid residues, and the number of amino acid residues is 9 or more and 13 or less;
(ii) the compound contains at least 2 N-substituted amino acid residues and at least 1 non-N-substituted amino acid residue;
(iii) the compound has a ClogP of 9 or more; and
(iv) the compound has a value of Caco-2 Papp (cm/sec) of 1.0E-5 or less.

The compound according to one embodiment is a peptide compound having a cyclic moiety and has the following features (i), (ii), (iii), and (iv):

(i) the compound has a cyclic moiety composed of 9 or more and 12 or less amino acid residues, and the number of amino acid residues is 9 or more and 13 or less;
(ii) the compound contains at least 3 N-substituted amino acid residues and at least 1 non-N-substituted amino acid residue;
(iii) the compound has a ClogP of 9 or more; and
(iv) the compound has a value of Caco-2 Papp (cm/sec) of 1.0E-5 or less.

The compound according to one embodiment is a peptide compound having a cyclic moiety and has the following features (i), (ii), (iii), and (iv):

(i) the compound has a cyclic moiety composed of 11 amino acid residues, and the number of amino acid residues is 11;
(ii) the compound contains at least 4 N-substituted amino acid residues and at least 1 non-N-substituted amino acid residue;
(iii) the compound has a ClogP of 11 or more; and
(iv) the compound has a value of Caco-2 Papp (cm/sec) of 6.0E-6 or less.

The compound according to one embodiment is a peptide compound having a cyclic moiety and has the following features (i) and (ii):

(i) the number of amino acid residues is 9 or more and 13 or less, and the number of ring atoms of the cyclic moiety is 34; and
(ii) the compound contains at least 2 N-substituted amino acid residues and at least 1 non-N-substituted amino acid residue.

The compound according to one embodiment is a peptide compound having a cyclic moiety and has the following features (i), (ii), (iii), (iv), and (v):

(i) the number of amino acid residues is 9 or more and 13 or less, and the number of ring atoms of the cyclic moiety is 34;
(ii) the compound contains at least 2 N-substituted amino acid residues and at least 1 non-N-substituted amino acid residue;
(iii) the compound has a ClogP of 9 or more;
(iv) the compound has a value of Caco-2 Papp (cm/sec) of 1.0E-5 or less; and
(v) the compound has a molecular weight of 1000 or more.

The compound according to one embodiment is a peptide compound having a cyclic moiety and has the following features (i), (ii), (iii), (iv), and (v):

(i) the number of amino acid residues is 11, and the number of ring atoms of the cyclic moiety is 34;
(ii) the compound contains at least 4 N-substituted amino acid residues and at least 1 non-N-substituted amino acid residue;
(iii) the compound has a ClogP of 13 or more;
(iv) the compound has a value of Caco-2 Papp (cm/sec) of 1.4E-7 or more and 6.0E-6 or less; and
(v) the compound has a molecular weight of 1000 or more.

The compound according to the present embodiment may not include, for example, cyclosporin and analogues thereof. Analogues of cyclosporin are those formed by chemically modifying cyclosporin while maintaining the biological actions that cyclosporin has. Examples of analogues of cyclosporin include dihydrocyclosporin D.

Also, the compound according to the present embodiment may not include, for example, (5S,8S,11S,15S,18S,23aS,29S,35S,37aS)-8-((S)-sec-butyl)-18-cyclopentyl-29-(3,5-difluoro-4-(trifluoromethyl)phenethyl)-36-ethyl-11-isobutyl-N,N,5,6,12,16,19,33-octamethyl-35-(4-methylbenzyl)-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontine-21,1'-cyclopentane]-15-carboxamide,(5S,8S,11S,15S,18S,23aS,25R,29S,35S,37aS)-8-((S)-sec-butyl)-35-(cyclohexylmethyl)-18-cyclopentyl-29-(3,5-difluoro-4-(trifluoromethyl)phenethyl)-25-ethoxy-11-isobutyl-N,N,5,6,12,16,19,33,36-nonamethyl-4,7,10,13,17,20,23,28,31,34,37-undecaoxotetratriacontahydro-2H,4H-spiro[azeto[2,1-u]pyrrolo[2,1-i][1,4,7,10,13,16,19,22,25,28,31]undecaazacyclotetratriacontine-21,1'-cyclopentane]-15-carboxamide, (3S,9S,18S,21S,25S,28S,34S)-3-[2-[3-chloro-4-(trifluoromethyl)phenyl]ethyl]-28-cyclohexyl-9-(cyclohexylmethyl)-21-isobutyl-7,10,13,16,22,26,29-heptamethyl-18-[(1S)-1-methylpropyl]-25-(piperidine-1-carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecaone, (5S,8S, 11 S, 15S, 18S,23aS,29S,35S,37aS)-8-((S)-sec-butyl)-29-(3-chloro-4-(trifluoromethyl)phenethyl)-35-(cyclohexylmethyl)-18-cyclopentyl-11-isobutyl-5,6,12,16,19,33,36-heptamethyl-15-(morpholine-4-carbonyl)docosahydro-2H,4H-spiro[azeto[2,1-u]pyrrolo[2,1-i][1,4,7,10,13,16,19,22,25,28,31]undecaazacyclotetratriacontine-21,1'-cyclopentane]-4,7,10,13,17,20,23,28,31,34,37(14H,22H)-undecaone, and (5S,8S,11S,15S,18S,23aS,29S,35S,37aS)-8-((S)-sec-butyl)-29-(3-chloro-4-(trifluoromethyl)phenethyl)-18-cyclopentyl-36-ethyl-11-isobutyl-5,6,12,16,19,33-hexamethyl-35-(4-methylbenzyl)-15-(morpholine-4-carbonyl)docosahydro-2H,4H-spiro[azeto[2,1-u]pyrrolo[2,1-i][1,4,7,10,13,16,19,22,25,28,31]undecaazacyclotetratriacontine-21,1'-cyclopentane]-4,7,10,13,17,20,23,28,31,34,37(14H,22H)-undecaone.

Only one kind of the compound according to the present embodiment is usually contained in one formulation.

(Oily component having polyoxyethylene structure)

The formulation according to the present embodiment comprises an oily component having a polyoxyethylene structure (sometimes simply referred to as "oily component"). The polyoxyethylene structure means a structure represented by $-(CH_2CH_2O)_n-$ (n is a natural number).
The average number of moles of ethylene oxide added of the oily component according to the present embodiment may be, for example, 2 or more and 150 or less. The average number of moles of ethylene oxide added of the oily component according to the present embodiment may be, for example, 5 or more, 10 or more, 15 or more, 20 or more, 25 or more, or 30 or more, and it may be 140 or less, 130 or less, 120 or less, 110 or less, 100 or less, 90 or less, 80 or less, 70 or less, 60 or less, 50 or less, 40 or less, 39 or less, 38 or less, 37 or less, or 36 or less. Examples of the range of the average number of moles of ethylene oxide added of the oily component according to the present embodiment include 10 or more and 90 or less, 15 or more and 60 or less, 20 or more and 50 or less, 25 or more and 40 or less, 30 or more and 39 or less, and 35.
Examples of the oily component according to the present embodiment may include polyoxyethylene castor oil, polyoxyethylene hydrogenated castor oil, and polyoxyethylene sorbitan fatty acid esters. More specific examples of the oily component according to the present embodiment may include a polyoxyethylene castor oil with an average number of moles of ethylene oxide added of 30 or more and 39 or less, and a polyoxyethylene castor oil with an average number of moles of ethylene oxide added of 35.
As the oily component according to the present embodiment, one kind may be used alone, or two or more kinds may be used in combination.

(Formulation)

The formulation according to the present embodiment at least comprises the above-described compound and oily component having a polyoxyethylene structure. The content of the oily component having a polyoxyethylene structure may be 2.0% by volume or more and 7.5% by volume or less based on 100% by volume of the formulation according to the present embodiment.
The content of the oily component having a polyoxyethylene structure may be, for example, 2.1% by volume or more, 2.2% by volume or more, 2.3% by volume or more, 2.4% by volume or more, 2.5% by volume or more, 2.6% by volume or more, 2.7% by volume or more, 2.8% by volume or more, 2.9% by volume or more, 3.0% by volume or more, 3.1% by volume or more, 3.2% by volume or more, 3.3% by volume or more, 3.4% by volume or more, 3.5% by volume or more, 3.6% by volume or more, 3.7% by volume or more, 3.8% by volume or more, 3.9% by volume or more, 4.0% by volume or more, 4.1% by volume or more, 4.2% by volume or more, 4.3% by volume or more, 4.4% by volume or more, 4.5% by volume or more, 4.6% by volume or more, 4.7% by volume or more, 4.8% by volume or more, 4.9% by volume or more, 5.0% by volume or more, 5.1% by volume or more, 5.2% by volume or more, 5.3% by volume or more, 5.4% by volume or more, 5.5% by volume or more, 5.6% by volume or more, 5.7% by volume or more, 5.8% by volume or more, 5.9% by volume or more, 6.0% by volume or more, 6.1% by volume or more, 6.2% by volume or more, 6.3% by volume or more, 6.4% by volume or more, 6.5% by volume or more, 6.6% by volume or more, 6.7% by volume or more, 6.8% by volume or more, 6.9% by volume or more, 7.0% by volume or more, 7.1% by volume or more, 7.2% by volume or more, 7.3% by volume or more, or 7.4% by volume or more based on 100% by volume of the formulation according to the present embodiment.
The content of the oily component having a polyoxyethylene structure may be, for example, 7.4% by volume or less, 7.3% by volume or less, 7.2% by volume or less, 7.1% by volume or less, 7.0% by volume or less, 6.9% by volume or less, 6.8% by volume or less, 6.7% by volume or less, 6.6% by volume or less, 6.5% by volume or less, 6.4% by volume or less, 6.3% by volume or less, 6.2% by volume or less, 6.1% by volume or less, 6.0% by volume or less, 5.9% by volume or less, 5.8% by volume or less, 5.7% by volume or less, 5.6% by volume or less, 5.5% by volume or less, 5.4% by volume or less, 5.3% by volume or less, 5.2% by volume or less, 5.1% by volume or less, 5.0% by volume or less, 4.9% by volume or less, 4.8% by volume or less, 4.7% by volume or less, 4.6% by volume or less, 4.5% by volume or less, 4.4% by volume or less, 4.3% by volume or less, 4.2% by volume or less, 4.1% by volume or less, 4.0% by volume or less, 3.9% by volume or less, 3.8% by volume or less, 3.7% by volume or less, 3.6% by volume or less, 3.5% by volume or less, 3.4% by volume or less, 3.3% by volume or less, 3.2% by volume or less, 3.1% by volume or less, 3.0% by volume or less, 2.9% by volume or less, 2.8% by volume or less, 2.7% by volume or less, 2.6% by volume or less, 2.5% by volume or less, 2.4% by volume or less, 2.3% by volume or less, 2.2% by volume or less, or 2.1% by volume or less based on 100% by volume of the formulation according to the present embodiment.
Examples of the range of the content of the oily component having a polyoxyethylene structure may include 2.1% by volume or more and 7.4% by volume or less, 2.2% by volume or more and 7.3% by volume or less, 2.3% by volume or more and 7.2% by volume or less, 2.4% by volume or more and 7.1% by volume or less, 2.5% by volume or more and 7.0% by volume or less, 2.6% by volume or more and 6.9% by volume or less, 2.7% by volume or more and

6.8% by volume or less, 2.8% by volume or more and 6.7% by volume or less, 2.9% by volume or more and 6.6% by volume or less, 3.0% by volume or more and 6.5% by volume or less, 3.1% by volume or more and 6.4% by volume or less, 3.2% by volume or more and 6.3% by volume or less, 3.3% by volume or more and 6.2% by volume or less, 3.4% by volume or more and 6.1% by volume or less, 3.5% by volume or more and 6.0% by volume or less, 3.6% by volume or more and 5.9% by volume or less, 3.7% by volume or more and 5.8% by volume or less, 3.8% by volume or more and 5.7% by volume or less, 3.9% by volume or more and 5.6% by volume or less, 4.0% by volume or more and 5.5% by volume or less, 4.1% by volume or more and 5.4% by volume or less, 4.2% by volume or more and 5.3% by volume or less, 4.3% by volume or more and 5.2% by volume or less, 4.4% by volume or more and 5.1% by volume or less, 4.5% by volume or more and 5.0% by volume or less, as well as 2.0% by volume or more and 3.9% by volume or less, 2.0% by volume or more and 3.5% by volume or less, 4.1% by volume or more and 4.9% by volume or less, 5.1% by volume or more and 7.5% by volume or less, 5.5% by volume or more and 7.5% by volume or less, and 6.0% by volume or more and 7.5% by volume or less based on 100% by volume of the formulation according to the present embodiment. Examples of the range of the content of the oily component having a polyoxyethylene structure may further include 2.0% by volume or more and less than 7.5% by volume, 2.0% by volume or more and 7.0% by volume or less, 2.0% by volume or more and 5.0% by volume or less, 2.5% by volume or more and 7.5% by volume or less, and 2.5% by volume or more and 5.0% by volume or less based on 100% by volume of the formulation according to the present embodiment.

There are no particular restrictions on the content of the compound in the formulation according to the present embodiment, and it may be, for example, 0.1 mg/mL or more and 100 mg/mL or less. The content of the compound in the formulation according to the present embodiment may be, for example, 0.2 mg/mL or more, 0.3 mg/mL or more, 0.4 mg/mL or more, 0.5 mg/mL or more, 0.6 mg/mL or more, 0.7 mg/mL or more, 0.8 mg/mL or more, 0.9 mg/mL or more, or 1 mg/mL or more, and it may be 90 mg/mL or less, 80 mg/mL or less, 70 mg/mL or less, 60 mg/mL or less, 50 mg/mL or less, 40 mg/mL or less, or 30 mg/mL or less. Examples of the range of the content of the compound in the formulation according to the present embodiment may include 0.2 mg/mL or more and 90 mg/mL or less, 0.3 mg/mL or more and 80 mg/mL or less, 0.4 mg/mL or more and 70 mg/mL or less, 0.5 mg/mL or more and 60 mg/mL or less, 0.6 mg/mL or more and 50 mg/mL or less, 0.7 mg/mL or more and 40 mg/mL or less, and 0.8 mg/mL or more and 30 mg/mL or less.

The formulation according to the present embodiment may comprise a carrier. Examples of the carrier include aqueous media such as saline, buffered saline, water, and isotonic aqueous buffer solution, dimethyl sulfoxide (DMSO), and combinations thereof.

When the formulation according to the present embodiment comprises DMSO, the content of DMSO may be, for example, 1% by volume or more and 30% by volume or less, 3% by volume or more and 25% by volume or less, 5% by volume or more and 20% by volume or less, 7% by volume or more and 15% by volume or less, or 10% by volume based on 100% by volume of the formulation.

The formulation according to the present embodiment may comprise components (other components) other than the above-described compound and oily component having a polyoxyethylene structure, to the extent that the effects according to the present invention are not impaired. Examples of the other components include surfactants other than the oily component having a polyoxyethylene structure (for example, lauroyl-L-carnitine), stabilizers, preservatives, antioxidants, disintegrants, excipients, binders, fluidizers, and lubricants.

Since the formulation according to the present embodiment comprises the compound and the oily component having a polyoxyethylene structure in a specific concentration, thereby enhancing the oral absorbability of the compound in a versatile manner, it may not comprise any surfactant other than the oily component having a polyoxyethylene structure. More specifically, the formulation according to the present embodiment may not comprise lauroyl-L-carnitine.

The components contained in the formulation according to the present embodiment may be substantially composed of water, the compound, the oily component having a polyoxyethylene structure, and DMSO, or may be composed only of water, the compound, the oily component having a polyoxyethylene structure, and DMSO. As used herein, the term "substantially" means that the components listed therein (examples include, but are not limited to, water, the compound, the oily component having a polyoxyethylene structure, and DMSO) are the main components, and other components may be contained as long as they are components that do not negatively affect the effects in one aspect of the present invention, or in amounts or in aspects that do not give negative influence. For example, components not listed therein (for example, ethanol and the like) may be contained as long as they are components that do not negatively affect the effects in one aspect of the present invention, or in amounts or in aspects that do not give negative influence.

There are no particular restrictions on the dosage form of the formulation according to the present embodiment, and it is typically a liquid formulation.

Since the formulation according to the present embodiment has enhanced oral absorbability of the compound, it is preferably made into an oral formulation.

There are no particular restrictions on the subject to which the formulation according to the present embodiment is administered, and it may be a human or a non-human animal. Examples of the non-human animal include dog, monkey, mini-pig, rabbit, rat, and mouse. The subject is preferably a non-human animal, a dog, a monkey, a mini-pig, a rabbit, a rat, and a mouse are more preferred, a rat and a mouse are still more preferred, and a mouse is even more preferred.

There are no particular restrictions on the method for administering the formulation according to the present embodiment, and it may be orally administered or parenterally administered. Meanwhile, since the formulation according to the present embodiment has enhanced oral absorbability of the compound, it is preferably orally administered.

There are no particular restrictions on the dosage of the formulation according to the present embodiment, and it may be administered such that the dose of the compound to be administered per body weight (kg) of the subject is 0.1 mg/kg or more and 1000 mg/kg or less, for example. The dose of the compound to be administered may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

The formulation according to the present embodiment can be obtained by a production method comprising compounding a compound and an oily component having a polyoxyethylene structure, wherein the oily component having a polyoxyethylene structure is compounded such that the content is 2.0% by volume or more and 7.5% by volume or less based on 100% by volume of a formulation.

More specifically, such a production method may comprise mixing a carrier such as an aqueous medium such as water and/or DMSO, the compound, and the oily component having a polyoxyethylene structure, by stirring or other means. Also, the above-described other components may be further added, if necessary.

In the formulation according to the present embodiment, the oily component having a polyoxyethylene structure can be regarded as exerting a promoting effect on absorption of the compound, and can be said to be an active component that can exert a promoting effect on absorption of the compound. Similarly, the oily component having a polyoxyethylene structure can also be regarded as exerting an improving effect on solubility of the compound, and can be said to be an active component that can exert an improving effect on solubility of the compound. Accordingly, there is provided, as one aspect of the present invention, an agent for promoting absorption of a compound, comprising an oily component having a polyoxyethylene structure as an active component, wherein the agent is used such that the content of the oily component is 2.0% by volume or more and 7.5% by volume or less based on 100% by volume of a formulation containing the compound. Similarly, there is provided, as another aspect of the present invention, an agent for improving solubility of a compound, comprising an oily component having a polyoxyethylene structure as an active component, wherein the solubility improving agent is used such that the content of the oily component is 2.0% by volume or more and 7.5% by volume or less based on 100% by volume of a formulation containing the compound.

From the above viewpoint, it is preferable that the formulation according to the present embodiment is one providing a promoted absorption of the compound compared to the formulation not comprising the oily component having a polyoxyethylene structure. It is also preferable that the formulation according to the present embodiment is one providing an improved solubility of the compound compared to the formulation not comprising the oily component having a polyoxyethylene structure. Here, the "formulation not comprising the oily component having a polyoxyethylene structure" to be compared means a formulation having compositional features in which the oily component having a polyoxyethylene structure is replaced with a carrier (for example, water) in the formulation according to the present embodiment.

Since the formulation according to the present embodiment has enhanced oral absorbability of the compound in a versatile manner, it can be suitably used for screening a candidate compound. Examples of the candidate compound include, among others, candidate compounds for a medicament (clinical candidate compounds). Next, a method for screening a candidate compound using the formulation according to the present embodiment will be described.

(Method for screening candidate compound)

A method for screening a candidate compound according to the present embodiment (hereinafter, also referred to simply as "screening method") comprises administering to a subject the above-described formulation according to the present invention. Note that, in the following description, the compound contained in the formulation is specifically referred to as "test compound".

The screening method according to the present embodiment may comprise selecting the candidate compound using at least one selected from the group consisting of the following (1), (2), and (3) as an indicator:

(1) drug efficacy;
(2) toxicity; and
(3) pharmacokinetic characteristics.

In the screening method according to the present embodiment, it is possible to measure the drug efficacy and/or toxicity of a test compound administered to a subject and select a test compound that is found to have drug efficacy or has high drug efficacy, or to select a test compound with low toxicity or no toxicity. The drug efficacy and/or toxicity of a test compound may be evaluated by, for example, comparing them with threshold values set in advance. It is also

possible to carry out the same measurement using a compound having drug efficacy as a comparative control and to select a test compound with higher drug efficacy or lower toxicity than such a control. There are no particular restrictions on the drug efficacy and examples thereof include inhibition of cell proliferation, cytotoxicity, and inhibition of tumor proliferation. There are no particular restrictions on the toxicity and examples thereof include hepatotoxicity, nephrotoxicity, and cardiotoxicity.

In the screening method according to the present embodiment, it is also possible to measure the blood concentration of a test compound in a subject to which the test compound has been administered, thereby evaluating the pharmacokinetic characteristics of the test compound. Here, the "pharmacokinetic characteristics" means characteristics such as the effective blood concentration, blood half-life, and/or rate of disappearance of a test compound in the body of a subject. The pharmacokinetic characteristics of a test compound may be evaluated by, for example, comparing them with threshold values set in advance. For example, usually, a substance with a higher effective blood concentration, a longer blood half-life, or a slower rate of disappearance is considered to have excellent pharmacokinetic characteristics, but this is not a limitation. Also, there are no particular limitations on the approach for measuring the blood concentration of a test compound.

The screening method according to the present embodiment may comprise selecting the candidate compound using (3) pharmacokinetic characteristics as an indicator.

In the screening method according to the present embodiment, the candidate compound may be selected from multiple test compounds. In this case, the multiple test compounds are each administered to a subject as a separate formulation. There are no particular restrictions on the number of test compounds, and it may be, for example, 2 or more, 3 or more, 4 or more, 5 or more, 6 or more, 7 or more, or 8 or more. Note that, since screening is to select those that match the conditions, the screening method according to the present embodiment can also be carried out for a single test compound.

In the screening method according to the present embodiment, there are no particular restrictions on a subject to which the formulation according to the present invention is administered, and it may be a non-human animal. Examples of the subject include dog, monkey, mini-pig, rabbit, rat, and mouse. The subject is preferably a rat or a mouse, and more preferably a mouse.

In the screening method according to the present embodiment, there are no particular restrictions on the method for administering the formulation according to the present invention, and it may be orally administered or parenterally administered. Meanwhile, since the formulation according to the present invention has enhanced oral absorbability of the compound, it is preferably orally administered.

In the screening method according to the present embodiment, there are no particular restrictions on the dosage of the formulation according to the present invention, and it may be administered such that the dose of the test compound to be administered per body weight (kg) of the subject is 0.1 mg/kg or more and 1000 mg/kg or less, for example. The dose of the test compound to be administered may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

[Method for comparing test compounds]

The method according to the present embodiment is a method for comparing multiple test compounds, comprising: administering to a subject the above-described formulation according to the present invention; and comparing multiple test compounds using at least one selected from the group consisting of the following (1), (2), and (3) as an indicator:

(1) drug efficacy;
(2) toxicity; and
(3) pharmacokinetic characteristics.

In the comparison method according to the present embodiment, the drug efficacy and/or toxicity of the test compounds administered to the subject are measured and compared among the test compounds. As a result, for example, a test compound with relatively high drug efficacy can be selected, or a test compound with relatively low toxicity can be selected. It is also possible to carry out the same measurement using a control compound having drug efficacy as a test compound and to select a test compound with higher drug efficacy or lower toxicity than such a control. There are no particular restrictions on the drug efficacy, and examples thereof include inhibition of cell proliferation, cytotoxicity, and inhibition of tumor proliferation. There are no particular restrictions on the toxicity, and examples thereof include hepatotoxicity, nephrotoxicity, and cardiotoxicity.

In the comparison method according to the present embodiment, it is also possible to measure the blood concentration of a test compound in a subject to which the test compound has been administered, thereby evaluating the pharmacokinetic characteristics of the test compound. Here, the "pharmacokinetic characteristics" means the concentration of the test compound in the body of the subject, and preferably means characteristics such as the effective blood concentration, blood half-life, and/or rate of disappearance of the test compound in the body of the subject. By comparing pharmacokinetic characteristics among the test compounds, it is possible to select a test compound with relatively excellent pharmacokinetic characteristics. It is also possible to carry out the same measurement using a control compound having drug efficacy as a test compound and to select a test compound with excellent pharmacokinetic characteristics compared to such a control. For example, usually a substance with a higher effective blood concentration, a longer blood half-life, or a slower rate of disappearance is considered to have excellent pharmacokinetic characteristics, but this is not a limitation. Also, there are no particular limitations on the approach for measuring the blood concentration of the test compound.

The comparison method according to the present embodiment may comprise comparing the test compounds using (3) pharmacokinetic characteristics as an indicator. There are no particular restrictions on the number of test compounds, and it may be, for example, 2 or more or 3 or more.

In the comparison method according to the present embodiment, there are no particular restrictions on a subject to which the formulation according to the present invention is administered, and it may be a non-human animal. Examples of the subject include dog, monkey, mini-pig, rabbit, rat and mouse. The subject is preferably a rat or a mouse and more preferably a mouse.

In the comparison method according to the present embodiment, there are no particular restrictions on the method for administering the formulation according to the present invention, and it may be orally administered or parenterally administered. Meanwhile, since the formulation according to the present invention has enhanced oral absorbability of the compound, it is preferably orally administered.

In the comparison method according to the present embodiment, there are no particular restrictions on the dosage of the formulation according to the present invention, and it may be administered such that the dose of the test compound to be administered per body weight (kg) of the subject is 0.1 mg/kg or more and 1000 mg/kg or less, for example. The dose of the test compound to be administered may be, for example, 1 mg/kg or more and 500 mg/kg or less, 1 mg/kg or more and 100 mg/kg or less, 1 mg/kg or more and 50 mg/kg or less, 3 mg/kg or more and 30 mg/kg or less, 10 mg/kg or more and 30 mg/kg or less, 0.1 mg/kg or more and 10 mg/kg or less, 1 mg/kg or more and 5 mg/kg or less, 10 mg/kg or more and 100 mg/kg or less, 15 mg/kg or more and 50 mg/kg or less, 20 mg/kg or more and 40 mg/kg or less, 25 mg/kg or more and 35 mg/kg or less, 3 mg/kg, or 30 mg/kg.

[Method for enhancing internal absorption of test compounds]

The method according to the present embodiment is a method for enhancing internal absorption of test compounds in a subject, comprising administering to the subject the above-described formulation according to the present invention. The internal absorption may be digestive absorption. Since the formulation according to the present invention has excellent oral absorbability of the compound, it can be suitably used in a method for enhancing internal absorption of the test compounds in the subject.

Examples

Hereinafter, the present invention will be described more specifically based on Examples. However, the present invention is not limited to the following Examples. All starting materials and reagents were obtained from commercial suppliers, or synthesized using known methods.

In Examples, the following abbreviations were used.

$BF_3OEt_2$: boron trifluoride diethyl ether complex
COMU: (1-cyano-2-ethoxy-2-oxoethylideneaminooxy)dimethylaminomorpholinocarbenium hexafluorophosphate
DBU: 1,8-diazabicyclo[5.4.0]-7-undecene
DCM: dichloromethane
DCE: 1,2-dichloroethane
DIAD: diisopropyl azodicarboxylate
DIC: N,N'-diisopropylcarbodiimide
DIPEA: N,N-diisopropylethylamine
DMA: dimethylacetamide
DMAP: N,N-dimethyl-4-aminopyridine
DMF: N,N-dimethylformamide

DMSO: dimethyl sulfoxide
dtbbpy: 4,4'-di-tert-butyl-2,2'-bipyridyl
EDTA: ethylenediaminetetraacetic acid
EtOAc: ethyl acetate
FA: formic acid
Fmoc: 9-fluorenylmethyloxycarbonyl group
Fmoc-OSu: N-(9-fluorenylmethylcarbonyloxy)succinimide
HATU: O-(7-aza-1H-benzotriazol-1-yl)-N,N,N',N'-tetramethyluronium hexafluorophosphate HOBt: 1-hydroxybenzotriazole
HBTU: O-(1H-benzotriazol-1-yl)-N,N,N',N'-tetramethyluronium hexafluorophosphate HMDS: 1,1,1,3,3,3-hexamethyldisilazane
H-SAR-OTBU HCl: sarcosine tert-butyl ester hydrochloride
IPAC: isopropyl acetate
MeOH: methyl alcohol
Ns: 2-nitrobenzenesulfonyl group
Oxyma: ethyl cyano(hydroxyimino)acetate
pip: piperidine
TBME: t-butyl methyl ether
TES: triethylsilane
TfOH: trifluoromethanesulfonic acid
TMSOTf: trimethylsilyl trifluoromethanesulfonate
THF: tetrahydrofuran
TsOH: p-toluenesulfonic acid
THP: tetrahydropyranyl group
Trt: triphenylmethyl group
WSC·HCl, WSCI: 1-ethyl-3-(3-dimethylaminopropyl)carbodiimide hydrochloride

[Example 1] Synthesis of cyclic peptide compounds

For the cyclic peptide compounds 1 to 10 (also referred to simply as compounds 1 to 10) shown in Table 2, elongation of the peptide was performed using the following basic route, in accordance with the peptide synthesis method by the Fmoc method described in International Publication No. WO 2013/100132 or International Publication No. WO 2018/225864. That is, the method included the following five steps:

1) a peptide elongation reaction from the N-terminus of an amino acid by the Fmoc method using one in which a carboxylic acid of the Asp side chain was supported on a 2-chlorotrityl resin;
2) an excising process of the peptide from the 2-chlorotrityl resin;
3) amide cyclization by condensation of the carboxylic acid of the Asp side chain derived from the 2-chlorotrityl resin by the excising process and the amino group at the N-terminus of the peptide chain;
4) deprotection of the protecting group of the side chain functional group contained in the peptide chain; and
5) purification of the compound by preparative HPLC.

In addition, all starting materials and reagents were obtained from commercial suppliers, or synthesized using known methods.

Synthesis of (S)-3-((((9H-fluoren-9-yl)methoxy)carbonyl)amino)-4-oxo-4-(piperidin-1-yl)butanoic acid-2-chlorotritylresin (Fmoc-Asp(O- Trt(2-Cl)-Resin)-pip)

[Formula 1]

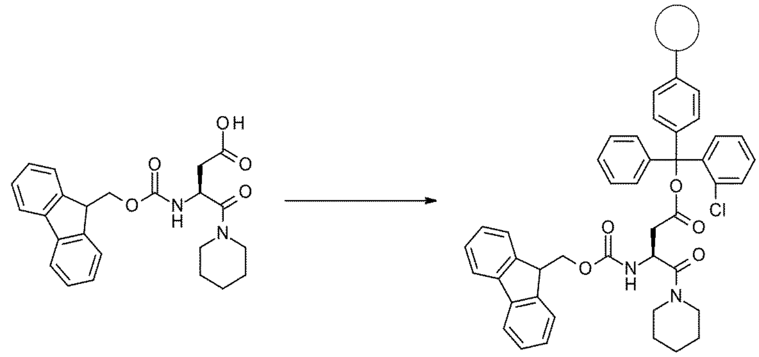

From (S)-3-((((9H-fluoren-9-yl)methoxy)carbonyl)amino)-4-oxo-4-(piperidin-1-yl)butanoic acid, synthesis was performed by the method described in International Publication No. WO 2013/100132.

Synthesis of compound 5

[Formula 2]

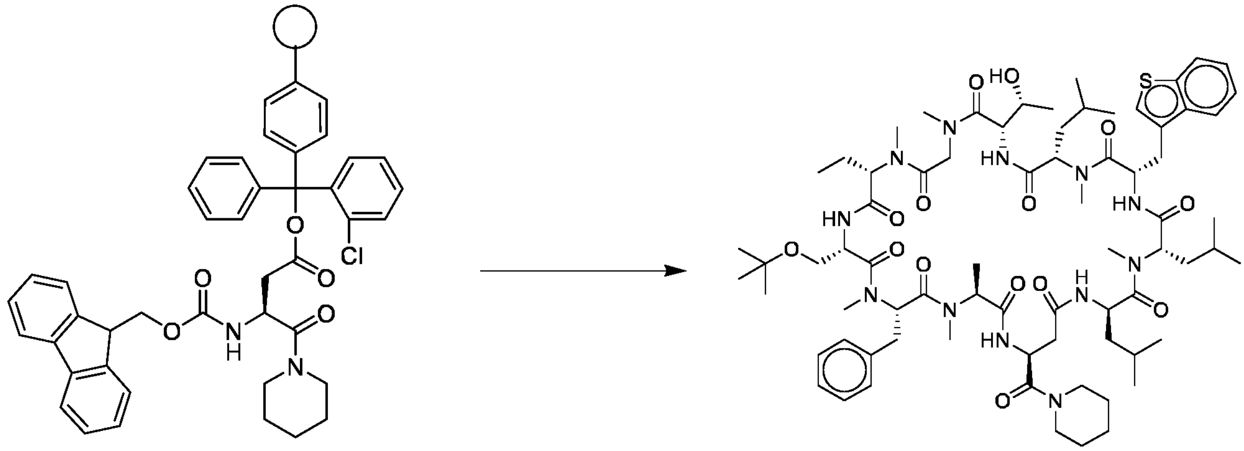

Using (S)-3-((((9H-fluoren-9-yl)methoxy)carbonyl)amino)-4-oxo-4-(piperidin-1-yl)butanoic acid-2-chlorotritylresin (Fmoc-Asp(O-Trt(2-Cl)-Resin)-pip) (0.269 mmol/g, 100 mg, 0.0269 mmol), the above-described peptide elongation reaction using Fmoc-MeAla-OH, Fmoc-MePhe-OH, Fmoc-Ser(tBu)-OH, Fmoc-MeAbu-OH, Fmoc-MeGly-OH, Fmoc-Thr(THP)-OH, Fmoc-MeLeu-OH, Fmoc-Ala(3-Bzt)-OH, and Fmoc-D-Leu-OH, cleavage of the elongated peptide from the resin, cyclization of the excised peptide (using O-(7-aza-1H-benzotriazol-1-yl)-N,N,N,N-tetramethyluronium hexafluorophosphate (HATU) as a cyclizing reagent), and purification of the cyclic peptide compound were performed, thereby obtaining the target compound 5 (29.72 mg).

Synthesis of compound 9

[Formula 3]

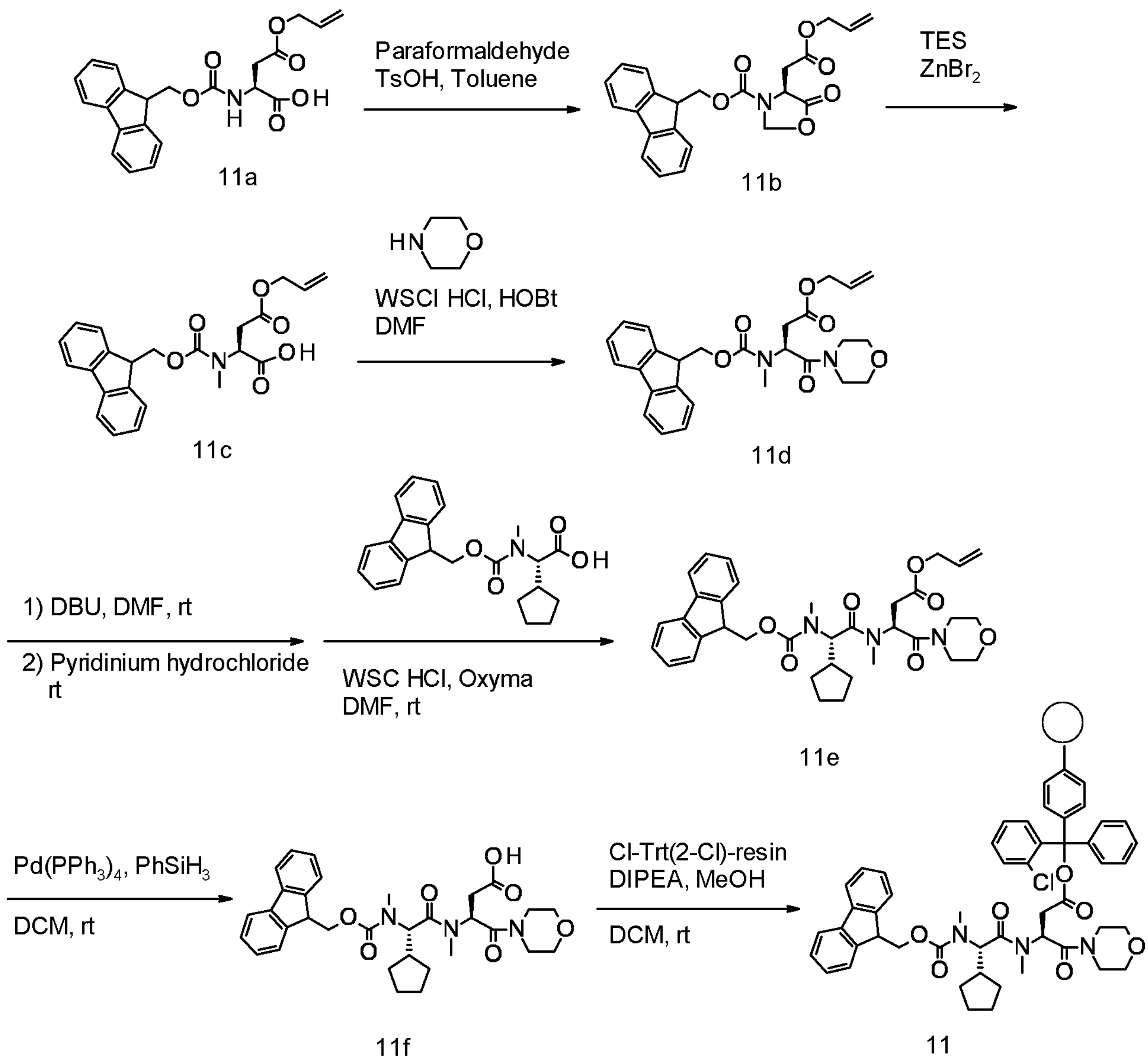


11a 11b 11c 11d 11e 11f 11

Fmoc-Asp(OAl)-OH (compound 11a, (2S)-2-(9H-fluoren-9-ylmethoxycarbonylamino)-4-oxo-4-prop-2-enoxybutanoic acid, CAS No. 146982-24-3) (200 g, 506 mmol), p-toluenesulfonic acid monohydrate (5.7 g, 0.05 equivalents), and paraformaldehyde (45.6 g, 3 equivalents) were mixed into toluene (2000 mL), and the mixture was stirred at 110°C for 16 hours. The solvent of the reaction solution was evaporated under reduced pressure, and the residue was dissolved in ethyl acetate and washed twice with an aqueous sodium hydrogen carbonate solution. The organic layer was dried over anhydrous sodium sulfate, and then the solvent was evaporated under reduced pressure. The residue was purified by silica gel column chromatography (ethyl acetate/petroleum ether, 0/100 to 30/70) to obtain the compound 11b (9H-fluoren-9-ylmethyl(4S)-5-oxo-4-(2-oxo-2-prop-2-enoxyethyl)-1,3-oxazolidine-3-carboxylate (175 g, 85%). Another batch that was similarly synthesized was mixed and used in the next reaction.

LCMS(ESI) m/z = 408 (M+H)+
Retention time: 1.407 minutes (analysis condition SMD method_20)

A mixed solution of the compound 11b (100 g, 245 mmol), zinc bromide (ZnBr2) (110 g, 496 mmol), and TES (56 g, 481.6 mmol) in DCM (1 L) was stirred under a nitrogen atmosphere at room temperature for 48 hours. Four batches of reaction solution at the same scale were mixed and the solvent was evaporated under reduced pressure. The residue was dissolved in TBME and extracted 10 times with a 0.5M phosphate buffer solution (pH = about 7.5). The aqueous layers were combined, adjusted to pH 2 with 5N hydrochloric acid, and extracted twice with isopropyl acetate (IPAC). The organic layers were combined, dried over anhydrous sodium sulfate, and the solvent was evaporated under reduced pressure. In order to remove IPAC, TBME was added to the obtained residue and the solvent was evaporated under

reduced pressure. This procedure was repeated 6 times to obtain the compound 11c ((2S)-2-[9H-fluoren-9-ylmethoxycarbonyl(methyl)amino]-4-oxo-4-prop-2-enoxybutanoic acid) (270 g, 54%).

LCMS(ESI) m/z = 410 (M+H)+
Retention time: 1.956 minutes (analysis condition SMD method_05)

WSCI·HCl (0.506 g, 2.64 mmol) was dissolved in DMF (4.4 mL), HOBt (0.356 g, 2.64 mmol) and the compound 11c ((2S)-2-[9H-fluoren-9-ylmethoxycarbonyl(methyl)amino]-4-oxo-4-prop-2-enoxybutanoic acid, Fmoc-MeAsp(OAl)-OH) (0.9 g, 2.2 mmol) were added, and the mixture was stirred at 0°C for 10 minutes. Morpholine (0.23 mL, 2.64 mmol) was added dropwise to the reaction solution, and the mixture was stirred at 0°C for 1 hour. To the reaction solution was added ethyl acetate (9 mL), and the mixture was washed with 0.5N hydrochloric acid, water, saturated aqueous sodium hydrogen carbonate solution/water (1/1), saturated saline/water (1/1), dried over anhydrous sodium sulfate, and then the solvent was evaporated under reduced pressure, thereby obtaining the compound 11d as a crude product (522 mg, 50%).

LCMS(ESI) m/z = 479 (M+H)+
Retention time: 0.87 minutes (analysis condition SQDFA05)

Under a nitrogen atmosphere, DBU (3.15 mL, 20.90 mmol) was added dropwise to a solution of the compound 11d (10 g, 20.90 mmol) in dehydrated DMF (50 mL) at room temperature and stirred for 10 minutes. To the reaction mixture was added pyridine hydrochloride (2.66 g, 22.99 mmol) at room temperature, and the mixture was stirred for 10 minutes. To this reaction mixture was added a separately prepared active ester solution (described later) at room temperature, followed by dropwise addition of DIPEA (4.01 mL, 22.99 mmol). The obtained reaction mixture was stirred at room temperature for 4 hours and 15 minutes, and ethyl acetate (100 mL), hexane (20 mL), and 1N hydrochloric acid (100 mL) were then added. The obtained mixture was extracted with ethyl acetate-hexane (5:1) (total amount of organic phase: about 300 mL), the organic phase was washed with 1N hydrochloric acid (100 mL), water (100 mL), an aqueous sodium hydrogen carbonate solution (100 mL × 2), and saline (100 mL) and dried over sodium sulfate, and the solvent was then evaporated under reduced pressure. The obtained crude product was purified by normal phase silica gel chromatography (hexane-ethyl acetate). This was dissolved in TBME (100 mL), and washed with an aqueous potassium carbonate solution (1 w/v%, 50 mL). The organic phase was washed with saturated saline/water (1/1, 50 mL), dried over sodium sulfate, and then the solvent was evaporated under reduced pressure to obtain the compound 11e (9.98 g, 81%).

The active ester solution was prepared in the following manner.

Under a nitrogen atmosphere, dehydrated DMF (55 mL) was added to a mixture of (αS)-α-[[(9H-fluoren-9-ylmethoxy)carbonyl]methylamino]cyclopentaneacetic acid (7.53 g, 19.85 mmol), WSCI·HCl (5.21 g, 27.2 mmol), and Oxyma (3.56 g, 25.08 mmol) at room temperature, and the mixture was stirred for 40 minutes. The reaction mixture thus obtained was used as an active ester solution.

LCMS(ESI) m/z = 618 (M+H)+
Retention time: 0.96 minutes (analysis condition SQDFA05)

Under a nitrogen atmosphere, dehydrated DCM (15 ml) was added to a mixture of the compound 11e (9.90 g, 16.03 mmol) and tetrakis(triphenylphosphine)palladium(0) (0.185 g, 0.16 mmol), and the mixture was stirred at room temperature. To the obtained solution, phenylsilane (1.38 mL, 11.22 mmol) was added dropwise. After stirring the obtained reaction mixture for 30 minutes, TBME (100 mL) was added, and an aqueous sodium hydrogen carbonate solution (saturated aqueous solution diluted by 2 times) (100 mL) was then slowly added dropwise to stop the reaction. The obtained mixture was extracted twice with water (total amount of aqueous phase: about 150 mL), and to the aqueous phase were added DCM (100 mL) and phosphoric acid (5.6 mL). The obtained mixture was extracted twice with DCM (total amount of organic phase: about 200 mL), the organic phase was washed with saline (80 mL × 2) and dried over sodium sulfate, and the solvent was then evaporated under reduced pressure to obtain the compound 11f (9.57 g, quant.), which was used for the subsequent reaction.

LCMS(ESI) m/z = 578 (M+H)+
Retention time: 0.79 minutes (analysis condition SQDFA05)

A reaction vessel with a filter (400 mL) was charged with 2-chlorotrityl chloride resin (1.36 mmol/g, 20.5 g, 15.07 mmol) and DCM (140 mL), and the mixture was allowed to stand still at room temperature for 1 hour. After DCM was removed from the reaction vessel by applying nitrogen pressure, a DCM (140 mL) solution containing the compound 11f (9.50 g, 16.45 mmol), methanol (5.32 mL, 132 mmol), and DIPEA (13.8 mL, 79 mmol) was added to the reaction

vessel, and the reaction vessel was shaken at 25°C and 60 rpm for 60 minutes. After the reaction solution was removed from the reaction vessel by applying nitrogen pressure, a DCM (140 mL) solution of methanol (20 mL, 493 mmol) and DIPEA (13.8 mL, 79 mmol) was added to the reaction vessel, which was shaken at 25°C and 60 rpm for 60 minutes. After the reaction solution was removed by applying nitrogen pressure, DCM (140 mL) was put in and removed by applying nitrogen pressure after mixing. This washing operation of the resin with DCM was repeated a total of 5 times, and the obtained resin was dried under reduced pressure for one day to obtain the compound 11 (26.89 g).

The calculation of the amount of support of amino acids on the resin was carried out as follows, referring to the method described in the literature (Letters in Peptide Science, 2002, 9, 203).

The obtained compound 11 (10 mg) was put in a reaction vessel, DMF (2 mL) was added, and the mixture was allowed to stand still at room temperature for 1 hour. Thereafter, DBU (40 μL) was added to the reaction vessel, which was shaken at 25°C for 30 minutes. Thereafter, DMF (8 mL) was added to the reaction mixed solution, and 1 mL of the resulting solution was diluted with DMF (11.5 mL). The absorbance of the obtained diluted solution (294 nm) was measured (measured using Shimadzu, UV-1600 PC (cell length: 1.0 cm)), and the amount of support of the compound 11 was calculated to be 0.415 mmol/g.

[Formula 4]

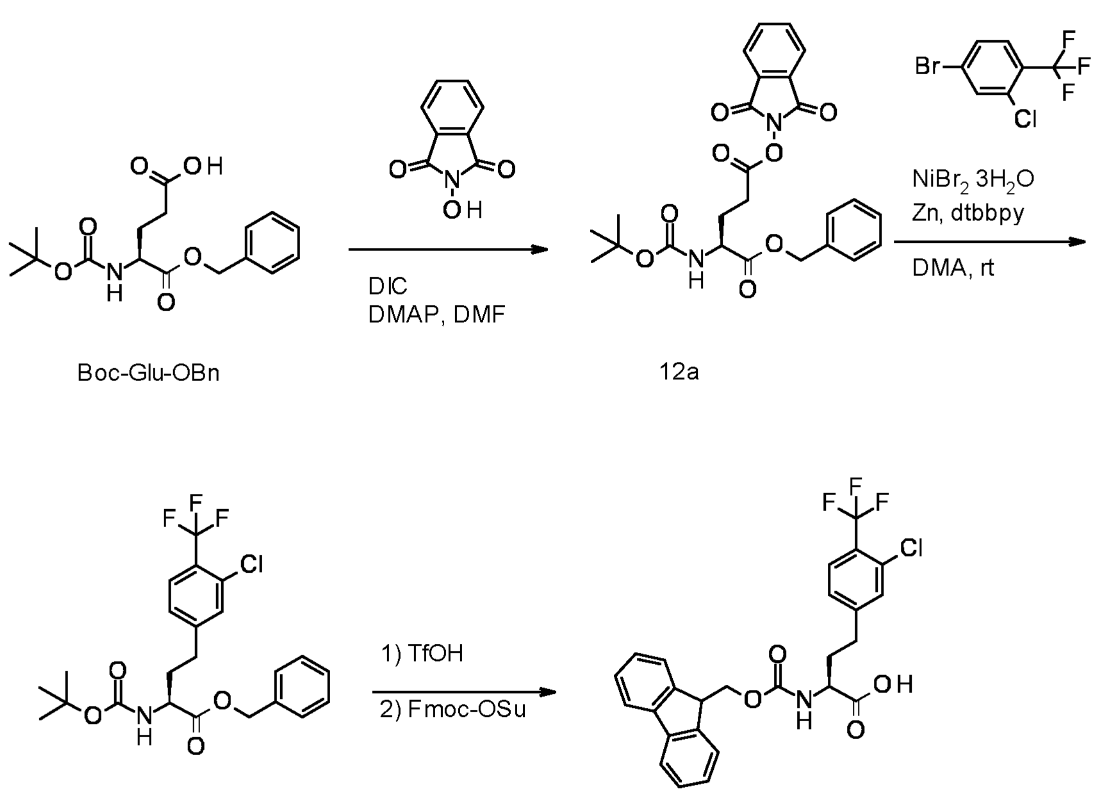


Boc-Glu-OBn

12a

12b

12

To a solution of (4S)-4-[(2-methylpropan-2-yl)oxycarbonylamino]-5-oxo-5-phenylmethoxypentanoic acid (Boc-Glu-OBn, CAS No. 30924-93-7) (200 g, 592.82 mmol), N-hydroxyphthalimide (106 g, 649.78 mmol, 1.10 equivalents), DMAP (3.6 g, 29.47 mmol, 0.05 equivalents) in THF (2 L), DIC (138 mL, 1.54 equivalents) was added dropwise under a nitrogen atmosphere at 0°C. The reaction solution was stirred at 25°C for 16 hours, then solid was removed by filtration, and the solvent of the filtrate was evaporated under reduced pressure. The residue was diluted with toluene, and the obtained solid was removed by filtration, and the solvent of the filtrate was evaporated under reduced pressure. The residue was purified by recrystallization (acetone/heptane) to obtain the compound 12a (1-O-benzyl-5-O-(1,3-dioxoisoindol-2-yl)(2S)-2[(2-methylpropan-2-yl)oxycarbonylamino]pentanedioate (230 g, 80%).

LCMS(ESI) m/z = 505.2 (M+Na)+
Retention time: 0.992 minutes (analysis condition SMD method_16)

Nickel bromide trihydrate ($NiBr_2 \cdot 3H_2O$) (4 g, 0.07 equivalents) and 4,4'-di-tert-butyl-2,2'-bipyridyl (dtbbpy, CAS

No. 72914-19-3) (3.9 g, 14.55 mmol, 0.07 equivalents) were added to DMA (500 mL), and the mixture was stirred under a nitrogen atmosphere at 50°C for 2 hours to prepare a nickel solution.

To a mixed solution of the compound 12a (100 g, 207.3 mmol), zinc powder (70 g, 5 equivalents), and 4-bromo-2-chloro-1-(trifluoromethyl)benzene (CAS No. 467435-07-0, 160 g, 617 mmol, 3 equivalents) in DMA (500 mL), the previously prepared nickel solution was added, and the mixture was stirred at 25°C for 16 hours. To the reaction solution, an aqueous EDTA·2Na solution (10%) was added, and the mixture was extracted with ethyl acetate. The combined organic layers were washed with saturated saline and then dried over anhydrous sodium sulfate, and the solvent was evaporated under reduced pressure. The obtained residue was purified by silica gel column chromatography (ethyl acetate/petroleum ether) to obtain the compound 12b (75 g, 77%).

LCMS(ESI) m/z = 494 (M+Na)+
Retention time: 2.863 minutes (analysis condition SMD method_17)

A solution of the compound 12b (75 g, 158.93 mmol) in toluene (900 mL) was cooled to 0°C, and trifluoromethanesulfonic acid (TfOH) (42 mL, 3.00 equivalents) was added dropwise. The mixture was stirred at room temperature for 1 hour, and water (75 mL) was then added. This mixed solution was extracted with water, and the combined aqueous layers were extracted with ethyl acetate. The combined organic layers were washed with water and dried over anhydrous sodium sulfate, and the solvent was then evaporated under reduced pressure. To the residue, acetonitrile/water (900/900 mL) was added, and the pH of the mixture was adjusted to 7 with an aqueous sodium hydroxide solution (48%). To this solution was added Fmoc-OSu (51.2 g, 151.93 mmol, 0.95 equivalents), and the mixture was stirred at room temperature for 16 hours while maintaining the pH at 7.8 to 8.0. The reaction solution was filtered, and the filtrate was adjusted to pH 2 with 6N hydrochloric acid. The precipitated solid was collected by filtration and dried at 50°C to obtain the compound 12 (Fmoc-Hph (4-$CF_3$-3-Cl)-OH) (70 g, 87%).

LCMS(ESI) m/z = 526 (M+Na)+
Retention time: 2.180 minutes (analysis condition SMD method_21)

[Formula 5]

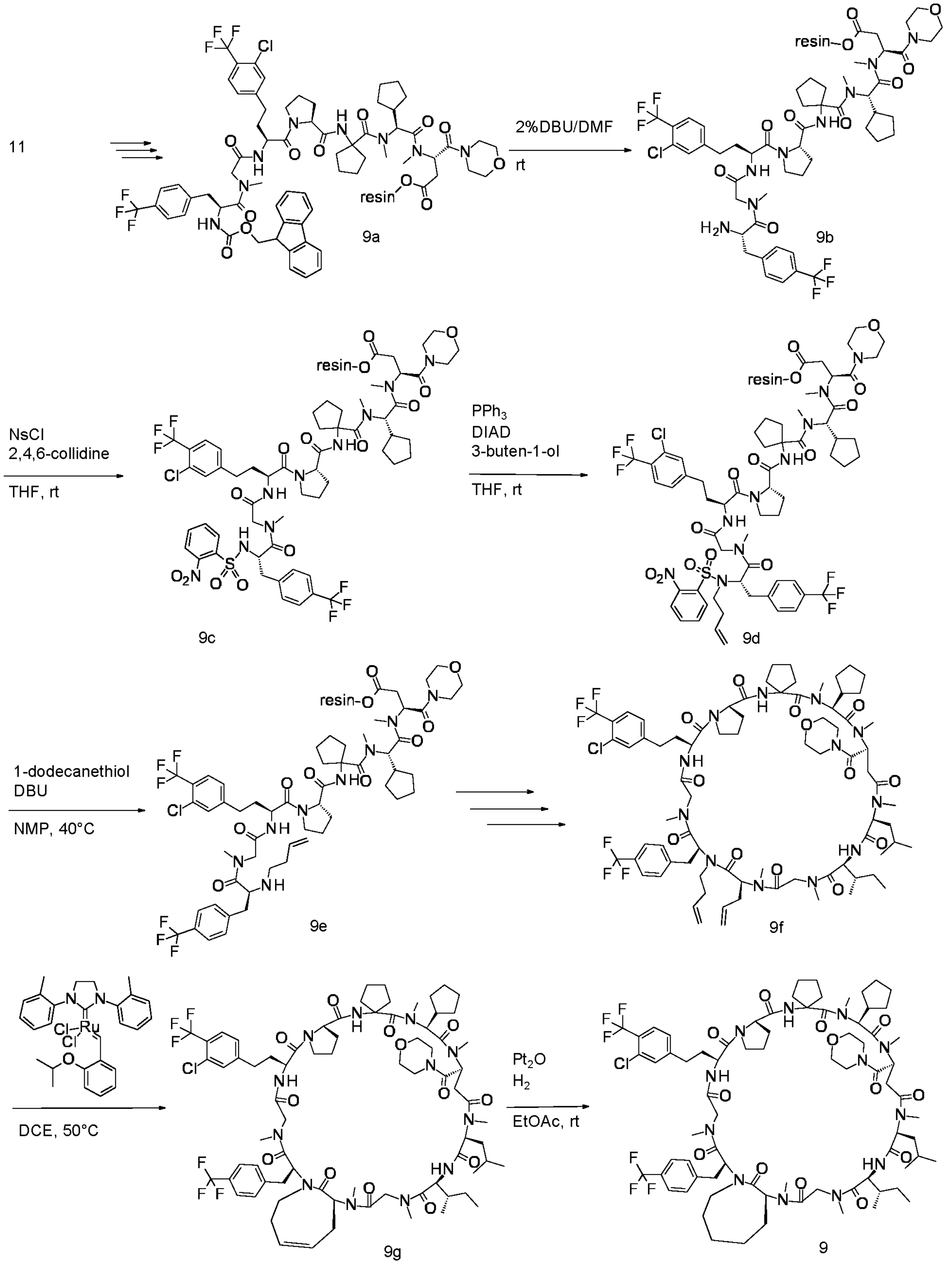


Using the compound 11, the peptide elongation reaction was performed in the same manner as in Example 1 using Fmoc-Phe(4-$CF_3$)-OH, Fmoc-MeGly-OH, the compound 12 (Fmoc-Hph(4-$CF_3$-3-Cl)-OH), Fmoc-Pro-OH, and Fmoc-cLeu-OH to obtain the compound 9a supported on the resin.

A reaction vessel with a filter was charged with the obtained compound 9a supported on the resin (4.2 g) and

DCM (45 mL) to swell the resin. After removing the DCM from the reaction vessel, the resin was washed twice with DMF (30 mL). A solution of DBU in DMF (2% v/v, 30 mL) was added and the mixture was shaken at room temperature for 10 minutes, after which the reaction solution was removed from the reaction vessel. The resin remaining in the reaction vessel was washed 4 times with NMP (30 mL), washed with a solution of triethylamine hydrochloride (520 mg, 3.78 mmol) in DCM (30 mL), washed 4 times with DCM (30 mL), and washed 4 times with THF (30 mL) to obtain the compound 9b supported on the resin.

To the reaction vessel in which the obtained compound 9b supported on the resin was placed, a solution of nosyl chloride (1.675 g, 7.56 mmol) in THF (27.5 mL) and 2,4,6-collidine (2.5 mL, 18.9 mmol) were added, and the reaction vessel was shaken at room temperature for 2 hours. Thereafter, the reaction solution was removed, and the resin was washed 4 times with THF (30 mL) and washed 4 times with DCM (30 mL) to obtain the compound 9c supported on the resin.

Using 2 g of the obtained compound 9c supported on the resin, DCM (20 mL) was added to swell the resin. After removing DCM, the resin was washed 4 times with THF (14 mL). A solution of triphenylphosphine (1.18 g, 4.50 mmol) in THF (7 mL) and a solution of DIAD (0.876 mL, 4.50 mmol) in THF (7 mL) were mixed and allowed to stand still for 15 minutes, to which 3-buten-1-ol (0.773 mL, 9.00 mmol) was added, and the mixture was allowed to stand still for 5 minutes. This was added to the above-described resin, the mixture was shaken at room temperature for 45 minutes, and then the reaction solution was removed. The resin was washed 5 times with THF (14 mL) and washed 5 times with DCM (14 mL) to obtain the compound 9d supported on the resin.

DCM (20 mL) was added to the compound 9d supported on the resin to swell the resin. After removing DCM, the resin was washed 4 times with THF (14 mL). A solution of DBU (0.678 mL, 4.50 mmol) in NMP (7 mL) and a solution of 1-dodecanethiol (2.05 mL, 0.262 mmol) in NMP (7 mL) were added, and the mixture was shaken at 40°C for 1 hour. Thereafter, the reaction solution was removed, and the resin was washed 5 times with DMF (14 mL) and washed 5 times with DCM (14 mL) to obtain the compound 9e supported on the resin.

Using the obtained 9e supported on the resin, the peptide elongation reaction using Fmoc-MeLeu-OH, Fmoc-Ile-OH, Fmoc-MeGly-OH, and Fmoc-MeAlgly-OH, cleavage of the elongated peptide from the resin, cyclization of the excised peptide (using (1-cyano-2-ethoxy-2-oxoethylideneaminooxy)dimethylamino-morpholino-carbenium hexafluorophosphate (COMU) as a cyclizing reagent), and purification of the cyclic peptide compound were performed in the same manner as in Example 1, thereby obtaining the compound 9f.

The compound 9f (78 mg, 0.050 mmol) and the Stewart-Grubbs catalyst (7.1 mg, 0.012 mmol) were dissolved in DCE (10 mL), degassed, and then stirred under a nitrogen atmosphere at 50°C for 6 hours. The Stewart-Grubbs catalyst (7.1 mg, 0.012 mmol) was added, and the mixture was degassed and stirred under a nitrogen atmosphere at 50°C for further 15 hours, and then the solvent was evaporated under reduced pressure. This was purified by reversed phase chromatography (acetonitrile with 0.1% formic acid/distilled water with 0.1% formic acid) to obtain the compound 9g (18 mg, 23%).

The compound 9g (16 mg, 0.010 mmol) was dissolved in ethyl acetate (1 mL), platinum(IV) oxide (1.6 mg, 0.007 mmol) was added, and the mixture was stirred under a hydrogen atmosphere at room temperature for 4 hours. Platinum(IV) oxide was filtered off, and the obtained filtrate was evaporated under reduced pressure. The obtained crude product was purified by reversed phase chromatography (acetonitrile with 0.1% formic acid/distilled water with 0.1% formic acid) to obtain the compound 9 (10 mg, 65%).

Synthesis of compound 10

[Formula 6]

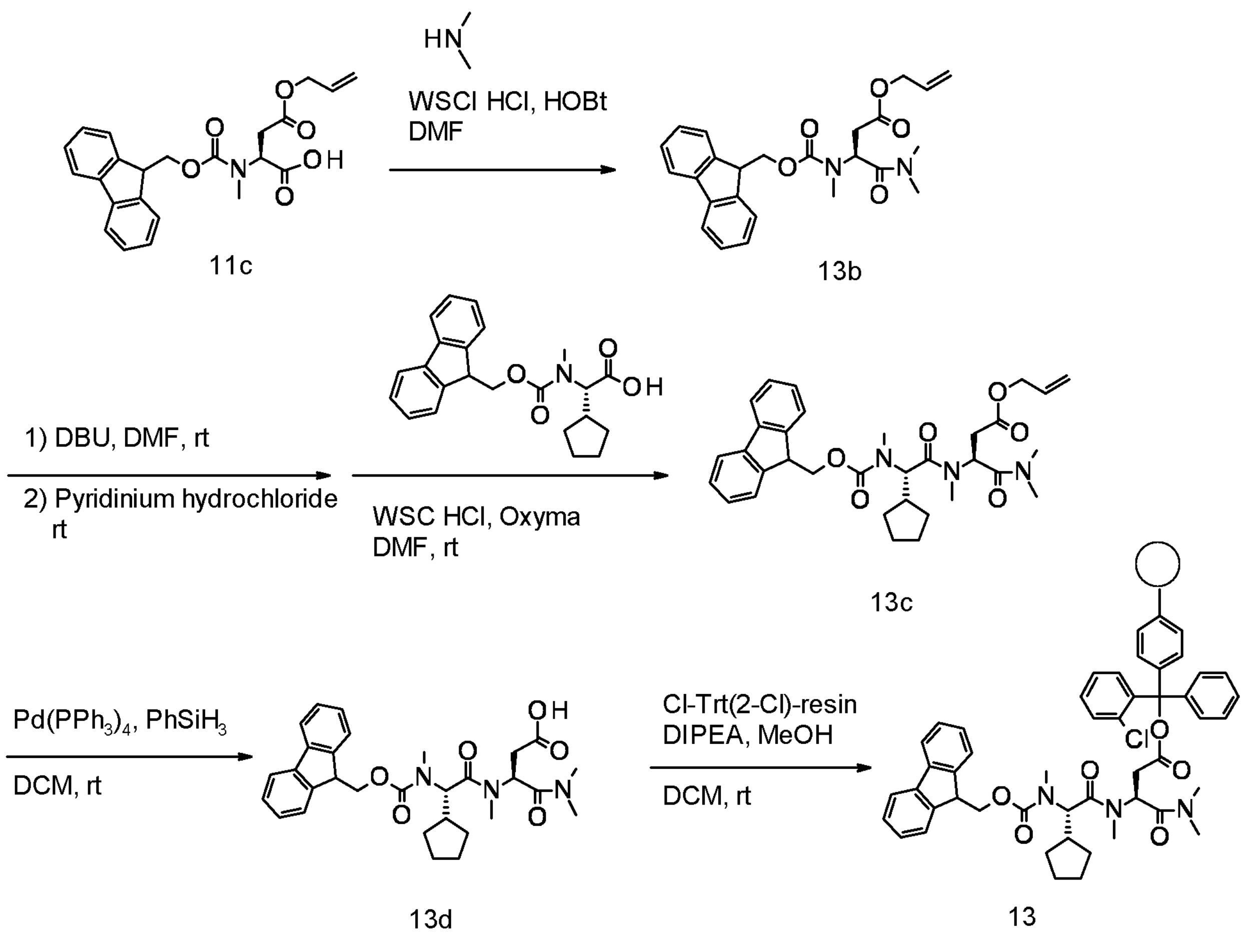


11c 13b 13c 13d 13

The compound 11c and ((2S)-2-[9H-fluoren-9-ylmethoxycarbonyl(methyl)amino]-4-oxo-4-prop-2-enoxybutanoic acid) (1.5 g, 3.66 mmol) were used as the starting materials, and by the same approach as for synthesis of the compound 11d, the compound 13b was obtained (1.47 g, 92%) by using a solution of dimethylamine in THF (2 mol/L) instead of morpholine.

LCMS(ESI) m/z = 437 (M+H)+
Retention time: 0.88 minutes (analysis condition SQDFA05)

Using the compound 13b as the starting material, by the same approach as for synthesis of the compound 11e, the compound 13c was obtained (5.0 g, 43%).

LCMS(ESI) m/z = 576.5 (M+H)+
Retention time: 1.02 minutes (analysis condition SQDFA05)

Using the obtained compound 13c, under the same reaction conditions as for synthesis of the compound 11f, the compound 13d supported on the resin was obtained (0.786 g, 92%).

LCMS(ESI) m/z = 536 (M+H)+
Retention time: 0.85 minutes (analysis condition SQDFA05)

Using the obtained compound 13d, under the same reaction conditions as for synthesis of the compound 11, the compound 13 was obtained (2.72 g). In the same manner as for the compound 11, the amount of support was calculated to be 0.345 mmol/g.

[Formula 7]

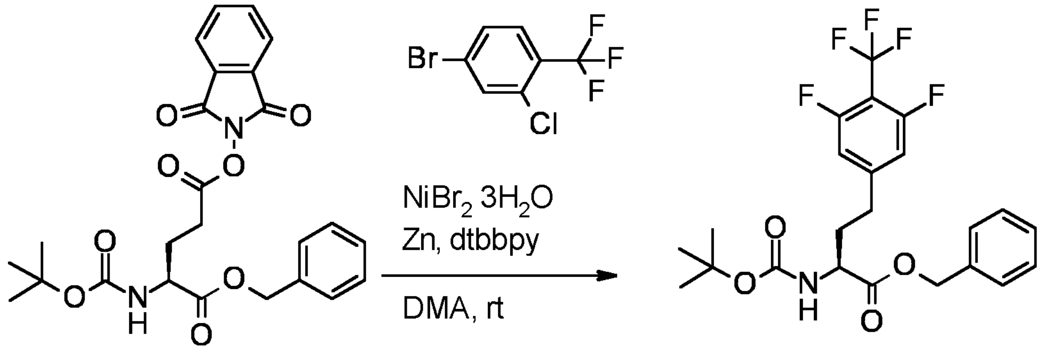


12a

14a

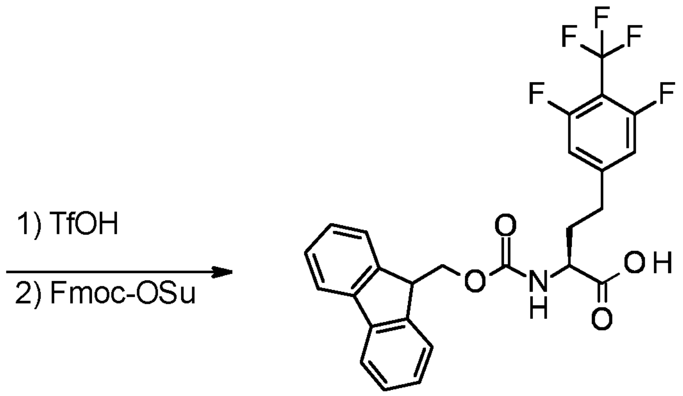


14

Nickel bromide trihydrate ($NiBr_2 \cdot 3H_2O$) (13.5 g, 49.7 mmol, 0.3 equivalents) and 4,4'-di-tert-butyl-2,2'-bipyridyl (dtbbpy, CAS No. 72914-19-3) (13.3 g, 49.7 mmol, 0.3 equivalents) were added to DMA (400 mL), and the mixture was stirred under a nitrogen atmosphere at 50°C for 3 hours to prepare a nickel solution.

A mixed solution of the compound 12a (1-O-benzyl-5-O-(1,3-dioxoisoindol-2-yl)(2S)-2[(2-methylpropan-2-yl)oxycarbonylamino]pentanedioate) (80 g, 166 mmol), zinc powder (54.2 g, 829 mmol, 5 equivalents), and 4-bromo-1,3-difluoro-2-(trifluoromethyl)benzene (CAS No. 156243-64-0, 86.6 g, 332 mmol, 2 equivalents) in DMA (400 mL) was stirred under a nitrogen atmosphere at room temperature for 1 hour, the previously prepared nickel solution was added, and the mixture was stirred at room temperature for 16 hours. An aqueous EDTA·2Na solution (800 mL, 10%) was added to the reaction solution, and the solid was removed by filtration. The filtrate was extracted with ethyl acetate, the combined organic layers were washed with saturated saline and then dried over anhydrous sodium sulfate, and the solvent was evaporated under reduced pressure. The obtained residue was purified by silica gel column chromatography (ethyl acetate/petroleum ether) to obtain the compound 14a (57.2 g, 69%).

LCMS(ESI) m/z = 496 (M+Na)+
Retention time: 1.544 minutes (analysis condition SMD method_15)

A mixed solution of the compound 14a (57.2 g, 121 mmol) in toluene (690 mL) was cooled to 0°C, and trifluoromethanesulfonic acid (TfOH) (54.4 g, 362 mmol, 3 equivalents) was added dropwise. The mixture was stirred at room temperature for 1 hour, and water (58 mL) was then added. This mixed solution was extracted with water, and the combined aqueous layers were extracted with ethyl acetate. The combined organic layers were washed with water and dried over anhydrous sodium sulfate, and the solvent was then evaporated under reduced pressure to obtain 60 g of the residue. To the residue, acetonitrile/water (400/400 mL) was added, and the pH of the mixture was adjusted to 7 with an aqueous sodium hydroxide solution (48%). Fmoc-OSu (36.6 g, 108.6 mmol, 0.9 equivalents) was added to this solution, the pH was adjusted to 8.0 with an aqueous sodium hydroxide solution (48%), and then the mixture was stirred at room temperature for 16 hours. The reaction solution was filtered while washing with acetonitrile/water (1/1) to remove the solid components. The filtrate was diluted with acetonitrile and acidified with 6N hydrochloric acid, and the precipitated

solid was collected by filtration to obtain the compound 14 (52 g, 83%).

LCMS(ESI) m/z = 528 (M+Na)+
Retention time: 3.538 minutes (analysis condition SMD method_14)

[Formula 8]

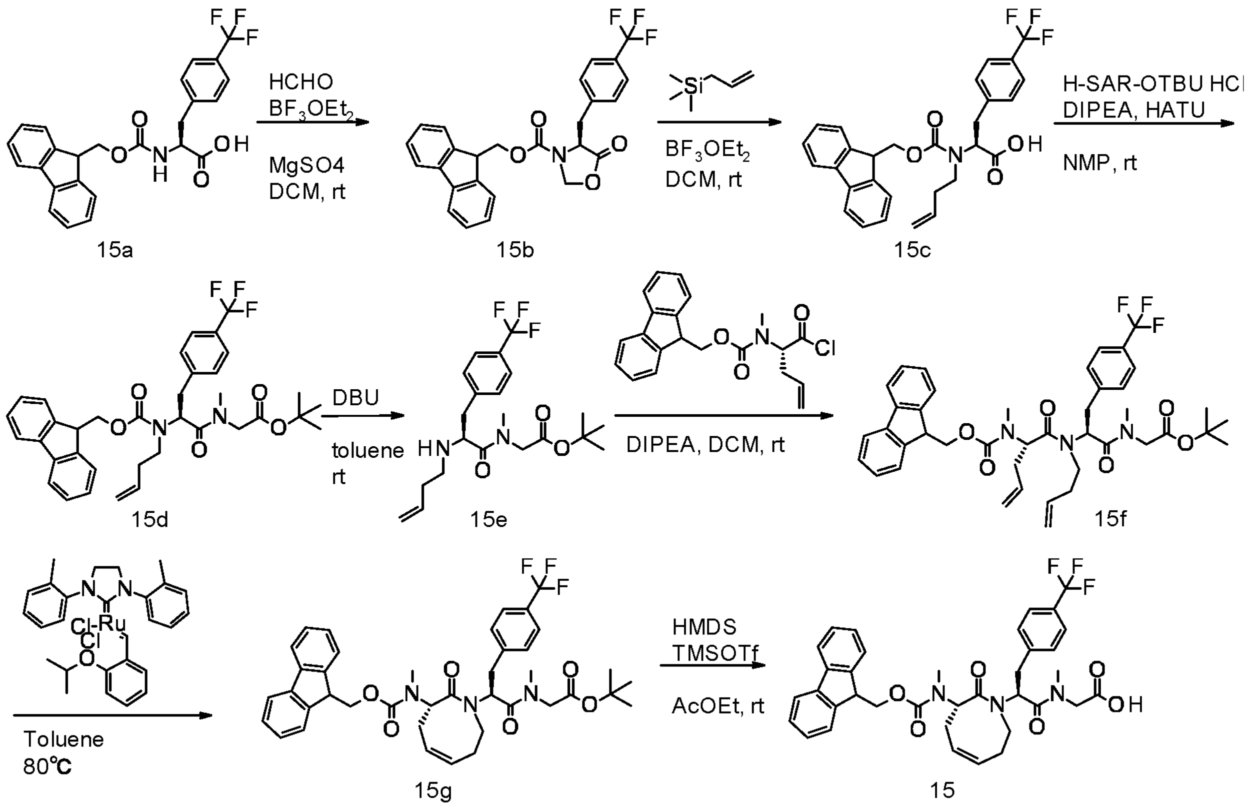


15a 15b 15c 15d 15e 15f 15g 15

To a solution of the compound 15a (N-([(9H-fluoren-9-yl)methoxy]carbonyl)-4-(trifluoromethyl)-L-phenylalanine) (10.0 g, 22.0 mmol) in dichloromethane (200 mL), magnesium sulfate (6.61 g, 54.9 mmol) and paraformaldehyde (1.98 g, 65.9 mmol) were added at room temperature, and then boron trifluoride diethyl ether complex (2.78 mL, 22.0 mmol) was added dropwise. After stirring the reaction mixture at room temperature for 1 hour, the reaction solution was filtered through silica gel (24 g) and then concentrated under reduced pressure to obtain the crude product 15b (10.5 g, quant.).

LCMS(ESI) m/z = 468 (M+H)+
Retention time: 1.45 minutes (analysis condition SMD method_04)

The obtained crude product 15b (10.3 g, 22.0 mmol) was dissolved in dichloromethane (200 mL), allyltrimethylsilane (12.2 mL, 77.0 mmol) was added under a nitrogen atmosphere, and then boron trifluoride diethyl ether complex (7.0 mL, 54.9 mmol) was added dropwise. After stirring the reaction mixture at room temperature for 6 hours, allyltrimethylsilane (12.2 mL, 77.0 mmol) and boron trifluoride diethyl ether complex (7.0 mL, 54.9 mmol) were added, and the mixture was stirred for 3 days. Water (40 mL) was added to the reaction solution, the mixture was stirred at room temperature for 10 minutes and then filtered through Celite, the filtrate was concentrated under reduced pressure, and the resulting residue was dissolved in toluene (24 mL). The organic layer was washed with water (48 mL), then hexane (100 mL) was added, and the mixture was extracted with a mixed solution of a 3.5% aqueous potassium hydrogen carbonate solution and acetonitrile (2:1, 300 mL). To the obtained aqueous layer, phosphoric acid was added until reaching pH 3, and the mixture was then extracted with ethyl acetate (200 mL). The organic layer was washed with saturated saline (100 mL), dried over anhydrous magnesium sulfate, and then filtered. The obtained solution was concentrated under reduced pressure, thereby obtaining the compound 15c (10.3 g, 92%).

LCMS(ESI) m/z = 510 (M+H)+
Retention time: 1.00 minutes (analysis condition SMD method_08)

To a solution of the compound 15c (67.5 g, 132 mmol) and sarcosine tert-butyl ester hydrochloride (25.3 g, 139 mmol) in NMP (379 mL), HATU (60.4 g, 159 mmol) was added and then DIPEA (69.2 mL, 397 mmol) was added dropwise. The reaction mixture was stirred at room temperature for 1.5 hours, then toluene (1.0 L, 15 v/w) was added, and the organic layer was washed with water (473 mL, 7 v/w), a 3.5% aqueous potassium hydrogen carbonate solution (473 mL, 7 v/w), and a 1M aqueous potassium dihydrogen phosphate solution (473 mL, 7 v/w). The organic layer was dried by passing it through an anhydrous magnesium sulfate pad, washed with toluene (67.5 mL, 1 v/w), and the obtained filtrate and washing solution were collectively regarded as the compound 15d and used in the next reaction as a solution.

LCMS(ESI) m/z = 637 (M+H)+
Retention time: 1.66 minutes (analysis condition SMD method_10)

To the above-described solution of the compound 15d (132 mmol) in toluene, DBU (1.97 mL, 13.2 mmol) was added at room temperature. After stirring the reaction mixture at room temperature for 17 hours, it was washed with a 1M aqueous potassium dihydrogen phosphate solution (420 mL, 5 v/w). The obtained organic layer was diluted with n-hexane (840 mL, 10 v/w), and extracted twice with a mixed solution of 2M hydrochloric acid (99 mL, 198 mmol), water (840 mL, 10 v/w), and acetonitrile (588 mL, 7 v/w). To the obtained aqueous layer, a 3.3M aqueous potassium phosphate solution was added to reach pH 9.5, and the mixture was extracted with ethyl acetate (840 mL, 10 v/w). The organic layer was concentrated under reduced pressure, and the obtained residue was dissolved in ethyl acetate (840 mL, 10 v/w), washed with saline (420 mL, 5 v/w), dried over anhydrous magnesium sulfate, and filtered. The obtained solution was concentrated under reduced pressure, thereby obtaining the compound 15e (49.0 g, 90%).

LCMS(ESI) m/z = 415 (M+H)+
Retention time: 1.31 minutes (analysis condition SMD method_10)

To a solution of the compound 15e (49.0 g, 118 mmol) in dichloromethane (276 mL), DIPEA (72.3 mL, 414 mmol) was added. The obtained mixture was added dropwise over 30 minutes to a separately prepared solution of (9H-fluoren-9-yl)methyl (S)-(1-chloro-1-oxopent-4-en-2-yl)(methyl)carbamate (described later) in dichloromethane (276 mL). After stirring the obtained reaction mixture at room temperature for 17 hours, the organic layer was washed with a 5% aqueous sodium dihydrogen phosphate (500 mL, 10 v/w), dried over anhydrous magnesium sulfate, and then filtered, and the solution was concentrated under reduced pressure. The obtained crude product was purified by normal phase silica gel chromatography (n-hexane-ethyl acetate), thereby obtaining the compound 15f (45.0 g, 51%).

(9H-Fluoren-9-yl)methyl (S)-(1-chloro-1-oxopent-4-en-2-yl)(methyl)carbamate was prepared as follows.

To a solution of (S)-2-((((9H-fluoren-9-yl)methoxy)carbonyl)(methyl)amino)pent-4-enoic acid (41.6 g, 118 mmol) in dichloromethane (296 mL), thionyl chloride (21.6 mL, 296 mmol) was added, and then DMF (0.92 mL, 11.8 mmol) was added dropwise. The obtained reaction mixture was stirred at room temperature for 15 minutes, and then concentrated under reduced pressure. To the obtained residue was added toluene (20 mL, 5 v/w), and the mixture was concentrated under reduced pressure. The obtained crude product was dissolved in dichloromethane (276 mL), which was used for the above-described reaction.

LCMS(ESI) m/z = 748 (M+H)+
Retention time: 1.71 minutes (analysis condition SMD method_10)

A solution of the compound 15f (16.7 g, 22.3 mmol), benzoquinone (0.24 g, 2.23 mmol), and the Stewart-Grubbs catalyst (0.51 g, 0.89 mmol) in dichloroethane (891 mL) was degassed 5 times, and stirred under a nitrogen atmosphere at 80°C for 4 hours. The reaction mixture was cooled to room temperature and then concentrated under reduced pressure, and the obtained crude product was purified by normal phase silica gel column chromatography (hexane-ethyl acetate), thereby obtaining the compound 15g (33.9 g, 39%).

LCMS(ESI) m/z = 720 (M+H)+
Retention time: 1.00 minutes (analysis condition SMD method_07)

Under a nitrogen atmosphere, hexamethyldisilazane (24.7 mL, 118 mmol) was added to a solution of the 15g (33.9 g, 47.1 mmol) in ethyl acetate (236 mL), and trimethylsilyl trifluoromethanesulfonate (17.0 mL, 94 mmol) was then added dropwise while cooling in a water bath (25°C). The reaction mixture was stirred at room temperature for 1.5 hours, then a 5% aqueous disodium hydrogen phosphate solution (340 mL, 10 v/w) was added while cooling in an ice bath to

stop the reaction. To the obtained mixture, water (170 mL, 5 v/w) and phosphoric acid were added, and the aqueous layer was adjusted to pH 3. The separated organic layer was washed with saturated saline (340 mL, 10 v/w), dried over anhydrous magnesium sulfate, and then filtered, and the solution was concentrated under reduced pressure. The obtained crude product was purified by reversed phase column chromatography (acetonitrile with 0.1% formic acid/distilled water with 0.1% formic acid), and the collected fractions were purified by reversed phase column chromatography (acetonitrile/distilled water), thereby obtaining the compound 15 (22.4 g, 72%).

LCMS(ESI) m/z = 664 (M+H)+
Retention time: 0.63 minutes (analysis condition SMD method_06)

Using the compound 13, the peptide elongation reaction using Fmoc-MeAbu-OH, Fmoc-Ile-OH, Fmoc-MeGly-OH, the compound 15, the compound 14 (Fmoc-Hph(4-$CF_3$-3,5-F2)-OH), Fmoc-Hyp(Et)-OH, and Fmoc-cVal-OH, cleavage of the elongated peptide from the resin, cyclization of the excised peptide (using (1-cyano-2-ethoxy-2-oxoethylideneaminooxy)dimethylamino-morpholino-carbenium hexafluorophosphate (COMU) as a cyclizing reagent), and purification of the cyclic peptide compound were performed in the same manner as in Example 1, thereby obtaining the compound 10.

Table 1 provides the abbreviations of amino acids. As used herein, when -OH is described in addition to the abbreviation of an amino acid in Table 1, it means that the C-terminal of that amino acid is a carboxyl group. For example, Gly-OH means that the C-terminal of glycine is a carboxyl group, and Fmoc-Gly-OH means that the N-terminal amino group of glycine is protected by a Fmoc group and the C-terminal is a carboxyl group.

[Table 1]

| Abbreviation | **Amino acid structural formula** | Abbreviation | **Amino acid structural formula** | Abbreviation | **Amino acid structural formula** |
|---|---|---|---|---|---|
| Ala(3-8zt) | 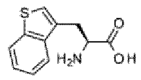 | Ile | 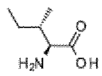 | MeIle | 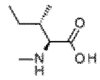 |
| Aze(2) | 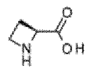 | Leu | 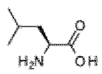 | MeLeu | 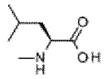 |
| ButenylPhe(4-CF3) | 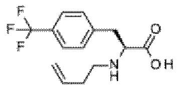 | MeAbu | 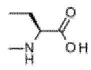 | MePhe | 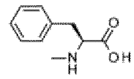 |
| cLeu | 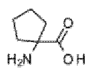 | MeAla | 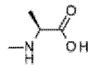 | MePhe(4-Me) | 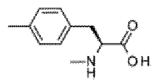 |
| eval | 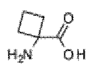 | MeAlgly | 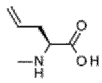 | MeVal | 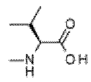 |
| D-Ala | 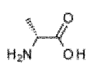 | MeCha | 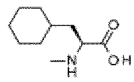 | Pro | 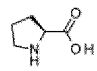 |
| D-L.eu | 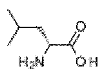 | MeChg | 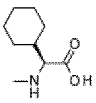 | Ser(tBu) | 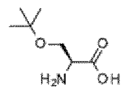 |
| D-Val | 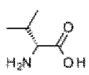 | MeGly | 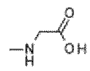 | Thr | 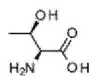 |
| Hyp(Et) | 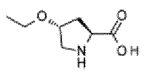 | MeGly(cPent) | 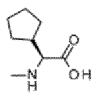 | | |
| Hph(4-CF3-35-F2) | 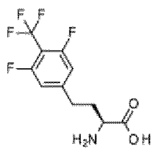 | Hph(4-CF3-3-Cl) | 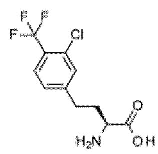 | | |

The structural formulae of the compounds 1 to 10 are shown in Table 2 to Table 4.

[Table 2]

| Compound No. | Structural formula | | Compound No. | Structural formula |
|---|---|---|---|---|
| Compound 1 | 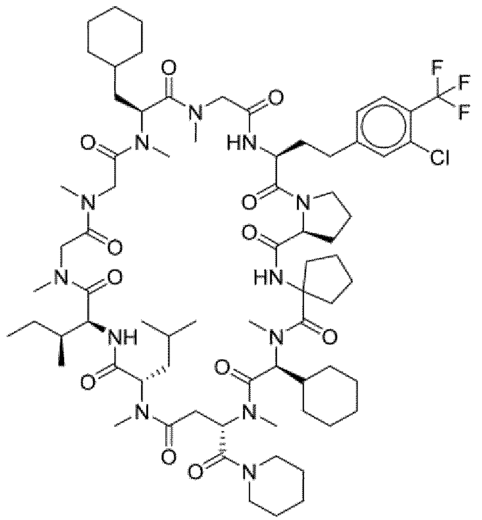 | | Compound 2 | 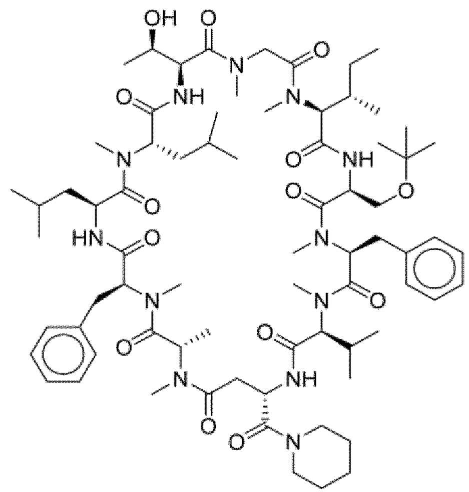 |
| Compound 3 | 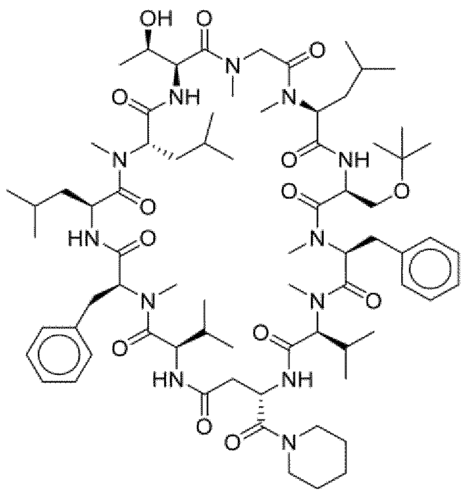 | | Compound 4 | 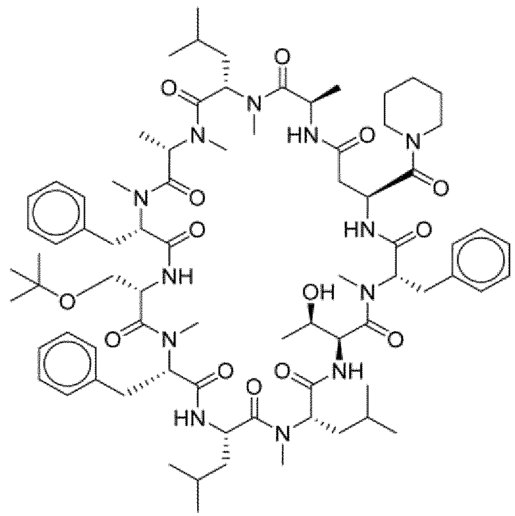 |

[Table 3]

| Compound No. | Structural formula | | Compound No. | Structural formula |
|---|---|---|---|---|
| Compound 5 | 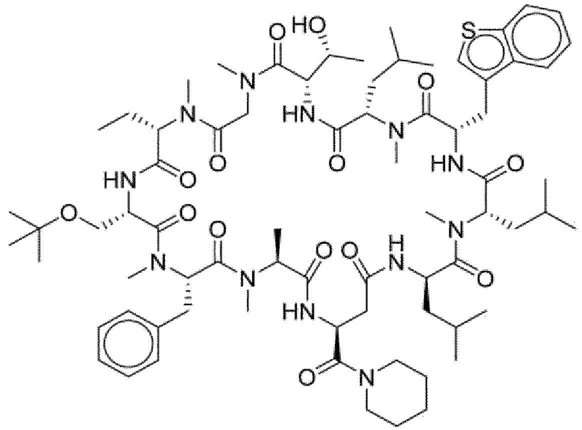 | | Compound 6 | 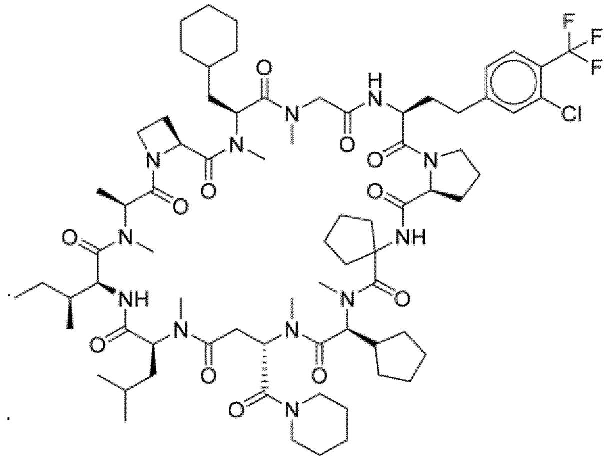 |
| Compound 7 | 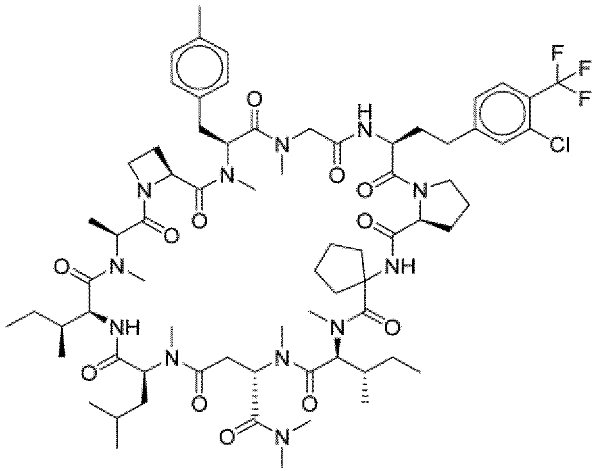 | | Compound 8 | 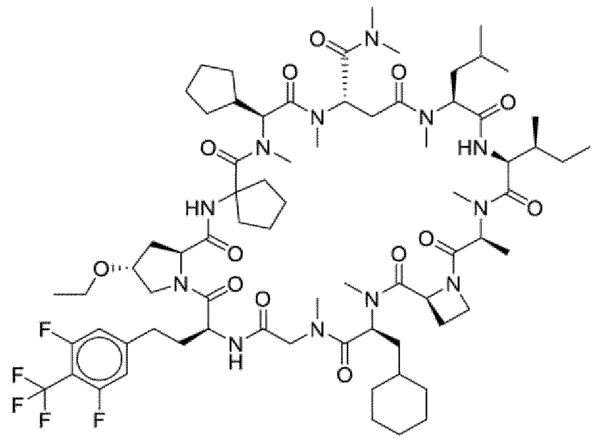 |

[Table 4]

| Compound No. | Structural formula | | Compound No. | Structural formula |
|---|---|---|---|---|
| Compound 9 | 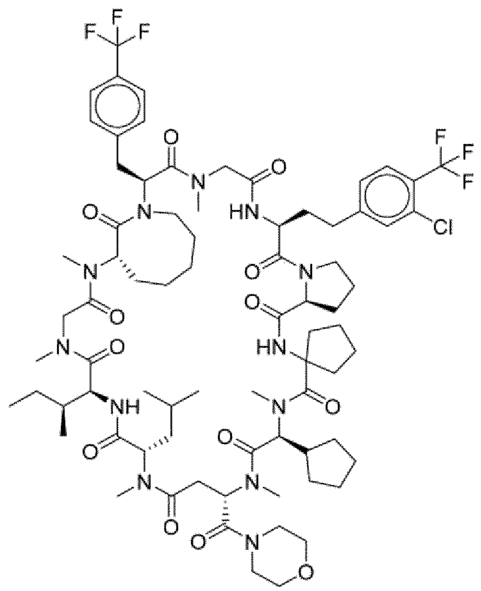 | | Compound 10 | 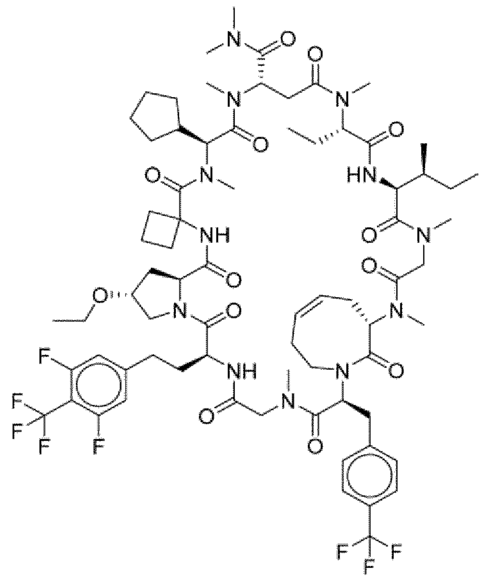 |

The analysis conditions of LC/MS are shown in Table 5.

[Table 5]

| Measurement condition | Device | Column (I.D. × length) (mm) | Mobile phase | Gradient (A/B) | Flow rate (mL/min) | Column temperature (°C) | Wavelength |
|---|---|---|---|---|---|---|---|
| SQDAA50 | Acquity UPLC/SQD | Ascentis Express C18 (2.1×50) | A) 10mM AcONH4, H2O B) MeOH | 50/50⇒0/100 (0.7 min) ⇒0/100 (0.7 min) | 1.0 | 35 | 210-400nM PDA total |
| SQDFA05 | Acquity UPLC/SQD | Ascentis Express C18 (2.1×50) | A) 0.1% FA, H2O B) 0.1% FA, MeCN | 95/5⇒0/100 (1.0 min) ⇒0/100 (0.4 min) | 1.0 | 35 | 210-400nM PDA total |
| SSC-AF-00 | Nexera UC/2020 | Ascentis Express C18 (2.1×50) | A) 10mM AcONH4, H2O B) MeOH | 95/5⇒0/100 (1.75 min) ⇒0/100 (1.25 min) | 1.0 | 35 | 210-400nM PDA total |
| SSC-A-FA-01 | Nexera UC/2020 | XSelect CSH C18 (2.1×50) | A) 0.1% FA, H2O B) 0.1% FA, MeCN | 70/30⇒10/90 (7.5 min) ⇒0/100 (0.01 min) ⇒0/100 (2.49 min ) | 0.5 | 50 | 210-400nM PDA total |
| SSC-FA-02 | Nexera UC/2020 | Ascentis Express C18 (2.1×50) | A)0.1% FA,H2O B) 0.1% FA,MeCN | 70/30=> 10/90(7.5min ) => 0/100(0.01 min) => 0/100 (2.49 min) | 0.5 | 50 | 210-400nm PDA total |
| SSC-FA-03 | Nexera UC/2020 | XSelect CSH C18 (2.1x50) | A) 0.1% FA, H2O B) 0.1% FA, MeCN | 95/5⇒0/100 (1.75 min) ⇒0/100 (1.25 min) | 1.0 | 35 | 210-400nM PDA total |
| SSC-TFA-07 | Nexera/2020 | Ascentis Express C18 (2.1×50) | A) 0.05% TFA, H2O B) 0.05% TFA, MeCN | 95/5⇒0/100 (1.5 min) =>0/100 (0.5 min) | 1.0 | 35 | 210-400nM PDA total |
| SMD method_04 | Nexera/2020 | Ascentis Express C18 (2.1×50) | A)0.1% FA,H2O B) 0.1% FA,MeCN | 95/5=> 0/100(1.5 min) ⇒ 0/100(0.5 min) | 1 | 35 | 210-400nm PDA total |
| SMD method_05 | Nexera/2020 | Ascentis Express RP-Amide (2.1×50) | A)0.1% FA,H2O B) 0.1% FA,MeCN | 95/5⇒0/100(1.5 min) ⇒0/100(0.5 min) | 1 | 35 | 210-400nm PDA total |
| SMD method_06 | Nexera/2020 | Ascentis Express C18 (2.1×50) | A)0.1% FA,H2O B) 0.1% FA,MeCN | 50/50⇒0/100(1min ) =>0/100(1 min) | 1 | 35 | 210-400nm PDA total |
| SMD method_07 | Nexera/2020 | Ascentis Express RP-Amide (2.1×50) | A)0.1% FA,H2O B)0.1% FA,MeCN | 50/50⇒0/100(1 min ) ⇒0/100(1 min) | 1 | 35 | 210-400nm PDA total |
| SMD method_08 | Nexera/2020 | Accucore C18 (2.1×50) | A) 10mM NH4HCO3 in H2O B) MeCN | 95/5⇒5/95(1.5 min) ⇒5/95(0.5 min ) | 1 | 35 | 210-400nm PDA total |
| SMD method_10 | Nexera/2020 | Kinetex EVO C18 (2.1×50) | A) 10mM NH4HCO3 in H2O B) MeCN | 95/5⇒5/95(1.5 min ) ⇒5/95(0.5 min ) | 1 | 35 | 210-400nm PDA total |

(continued)

| Measurement condition | Device | Column (I.D. × length) (mm) | Mobile phase | Gradient (A/B) | Flow rate (mL/min) | Column temperature (°C) | Wavelength |
|---|---|---|---|---|---|---|---|
| SMD method_ 14 | Shimazu LCMS-2020 | Shim-Pack XR-ODS (3.0×50) | A)0.05% TFA,H2O B) 0.05% TFA,MeCN | 95/5⇒ 0/100(1.1 min) ⇒ 0/100(0.6 min) | 1.2 | 40 | 190-400nm PDA total |
| SMD method_ 15 | Shimazu LCMS-2020 | Shim-Pack XR-ODS-C18 (3.0×50) | A)0.05% TFA,H2O B) 0.05% TFA,MeCN | 70/30⇒ 5195(3.8 min) ⇒ 5/95(0.8 min) | 1.2 | 40 | 190-400nm PDA total |
| SMD method_ 16 | Shimazu LCMS-2020 | Shim-Pack XR-OOS-C18 (3.0×50) | A)0.05% TFA,H2O B) 0.05% TFA,MeCN | 95/5⇒ 5/95(3.0 min) ⇒ 5/95(0.7 min) | 1.2 | 40 | 190-400nm PDA total |
| SMD method_ 17 | Shimadzu LCMS-2020 LC-20ADXR | kinetex XB-C18 (3.0×50) | A)0.1% FA,H2O B) 0.1% FA,MeCN | 90/10⇒ 0/100(1.2 min) ⇒ 0/100(0.5 min) ⇒90/10 (0.1 min) | 1.5 | 40 | 190-400nm PDA total |
| SMD method_ 20 | Shimazu LCMS-2020 | Hate C18 (3.0×30) | A)0.05% TFA,H2O B) 0.05% TFA,MeCN | 95/5⇒ 5/95(1.2 min) => 5/95(0.6 min) => 95/5(0.02 min ) | 1.5 | 40 | 190-400nm PDA total |
| SMD method_ 21 | Shimadzu LCMS-2020 LC-20AD | Ascentis Express C18 (4.6×100) | A)0.1% FA,H2O B)0.1% FA,MeCN | 95/5⇒ 70/30(15.0 min ) ⇒ 70/30(3.0 min) ⇒5/95(2.0 min) | 1 | an | 190-400nm PDA total |

The values in mass spectrum and retention time in liquid chromatography of the obtained compounds 1 to 10 were described in Table 6.

Table 6

| Compound No. | Measurement condition | Retention time (min) | MS found (m/z) | MS polarity |
|---|---|---|---|---|
| Compound 1 | SSC-TFA-07 | 1.637 | 1441.8 | (M+H)+ |
| Compound 2 | SQDAA50 | 0.86 | 1386 | (M+H)+ |
| Compound 3 | SQDAA50 | 0.88 | 1398 | (M-H)- |
| Compound 4 | SQDFA05 | 1.15 | 1434 | (M+H)+ |
| Compound 5 | SQDAA50 | 0.91 | 1413.8 | (M+H)+ |
| Compound 6 | SSC-FA-03 | 2.02 | 1451.8 | (M-H)- |
| Compound 7 | SSC-AF-00 | 2.085 | 1407.9 | (M-H)- |
| Compound 8 | SSC-A-FA-01 | 5.771 | 1458.1 | (M-H)- |
| Compound 9 | SSC-FA-02 | 6.311 | 1544 | (M-H)- |
| Compound 10 | SSC-FA-03 | 5.619 | 1505.7 | (M+H)+ |

Note that the molecular weights of the compounds 1 to 10 are as follows:

Compound 1: 1442.2

Compound 2: 1385.8

Compound 3: 1399.8

Compound 4: 1433.8

Compound 5: 1413.8

Compound 6: 1454.2

Compound 7: 1410.1

Compound 8: 1459.7

Compound 9: 1546.2

Compound 10: 1505.6

[Example 2] Effects of surfactant

(Example 2-1) In vitro solubility evaluation

Evaluation of the solubility of the compounds 1 and 2 was performed. To lyophilized powder of the compound, a 50 mM phosphate buffer solution (PPB: Phosphate buffer, pH 6.5) was added, and after shaking (at room temperature, 1800 rpm, for 24 hours), the mixed solution was filtered through a filter, and the compound concentration of the filtrate was measured by LC/MS/MS. Evaluation of the solubility of the compounds 1 and 2 in the presence of a surfactant was performed. To the compound powder, Cremophor EL (CreEL) (manufactured by Sigma-Aldrich Co., Ltd., "Kolliphor EL", generic name: polyoxyethylene castor oil, average number of moles of ethylene oxide added 35), Vitamin E TPGS (manufactured by Sigma-Aldrich Co., Ltd.), an aqueous solution containing 1% TW80 (manufactured by Nacalai Tesque, Inc.), an aqueous solution containing 1.5% Ploxamer 188 (manufactured by BASF SE), or an aqueous solution containing 0.5% Triton X-100 was added, the mixed solution was stirred (at room temperature, for 20 hours) with a stirrer, and then filtered through a filter, and the compound concentration of the filtrate was measured by LC/UV. Solubility (μg/mL) was

calculated from the measured compound concentration. The results are shown in Table 7. For the compounds 1 and 2, a larger solubility was observed in the presence of the surfactants than in the absence of the surfactants. Among others, a larger solubility was observed in the presence of CreEL or Vitamin E TPGS than in the presence of other surfactants (Table 7). This confirmed that the use of CreEL or Vitamin E TPGS shows superior solubility improvement independently of compounds, compared to the case where no surfactant is used or other surfactants are used for compounds with low membrane permeability. From the above, CreEL and Vitamin E TPGS were selected as substrates that improve solubility independently of compounds, and evaluation of oral absorbability was performed.

[Table 7]

| Compound No. | PPb | CreEL (1% aqueous solution) | VitaminE TPGS (1% aqueous solution) | Poloxamer 188 (1.5% aqueous solution) | TW80 (1% aqueous solution) | TritonX-100 (0.5% aqueous solution) |
|---|---|---|---|---|---|---|
| Compound 1 | 1 | 3385 | 3104 | < 13 | 1268 | 743 |
| Compound 2 | 11 | 1053 | 1953 | 31 | 707 | 498 |
| *The numeric values represent solubilities (μg/mL). | | | | | | |

(Example 2-2) Preparation of formulation

By mixing and stirring the compounds 1 and 2, water for injection (manufactured by Otsuka Pharmaceutical Factory, Inc.), dimethyl sulfoxide (DMSO) (manufactured by Wako Pure Chemical Industries, Ltd.), and Cremophor EL so as to achieve the compositional features shown in Table 8, Example formulations (1) and (2) were prepared. It should be noted that the compounds 1 and 2 were mixed as a 3 mg/mL solution in dimethyl sulfoxide.

By mixing and stirring the compounds 1 and 2, water for injection (manufactured by Otsuka Pharmaceutical Factory, Inc.), dimethyl sulfoxide (manufactured by Wako Pure Chemical Industries, Ltd.), and Vitamin E TPGS (manufactured by Sigma-Aldrich Co., Ltd.) so as to achieve the compositional features shown in Table 8, Comparative formulations (1-1) to (2-2) were prepared. It should be noted that the compounds 1 and 2 were mixed as a 3 mg/mL solution in dimethyl sulfoxide.

[Table 8]

| | Solute | | Solvent | | | |
|---|---|---|---|---|---|---|
| | Compound 1 | Compound 2 | DMSO | VitaminETPGS | CreEL | Water for injection |
| | mg/mL | mg/mL | % by volume | % by weight | % by volume | % by volume |
| Comparative formulation (1-1) | 0.3 | - | 10 | 2 | - | 88 |
| Comparative formulation (1-2) | 0.3 | - | 10 | 10 | - | 80 |
| Example formulation (1) | 0.3 | - | 10 | - | 2 | 88 |
| Comparative formulation (2-1) | - | 0.3 | 10 | 1 | - | 89 |
| Comparative formulation (2-2) | - | 0.3 | 10 | 6 | - | 84 |
| Example formulation (2) | - | 0.3 | 10 | - | 2 | 88 |

(Example 2-3) Rat PK test (3 mg/kg)

Blood kinetics after oral administration of Example formulations (1) and (2), as well as Comparative formulations (1-1), (1-2), (2-1), and (2-2), in rats were evaluated. To male rats (WIST, 7 weeks of age, manufactured by Japan Laboratory Animals, Inc.: 4 rats per group), any one of Example formulations (1) and (2), as well as Comparative formulations (1-1), (1-2), (2-1), and (2-2), was orally administered at a dose of 3 mg/kg of the compound 1, and blood was collected from the jugular vein over time up to 24 hours after administration using a syringe treated with heparin as an anticoagulant. Plasma was separated from the blood by centrifugation, and after the deproteinization with acetonitrile, the plasma concentration of the compounds 1 and 2 was measured using a LC/MS/MS device. From the obtained changes in plasma concentration, the pharmacokinetic parameters were calculated by non-compartmental analysis using analysis software Phoenix WinNonlin 8.2 (manufactured by Certara L.P.). The results are shown in Table 9.

As the pharmacokinetic parameters, the area under the plasma drug concentration-time curve (AUClast; ng-h/mL) from 0 hours after administration to the last time point at which a concentration above the quantitative limit was obtained, and the maximum plasma concentration after administration (Cmax; ng/mL) were calculated. For the compounds 1 and 2, it was confirmed that the AUClast in both the administration groups of Example formulations (1) and (2) was greater than the AUClast in the administration groups of Comparative formulations (1-1), (1-2), (2-1), and (2-2), and also observed that the Cmax was increased (Table 9). From this, it was confirmed that, with the use of Cremophor EL, the compounds with low membrane permeability exhibit higher absorbability compared to the case where Cremophor EL is not used.

[Table 9]

| Formulation | AUClast (ng·h/mL) | Cmax (ng/mL) |
|---|---|---|
| Comparative formulation (1-1) | 13.1 | 5.85 |
| Comparative formulation (1-2) | 3.63 | 2.17 |
| Example formulation (1) | 895 | 347 |
| Comparative formulation (2-1) | 778 | 163 |
| Comparative formulation (2-2) | 131 | 32.0 |
| Example formulation (2) | 1470 | 345 |

[Example 3] Evaluation of physical properties of cyclic peptide compound

(Example 3-1) Evaluation of Caco-2 membrane permeability

Caco-2 cells were cultured on 96-well Transwell for 3 weeks, then DMEM, FaSSIF (1% DMSO), and the compound were added to the Apical side, DMEM was added to the Basal side, and the cells were preincubated under 5% $CO_2$ at 37°C with shaking at 80 rpm for 18 to 24 hours. After the preincubation, the preincubation solutions on the Apical side and Basal side were sucked off and washed, a FaSSIF/HBSS buffer solution (1% DMSO) (pH 6.0) containing the compound was added to the Apical side, a HBSS buffer solution (4% BSA) (pH 7.4) was added to the Basal side, and measurement of membrane permeability was started. Each well was shaken under 5% $CO_2$ at 37°C and 80 rpm, and at 180 minutes after the start of shaking, a sample on the Basal side was collected, and the permeation amount was measured by LC/MS. From the permeation amount, the membrane permeability coefficient (Papp) was calculated. Note that the description "Caco-2 (cm/sec)" in the column in the table herein is used synonymously with "Papp (cm/sec)".

Caco-2 value of cyclic peptide compound

[Table 10]

| | Caco2 |
|---|---|
| | ×E-6 cm/sec |
| Compound 1 | 0.71 |
| Compound 2 | 5.99 |
| Compound 3 | 4.07 |
| Compound 4 | 1.45 |

(continued)

| | Caco2 |
|---|---|
| | ×E-6 cm/sec |
| Compound 5 | 1.42 |
| Compound 6 | 0.61 |
| Compound 7 | 2.96 |
| Compound 8 | 2.39 |
| Compound 9 | 0.14 |
| Compound 10 | 0.70 |

(Example 3-2) Evaluation of lipophilicity of cyclic peptide compound

The ClogP of the compounds 1 to 10 is as follows:

[Table 11]

| | ClogP |
|---|---|
| Compound 1 | 16.1 |
| Compound 2 | 13.2 |
| Compound 3 | 13.6 |
| Compound 4 | 13.6 |
| Compound 5 | 13.3 |
| Compound 6 | 15.9 |
| Compound 7 | 14.2 |
| Compound 8 | 14.9 |
| Compound 9 | 15.9 |
| Compound 10 | 13.7 |

(Example 3-3) Evaluation of solubility of cyclic peptide compound

Evaluation of the solubility of the cyclic peptide compound was performed. To lyophilized powder of the compound, a 50 mM phosphate buffer solution (PPB: Phosphate buffer, pH 6.5) was added, and after shaking (at 37°C, 1800 rpm, for 22 to 24 hours), the mixed solution was filtered through a filter, and the compound concentration of the filtrate was measured by LC/MS/MS. Solubility was calculated from the measured compound concentration. The solubilities of the compounds 3, 4, 6, 7, and 8 were 11 μg/mL, 1 μg/mL, 1 μg/mL, 15 μg/mL, and 5 μg/mL, respectively. From these results, it was confirmed that the cyclic peptide compound group uniformly exhibited low solubility.

Evaluation of the solubility of the cyclic peptide compound in the presence of a surfactant was performed. To lyophilized powder of the compound, an aqueous solution containing 0.3% or 1% Cremophor EL (CreEL) was added, and after shaking (at 25°C, 1000 rpm, for 22 to 24 hours), the mixed solution was filtered through a filter, and the compound concentration of the filtrate was measured by LC/UV. Solubility (μg/mL) was calculated from the measured compound concentration. The results are shown in Table 12. The cyclic peptide compound groups all exhibited a solubility of 100 μg/mL or more in the aqueous CreEL solution. From these results, it was confirmed that, with the use of CreEL, the compounds with low membrane permeability exhibit improvement in solubility compared to the case where no surfactant is used.

Table 12]

| | 0.3% Cremophor EL | 1% Cremophor EL |
|---|---|---|
| | μg/mL | μg/mL |
| Compound 3 | 101 | 395 |
| Compound 4 | 214 | 789 |
| Compound 5 | 295 | 1110 |
| Compound 6 | 434 | 1453 |
| Compound 7 | 492 | 1645 |
| Compound 8 | 356 | 1337 |
| Compound 9 | 450 | 1512 |
| Compound 10 | 583 | 1683 |

[Example 4] Preparation of formulation

(Example 4-1) Preparation of formulation using Cremophor EL

By mixing and stirring the cyclic peptide compound, water for injection (manufactured by Shanghai Pujin Lin Pharmaceutical Co., Ltd. or Otsuka Pharmaceutical Factory, Ltd.), dimethyl sulfoxide (manufactured by Wako Pure Chemica Industries, Ltd.), and Cremophor EL (manufactured by Sigma-Aldrich Co., Ltd., "Kolliphor EL", generic name: polyoxyethylene castor oil, average number of moles of ethylene oxide added 35) so as to achieve the compositional features of Table 13 to 33, Example formulations and Comparative formulations were prepared.

[Table 13]

| | Solute | Solvent | | |
|---|---|---|---|---|
| | Compound 3 | DMSO | CreEL | Water for injection |
| | mg/mL | % by volume | % by volume | % by volume |
| Example formulation 3-1 | 1.5 | 10 | 4 | 86 |
| Example formulation 3-2 | 1.5 | 10 | 5 | 85 |
| Example formulation 3-3 | 1.5 | 10 | 7 | 83 |
| Comparative formulation 3-1 | 1.5 | 10 | 12 | 78 |
| Comparative formulation 3-2 | 1.5 | 10 | 32 | 58 |

[Table 14]

| | Solute | Solvent | | |
|---|---|---|---|---|
| | Compound 4 | DMSO | CreEL | Water for injection |
| | mg/mL | % by volume | % by volume | % by volume |
| Example formulation 4-1 | 1.5 | 10 | 2 | 88 |
| Example formulation 4-2 | 1.5 | 10 | 2.7 | 87.3 |
| Example formulation 4-3 | 1.5 | 10 | 4 | 86 |
| Example formulation 4-4 | 1.5 | 10 | 6 | 84 |
| Comparative formulation 4-1 | 1.5 | 10 | 19 | 71 |

[Table 15]

| | Solute | Solvent | | |
|---|---|---|---|---|
| | Compound 5 | DMSO | CreEL | Water for injection |
| | mg/mL | % by volume | % by volume | % by volume |
| Comparative formulation 5-1 | 1.5 | 10 | 1.5 | 88.5 |
| Example formulation 5-1 | 1.5 | 10 | 2 | 88 |
| Example formulation 5-2 | 1.5 | 10 | 2.7 | 87.3 |
| Example formulation 5-3 | 1.5 | 10 | 2 | 88 |
| Comparative formulation 5-2 | 1.5 | 10 | 10 | 80 |

[Table 16]

| | Solute | Solvent | | |
|---|---|---|---|---|
| | Compound 6 | DMSO | CreEL | Water for injection |
| | mg/mL | % by volume | % by volume | % by volume |
| Comparative formulation 6-1 | 1.5 | 10 | 1.5 | 88.5 |
| Example formulation 6-1 | 1.5 | 10 | 2 | 88 |
| Example formulation 6-2 | 1.5 | 10 | 3.5 | 86.5 |

[Table 17]

| | Solute | Solvent | | |
|---|---|---|---|---|
| | Compound 7 | DMSO | CreEL | Water for injection |
| | mg/mL | % by volume | % by volume | % by volume |
| Comparative formulation 7-1 | 1.5 | 10 | 1 | 89 |
| Comparative formulation 7-2 | 1.5 | 10 | 1.3 | 88.7 |
| Example formulation 7-1 | 1.5 | 10 | 2 | 88 |
| Example formulation 7-2 | 1.5 | 10 | 3 | 87 |
| Comparative formulation 7-3 | 1.5 | 10 | 9 | 81 |

[Table 18]

| | Solute | Solvent | | |
|---|---|---|---|---|
| | Compound 8 | DMSO | CreEL | Water for injection |
| | mg/mL | % by volume | % by volume | % by volume |
| Comparative formulation 8-1 | 1.5 | 10 | 1.2 | 88.8 |
| Comparative formulation 8-2 | 1.5 | 10 | 1.5 | 88.5 |
| Example formulation 8-1 | 1.5 | 10 | 2 | 88 |
| Example formulation 8-2 | 1.5 | 10 | 4 | 86 |
| Comparative formulation 8-3 | 1.5 | 10 | 12 | 78 |

[Table 19]

| | Solute | Solvent | | |
|---|---|---|---|---|
| | Compound 9 | DMSO | CreEL | Water for injection |
| | mg/mL | % by volume | % by volume | % by volume |
| Comparative formulation 9-1 | 1.5 | 10 | 1.1 | 88.9 |
| Comparative formulation 9-2 | 1.5 | 10 | 1.4 | 88.6 |
| Example formulation 9-1 | 1.5 | 10 | 2 | 88 |
| Example formulation 9-2 | 1.5 | 10 | 3 | 87 |
| Comparative formulation 9-3 | 1.5 | 10 | 10 | 80 |

[Table 20]

| | Solute | Solvent | | |
|---|---|---|---|---|
| | Compound 10 | DMSO | CreEL | Water for injection |
| | mg/mL | % by volume | % by volume | % by volume |
| Comparative formulation 10-1 | 1.5 | 10 | 1 | 89 |
| Comparative formulation 10-2 | 1.5 | 10 | 1.25 | 88.75 |
| Example formulation 10-1 | 1.5 | 10 | 3 | 87 |

[Table 21]

| | Solute | Solvent | | |
|---|---|---|---|---|
| | Compound 3 | DMSO | CreEL | Water for injection |
| | mg/mL | % by volume | % by volume | % by volume |
| Comparative formulation 3-2-1 | 0.15 | 10 | 0.45 | 89.55 |
| Comparative formulation 3-2-2 | 0.15 | 10 | 0.6 | 89.4 |
| Comparative formulation 3-2-3 | 0.15 | 10 | 0.85 | 89.15 |
| Comparative formulation 3-2-4 | 0.15 | 10 | 1.4 | 88.6 |
| Example formulation 3-2-5 | 0.15 | 10 | 4 | 86 |
| Example formulation 3-2-6 | 0.15 | 10 | 5 | 85 |

[Table 22]

| | Solute | Solvent | | |
|---|---|---|---|---|
| | Compound 3 | DMSO | CreEL | Water for injection |
| | mg/mL | % by volume | % by volume | % by volume |
| Comparative formulation 3-2-7 | 0.15 | 10 | 0.8 | 89.2 |
| Comparative formulation 3-2-8 | 0.15 | 10 | 1.4 | 88.6 |
| Example formulation 3-2-9 | 0.15 | 10 | 2.5 | 87.5 |
| Example formulation 3-2-10 | 0.15 | 10 | 5 | 85 |
| Example formulation 3-2-11 | 0.15 | 10 | 7.5 | 82.5 |

(continued)

| | Solute | Solvent | | |
|---|---|---|---|---|
| | Compound 3 | DMSO | CreEL | Water for injection |
| | mg/mL | % by volume | % by volume | % by volume |
| Comparative formulation 3-2-12 | 0.15 | 10 | 15 | 75 |

[Table 23]

| | Solute | Solvent | | |
|---|---|---|---|---|
| | Compound 4 | DMSO | CreEL | Water for injection |
| | mg/mL | % by volume | % by volume | % by volume |
| Comparative formulation 4-2-1 | 0.15 | 10 | 0.45 | 89.55 |
| Comparative formulation 4-2-2 | 0.15 | 10 | 0.75 | 89.25 |
| Example formulation 4-2-3 | 0.15 | 10 | 2.5 | 87.5 |
| Example formulation 4-2-4 | 0.15 | 10 | 5 | 85 |
| Example formulation 4-2-5 | 0.15 | 10 | 7.5 | 82.5 |
| Comparative formulation 4-2-6 | 0.15 | 10 | 15 | 75 |

[Table 24]

| | Solute | Solvent | | |
|---|---|---|---|---|
| | Compound 6 | DMSO | CreEL | Water for injection |
| | mg/mL | % by volume | % by volume | % by volume |
| Comparative formulation 6-2-1 | 0.15 | 10 | 0.12 | 89.88 |
| Comparative formulation 6-2-2 | 0.15 | 10 | 0.15 | 89.85 |
| Comparative formulation 6-2-3 | 0.15 | 10 | 0.2 | 89.8 |
| Comparative formulation 6-2-4 | 0.15 | 10 | 0.35 | 89.65 |
| Comparative formulation 6-2-5 | 0.15 | 10 | 1 | 89 |
| Example formulation 6-2-6 | 0.15 | 10 | 5 | 85 |

[Table 25]

| | Solute | Solvent | | |
|---|---|---|---|---|
| | Compound 6 | DMSO | CreEL | Water for injection |
| | mg/mL | % by volume | % by volume | % by volume |
| Comparative formulation 6-2-7 | 0.15 | 10 | 0.2 | 89.8 |
| Comparative formulation 6-2-8 | 0.15 | 10 | 0.35 | 89.65 |
| Example formulation 6-2-9 | 0.15 | 10 | 2.5 | 87.5 |
| Example formulation 6-2-10 | 0.15 | 10 | 5 | 85 |
| Example formulation 6-2-11 | 0.15 | 10 | 7.5 | 82.5 |
| Comparative formulation 6-2-12 | 0.15 | 10 | 15 | 75 |

[Table 26]

| | Solute | Solvent | | |
|---|---|---|---|---|
| | Compound 7 | DMSO | CreEL | Water for injection |
| | mg/mL | % by volume | % by volume | % by volume |
| Comparative formulation 7-2-1 | 0.15 | 10 | 0.11 | 89.89 |
| Comparative formulation 7-2-2 | 0.15 | 10 | 0.15 | 89.85 |
| Comparative formulation 7-2-3 | 0.15 | 10 | 0.2 | 89.8 |
| Comparative formulation 7-2-4 | 0.15 | 10 | 0.3 | 89.7 |
| Comparative formulation 7-2-5 | 0.15 | 10 | 1 | 89 |
| Example formulation 7-2-6 | 0.15 | 10 | 5 | 85 |

[Table 27]

| | Solute | Solvent | | |
|---|---|---|---|---|
| | Compound 7 | DMSO | CreEL | Water for injection |
| | mg/mL | % by volume | % by volume | % by volume |
| Comparative formulation 7-2-7 | 0.15 | 10 | 0.2 | 89.8 |
| Comparative formulation 7-2-8 | 0.15 | 10 | 0.3 | 89.7 |
| Example formulation 7-2-9 | 0.15 | 10 | 2.5 | 87.5 |
| Example formulation 7-2-10 | 0.15 | 10 | 5 | 85 |
| Example formulation 7-2-11 | 0.15 | 10 | 7.5 | 82.5 |
| Comparative formulation 7-2-12 | 0.15 | 10 | 15 | 75 |

[Table 28]

| | Solute | Solvent | | |
|---|---|---|---|---|
| | Compound 8 | DMSO | CreEL | Water for injection |
| | mg/mL | % by volume | % by volume | % by volume |
| Comparative formulation 8-2-1 | 0.15 | 10 | 0.17 | 89.83 |
| Comparative formulation 8-2-2 | 0.15 | 10 | 0.2 | 89.8 |
| Comparative formulation 8-2-3 | 0.15 | 10 | 0.3 | 89.7 |
| Comparative formulation 8-2-4 | 0.15 | 10 | 0.5 | 89.5 |
| Comparative formulation 8-2-5 | 0.15 | 10 | 1.5 | 88.5 |
| Example formulation 8-2-6 | 0.15 | 10 | 5 | 85 |

[Table 29]

| | Solute | Solvent | | |
|---|---|---|---|---|
| | Compound 8 | DMSO | CreEL | Water for injection |
| | mg/mL | % by volume | % by volume | % by volume |
| Comparative formulation 8-2-7 | 0.15 | 10 | 0.3 | 89.7 |

(continued)

| | Solute | Solvent | | |
|---|---|---|---|---|
| | Compound 8 | DMSO | CreEL | Water for injection |
| | mg/mL | % by volume | % by volume | % by volume |
| Comparative formulation 8-2-8 | 0.15 | 10 | 0.5 | 89.5 |
| Example formulation 8-2-9 | 0.15 | 10 | 2.5 | 87.5 |
| Example formulation 8-2-10 | 0.15 | 10 | 5 | 85 |
| Example formulation 8-2-11 | 0.15 | 10 | 7.5 | 82.5 |
| Comparative formulation 8-2-12 | 0.15 | 10 | 15 | 75 |

[Table 30]

| | Solute | Solvent | | |
|---|---|---|---|---|
| | Compound 9 | DMSO | CreEL | Water for injection |
| | mg/mL | % by volume | % by volume | % by volume |
| Comparative formulation 9-2-1 | 0.15 | 10 | 0.11 | 89.89 |
| Comparative formulation 9-2-2 | 0.15 | 10 | 0.15 | 89.85 |
| Comparative formulation 9-2-3 | 0.15 | 10 | 0.2 | 89.8 |
| Comparative formulation 9-2-4 | 0.15 | 10 | 0.35 | 89.65 |
| Comparative formulation 9-2-5 | 0.15 | 10 | 1 | 89 |
| Example formulation 9-2-6 | 0.15 | 10 | 5 | 85 |

[Table 31]

| | Solute | Solvent | | |
|---|---|---|---|---|
| | Compound 9 | DMSO | CreEL | Water for injection |
| | mg/mL | % by volume | % by volume | % by volume |
| Comparative formulation 9-2-7 | 0.15 | 10 | 0.2 | 89.8 |
| Comparative formulation 9-2-8 | 0.15 | 10 | 0.35 | 89.65 |
| Example formulation 9-2-9 | 0.15 | 10 | 2.5 | 87.5 |
| Example formulation 9-2-10 | 0.15 | 10 | 5 | 85 |
| Example formulation 9-2-11 | 0.15 | 10 | 7.5 | 82.5 |
| Comparative formulation 9-2-12 | 0.15 | 10 | 15 | 75 |

[Table 32]

| | Solute | Solvent | | |
|---|---|---|---|---|
| | Compound 10 | DMSO | CreEL | Water for injection |
| | mg/mL | % by volume | % by volume | % by volume |
| Comparative formulation 10-2-1 | 0.15 | 10 | 0.03 | 89.97 |
| Comparative formulation 10-2-2 | 0.15 | 10 | 0.04 | 89.96 |
| Comparative formulation 10-2-3 | 0.15 | 10 | 0.05 | 89.95 |

(continued)

| | Solute | Solvent | | |
|---|---|---|---|---|
| | Compound 10 | DMSO | CreEL | Water for injection |
| | mg/mL | % by volume | % by volume | % by volume |
| Comparative formulation 10-2-4 | 0.15 | 10 | 0.08 | 89.92 |
| Comparative formulation 10-2-5 | 0.15 | 10 | 0.25 | 89.75 |
| Example formulation 10-2-6 | 0.15 | 10 | 5 | 85 |

[Table 33]

| | Solute | Solvent | | |
|---|---|---|---|---|
| | Compound 10 | DMSO | CreEL | Water for injection |
| | mg/mL | % by volume | % by volume | % by volume |
| Comparative formulation 10-2-7 | 0.15 | 10 | 0.05 | 89.95 |
| Comparative formulation 10-2-8 | 0.15 | 10 | 0.08 | 89.92 |
| Example formulation 10-2-9 | 0.15 | 10 | 2.5 | 87.5 |
| Example formulation 10-2-10 | 0.15 | 10 | 5 | 85 |
| Example formulation 10-2-11 | 0.15 | 10 | 7.5 | 82.5 |
| Comparative formulation 10-2-12 | 0.15 | 10 | 15 | 75 |

[Example 5] PK test

(Example 5-1) Mouse PK test (30 mg/kg)

Blood kinetics after oral administration of Example formulations and Comparative formulations in mice were evaluated. To male mice (C57BL/6J, 7 to 11 weeks of age, manufactured by SiBeiFu Laboratory Animal Technology Co., Ltd. or Charles River Laboratories Japan, Inc.: 3 mice per group), any one of the prepared Example formulations and Comparative formulations was orally administered at a dose of 30 mg/kg of the compounds 3 to 10, and blood was collected from the jugular vein and the caudal vena cava, or the back leg vein and heart over time up to 24 hours after administration using heparin as an anticoagulant. Plasma was separated from the blood by centrifugation, and after the deproteinization with acetonitrile or liquid-liquid extraction with ethyl acetate, the plasma concentration of the compounds 3 to 10 was measured using a LC/MS/MS device. From the obtained changes in plasma concentration, the pharmacokinetic parameters were calculated by non-compartmental analysis using analysis software Phoenix WinNonlin 8.2 (manufactured by Certara L.P.). The results are shown in Tables 34 to 42.

As the pharmacokinetic parameters, the area under the plasma drug concentration-time curve (AUClast; ng-h/mL) from 0 hours after administration to the last time point at which a concentration above the quantitative limit was obtained, and the maximum plasma concentration after administration (Cmax; ng/mL) were calculated. The AUClast ratio was calculated as the ratio of the AUClast of the Example formulation or Comparative formulation (AUC for comparison) to the AUClast of the Example formulation that exhibited the maximum AUClast (maximum AUC), when the same compound was administered in the same test (AUC for comparison/maximum AUC). For the cyclic peptide compound group, it was confirmed that the AUClast in all of the administration groups of Example formulations was greater than the AUClast in the administration groups of Comparative formulations (AUClast ratio of 0.8 or more), and also observed that the Cmax was increased. As shown in Figure 1, for all compounds, with the use of Cremophor EL formulations with a Cremophor EL content of 2.0% by volume to 7.5% by volume based on 100% by volume of the formulation, compounds with low membrane permeability were confirmed to exhibit higher absorbability (AUClast ratio of 0.8 or more) compared to the case where formulations with a Cremophor EL concentration such that the Cremophor EL content is less than 2.0% by volume and greater than 7.5% by volume based on 100% by volume of the formulation.

[Table 34]

| Analyte | CreEL (% by volume) | AUClast (ng·h/mL) | AUClast ratio | Cmax (ng/mL) |
|---|---|---|---|---|
| Example formulation 3-1 | 4 | 47300 | 1.00 | 4030 |
| Example formulation 3-2 | 5 | 41500 | 0.88 | 3660 |
| Example formulation 3-3 | 7 | 45100 | 0.95 | 3920 |
| Comparative formulation 3-1 | 12 | 33600 | 0.71 | 2570 |
| Comparative formulation 3-2 | 32 | 8460 | 0.18 | 578 |

[Table 35]

| Analyte | CreEL (% by volume) | AUClast (ng·h/mL) | AUClast ratio | Cmax (ng/mL) |
|---|---|---|---|---|
| Example formulation 4-1 | 2 | 78300 | 0.87 | 6300 |
| Example formulation 4-2 | 2.7 | 72500 | 0.80 | 6140 |
| Example formulation 4-3 | 4 | 78300 | 0.87 | 6030 |
| Example formulation 4-4 | 6 | 90200 | 1.00 | 7730 |
| Comparative formulation 4-1 | 19 | 34400 | 0.38 | 3780 |

[Table 36]

| Analyte | CreEL (% by volume) | AUClast (ng·h/mL) | AUClast ratio | Cmax (ng/mL) |
|---|---|---|---|---|
| Comparative formulation 5-1 | 1.5 | 13300 | 0.52 | 3670 |
| Example formulation 5-1 | 2 | 25800 | 1.00 | 5320 |
| Example formulation 5-2 | 2.7 | 22300 | 0.86 | 4800 |

[Table 37]

| Analyte | CreEL (% by volume) | AUClast (ng·h/mL) | AUClast ratio | Cmax (ng/mL) |
|---|---|---|---|---|
| Example formulation 5-3 | 2 | 23400 | 1.00 | 6040 |
| Comparative formulation 5-2 | 10 | 8110 | 0.35 | 1990 |

[Table 38]

| Analyte | CreEL (% by volume) | AUClast (ng·h/mL) | AUClast ratio | Cmax (ng/mL) |
|---|---|---|---|---|
| Comparative formulation 6-1 | 1.5 | 176000 | 0.75 | 14700 |
| Example formulation 6-1 | 2 | 224000 | 0.95 | 16700 |
| Example formulation 6-2 | 3.5 | 235000 | 1.00 | 17600 |

[Table 39]

| Analyte | CreEL (% by volume) | AUClast (ng·h/mL) | AUClast ratio | Cmax (ng/mL) |
|---|---|---|---|---|
| Comparative formulation 7-1 | 1 | 59500 | 0.76 | 8460 |
| Comparative formulation 7-2 | 1.3 | 53400 | 0.68 | 7290 |
| Example formulation 7-1 | 2 | 70800 | 0.91 | 9800 |

(continued)

| Analyte | CreEL (% by volume) | AUClast (ng·h/mL) | AUClast ratio | Cmax (ng/mL) |
|---|---|---|---|---|
| Example formulation 7-2 | 3 | 78200 | 1.00 | 11800 |
| Comparative formulation 7-3 | 9 | 67800 | 0.87 | 9710 |

[Table 40]

| Analyte | CreEL (% by volume) | AUClast (ng·h/mL) | AUClast ratio | Cmax (ng/mL) |
|---|---|---|---|---|
| Comparative formulation 8-1 | 1.2 | 48200 | 0.62 | 7740 |
| Comparative formulation 8-2 | 1.5 | 47400 | 0.61 | 7930 |
| Example formulation 8-1 | 2 | 77200 | 0.99 | 9490 |
| Example formulation 8-2 | 4 | 77800 | 1.00 | 15000 |
| Comparative formulation 8-3 | 12 | 21200 | 0.27 | 2840 |

[Table 41]

| Analyte | CreEL (% by volume) | AUClast (ng·h/mL) | AUClast ratio | Cmax (ng/mL) |
|---|---|---|---|---|
| Comparative formulation 9-1 | 1.1 | 15700 | 0.69 | 2360 |
| Comparative formulation 9-2 | 1.4 | 17500 | 0.77 | 2600 |
| Example formulation 9-1 | 2 | 19200 | 0.85 | 2740 |
| Example formulation 9-2 | 3 | 22600 | 1.00 | 2800 |
| Comparative formulation 9-3 | 10 | 6920 | 0.31 | 1020 |

[Table 42]

| Analyte | CreEL (% by volume) | AUClast (ng·h/mL) | AUClast ratio | Cmax (ng/mL) |
|---|---|---|---|---|
| Comparative formulation 10-1 | 1 | 4580 | 0.60 | 1220 |
| Comparative formulation 10-2 | 1.25 | 4500 | 0.59 | 1130 |
| Example formulation 10-1 | 3 | 7650 | 1.00 | 1850 |

Table 43 summarizes the proportion (%) of the number of plots corresponding to an AUClast ratio ≥ 0.8 to the total plots for each Cremophor EL concentration.

Table 43]

| | Proportion (%) of number of plots corresponding to AUClast ratio ≥ 0.8 to total plots |
|---|---|
| CreEL < 2.0% | 0 |
| CreEL 2.0 to 7.5% | 100 |
| Cre EL > 7.5% | 14 |

As shown in Table 43, when the Cremophor EL concentration is greater than 7.5% by volume, the probability of an AUClast ratio of 0.8 or more is 14%, and when the Cremophor EL concentration is less than 2% by volume, the probability of an AUClast ratio of 0.8 or more is 0%, whereas when the Cremophor EL concentration is 2.0 to 7.5% by volume, the proportion of an AUClast ratio of 0.8 or higher is improved to 100%. That is, for cyclic peptide compounds, with the use of 2.0 to 7.5% by volume of Cremophor EL, compounds with low membrane permeability were confirmed to exhibit higher absorbability (AUClast ratio of 0.8 or more) compared to the case where a Cremophor EL concentration

of less than 2.0% by volume and greater than 7.5% by volume was used.

(Example 5-2) Mouse PK test (3 mg/kg)

Blood kinetics after oral administration of Example formulations and Comparative formulations in mice were evaluated. To male mice (C57BL/6, 10 weeks of age, manufactured by SiBeiFu Laboratory Animal Technology Co., Ltd. or Charles River Laboratories Japan, Inc.: 3 mice per group), any one of the prepared Example formulations and Comparative formulations was orally administered at a dose of 3 mg/kg of the compounds 3, 4, and 6 to 10, and blood was collected from the jugular vein and the caudal vena cava, or the back leg vein and heart over time up to 24 hours after administration using heparin as an anticoagulant. Plasma was separated from the blood by centrifugation, and after the deproteinization with acetonitrile or liquid-liquid extraction with ethyl acetate, the plasma concentration of the compounds 3, 4, and 6 to 10 was measured using a LC/MS/MS device. From the obtained changes in plasma concentration, the pharmacokinetic parameters were calculated by non-compartmental analysis using analysis software Phoenix WinNonlin 8.2 (manufactured by Certara L.P.). The results are shown in Tables 44 to 56.

As the pharmacokinetic parameters, the area under the plasma drug concentration-time curve (AUClast; ng·h/mL) from 0 hours after administration to the last time point at which a concentration above the quantitative limit was obtained, and the maximum plasma concentration after administration (Cmax; ng/mL) were calculated. The AUClast ratio was calculated as the ratio of the AUClast of the Example formulation or Comparative formulation (AUC for comparison) to the AUClast of the Example formulation that exhibited the maximum AUClast (maximum AUC), when the same compound was administered in the same test (AUC for comparison/maximum AUC). For the cyclic peptide compound group, it was confirmed that the AUClast in the administration groups of Example formulations had a higher probability of being greater than the AUClast in the administration groups of Comparative formulations (AUClast ratio of 0.7 or more), and also observed that the Cmax was increased. As shown in Figure 2, for all compounds, with the use of 2.0% by volume to 7.5% by volume of Cremophor EL, compounds with low membrane permeability were confirmed to exhibit higher absorbability (AUClast ratio of 0.7 or more) compared to the case where a Cremophor EL concentration of less than 2.0% by volume and greater than 7.5% by volume was used.

[Table 44]

| Analyte | CreEL (% by volume) | AUClast (ng·h/mL) | AUClast ratio | Cmax (ng/mL) |
|---|---|---|---|---|
| Comparative formulation 3-2-1 | 0.45 | 1620 | 0.38 | 188 |
| Comparative formulation 3-2-2 | 0.6 | 2350 | 0.56 | 292 |
| Comparative formulation 3-2-3 | 0.85 | 3530 | 0.83 | 330 |
| Comparative formulation 3-2-4 | 1.4 | 3370 | 0.80 | 439 |
| Example formulation 3-2-5 | 4 | 4230 | 1.00 | 400 |
| Example formulation 3-2-6 | 5 | 3670 | 0.87 | 378 |

[Table 45]

| Analyte | CreEL (% by volume) | AUClast (ng·h/mL) | AUClast ratio | Cmax (ng/mL) |
|---|---|---|---|---|
| Comparative formulation 3-2-7 | 0.8 | 1370 | 0.46 | 148 |
| Comparative formulation 3-2-8 | 1.4 | 2840 | 0.95 | 305 |
| Example formulation 3-2-9 | 2.5 | 2760 | 0.92 | 314 |
| Example formulation 3-2-10 | 5 | 2400 | 0.80 | 309 |
| Example formulation 3-2-11 | 7.5 | 2990 | 1.00 | 297 |
| Comparative formulation 3-2-12 | 15 | 1310 | 0.44 | 140 |

[Table 46]

| Analyte | CreEL (% by volume) | AUClast (ng·h/mL) | AUClast ratio | Cmax (ng/mL) |
|---|---|---|---|---|
| Comparative formulation 4-2-1 | 0.45 | 8620 | 0.85 | 1030 |
| Comparative formulation 4-2-2 | 0.75 | 10100 | 1.00 | 1320 |
| Example formulation 4-2-3 | 2.5 | 8460 | 0.84 | 988 |
| Example formulation 4-2-4 | 5 | 7860 | 0.78 | 1110 |
| Example formulation 4-2-5 | 7.5 | 9130 | 0.90 | 1150 |
| Comparative formulation 4-2-6 | 15 | 4260 | 0.42 | 524 |

[Table 47]

| Analyte | CreEL (% by volume) | AUClast (ng·h/mL) | AUClast ratio | Cmax (ng/mL) |
|---|---|---|---|---|
| Comparative formulation 6-2-1 | 0.12 | 1380 | 0.39 | 164 |
| Comparative formulation 6-2-2 | 0.15 | 2560 | 0.73 | 315 |
| Comparative formulation 6-2-3 | 0.2 | 1740 | 0.50 | 227 |
| Comparative formulation 6-2-4 | 0.35 | 2700 | 0.77 | 326 |
| Comparative formulation 6-2-5 | 1 | 3270 | 0.93 | 350 |
| Example formulation 6-2-6 | 5 | 3500 | 1.00 | 407 |

[Table 48]

| Analyte | CreEL (% by volume) | AUClast (ng·h/mL) | AUClast ratio | Cmax (ng/mL) |
|---|---|---|---|---|
| Comparative formulation 6-2-7 | 0.2 | 428 | 0.33 | 60.6 |
| Comparative formulation 6-2-8 | 0.35 | 535 | 0.41 | 66.8 |
| Example formulation 6-2-9 | 2.5 | 1180 | 0.91 | 162 |
| Example formulation 6-2-10 | 5 | 1290 | 1.00 | 173 |
| Example formulation 6-2-11 | 7.5 | 1280 | 0.99 | 310 |
| Comparative formulation 6-2-12 | 15 | 586 | 0.45 | 71.1 |

[Table 49]

| Analyte | CreEL (% by volume) | AUClast (ng·h/mL) | AUClast ratio | Cmax (ng/mL) |
|---|---|---|---|---|
| Comparative formulation 7-2-1 | 0.11 | 442 | 0.50 | 88.8 |
| Comparative formulation 7-2-2 | 0.15 | 327 | 0.37 | 73.1 |
| Comparative formulation 7-2-3 | 0.2 | 411 | 0.46 | 83.9 |
| Comparative formulation 7-2-4 | 0.3 | 604 | 0.68 | 145 |
| Comparative formulation 7-2-5 | 1 | 707 | 0.80 | 194 |
| Example formulation 7-2-6 | 5 | 887 | 1.00 | 177 |

[Table 50]

| Analyte | CreEL (% by volume) | AUClast (ng·h/mL) | AUClast ratio | Cmax (ng/mL) |
|---|---|---|---|---|
| Comparative formulation 7-2-7 | 0.2 | 310 | 0.31 | 68.5 |
| Comparative formulation 7-2-8 | 0.3 | 991 | 1.00 | 225 |
| Example formulation 7-2-9 | 2.5 | 734 | 0.74 | 176 |
| Example formulation 7-2-10 | 5 | 782 | 0.79 | 215 |
| Example formulation 7-2-11 | 7.5 | 472 | 0.48 | 92.8 |
| Comparative formulation 7-2-12 | 15 | 449 | 0.45 | 104 |

[Table 51]

| Analyte | CreEL (% by volume) | AUClast (ng·h/mL) | AUClast ratio | Cmax (ng/mL) |
|---|---|---|---|---|
| Comparative formulation 8-2-1 | 0.17 | 1010 | 0.42 | 144 |
| Comparative formulation 8-2-2 | 0.2 | 969 | 0.40 | 209 |
| Comparative formulation 8-2-3 | 0.3 | 883 | 0.36 | 174 |
| Comparative formulation 8-2-4 | 0.5 | 992 | 0.41 | 178 |
| Comparative formulation 8-2-5 | 1.5 | 1400 | 0.58 | 226 |
| Example formulation 8-2-6 | 5 | 2420 | 1.00 | 335 |

[Table 52]

| Analyte | CreEL (% by volume) | AUClast (ng·h/mL) | AUClast ratio | Cmax (ng/mL) |
|---|---|---|---|---|
| Comparative formulation 8-2-7 | 0.3 | 541 | 0.47 | 127 |
| Comparative formulation 8-2-8 | 0.5 | 1070 | 0.93 | 164 |
| Example formulation 8-2-9 | 2.5 | 1150 | 1.00 | 215 |
| Example formulation 8-2-10 | 5 | 927 | 0.81 | 182 |
| Example formulation 8-2-11 | 7.5 | 689 | 0.60 | 161 |
| Comparative formulation 8-2-12 | 15 | 564 | 0.49 | 86.1 |

[Table 53]

| Analyte | CreEL (% by volume) | AUClast (ng·h/mL) | AUClast ratio | Cmax (ng/mL) |
|---|---|---|---|---|
| Comparative formulation 9-2-1 | 0.11 | 307 | 0.68 | 65.5 |
| Comparative formulation 9-2-2 | 0.15 | 450 | 1.00 | 127 |
| Comparative formulation 9-2-3 | 0.2 | 314 | 0.70 | 80.3 |
| Comparative formulation 9-2-4 | 0.35 | 154 | 0.34 | 38.3 |
| Comparative formulation 9-2-5 | 1 | 276 | 0.61 | 71.7 |
| Example formulation 9-2-6 | 5 | 330 | 0.73 | 109 |

[Table 54]

| Analyte | CreEL (% by volume) | AUClast (ng·h/mL) | AUClast ratio | Cmax (ng/mL) |
|---|---|---|---|---|
| Comparative formulation 9-2-7 | 0.2 | 238 | 1.00 | 53.1 |
| Comparative formulation 9-2-8 | 0.35 | 170 | 0.71 | 34.8 |
| Example formulation 9-2-9 | 2.5 | 214 | 0.90 | 61.3 |
| Example formulation 9-2-10 | 5 | 168 | 0.71 | 41.9 |
| Example formulation 9-2-11 | 7.5 | 156 | 0.66 | 48.0 |
| Comparative formulation 9-2-12 | 15 | 83.0 | 0.35 | 17.6 |

[Table 55]

| Analyte | CreEL (% by volume) | AUClast (ng-h/mL) | AUClast ratio | Cmax (ng/mL) |
|---|---|---|---|---|
| Comparative formulation 10-2-1 | 0.03 | 106 | 0.70 | 33.2 |
| Comparative formulation 10-2-2 | 0.04 | 151 | 1.00 | 35.2 |
| Comparative formulation 10-2-3 | 0.05 | 142 | 0.94 | 34.7 |
| Comparative formulation 10-2-4 | 0.08 | 86.7 | 0.57 | 24.6 |
| Comparative formulation 10-2-5 | 0.25 | 145 | 0.96 | 34.6 |
| Example formulation 10-2-6 | 5 | 144 | 0.95 | 46.6 |

[Table 56]

| Analyte | CreEL (% by volume) | AUClast (ng·h/mL) | AUClast ratio | Cmax (ng/mL) |
|---|---|---|---|---|
| Comparative formulation 10-2-7 | 0.05 | 79.4 | 0.40 | 27.1 |
| Comparative formulation 10-2-8 | 0.08 | 92.6 | 0.47 | 23.9 |
| Example formulation 10-2-9 | 2.5 | 197 | 1.00 | 55.9 |
| Example formulation 10-2-10 | 5 | 182 | 0.92 | 49.6 |
| Example formulation 10-2-11 | 7.5 | 113 | 0.57 | 38.9 |
| Comparative formulation 10-2-12 | 15 | 82.5 | 0.42 | 21.9 |

Table 57 summarizes the proportion (%) of the number of plots corresponding to an AUClast ratio ≥ 0.7 to the total plots for each Cremophor EL concentration.

[Table 57]

| | Proportion (%) of number of plots corresponding to AUClast ratio ≥ 0.7 to total plots |
|---|---|
| CreEL < 2.0% | 42 |
| CreEL 2.0 to 7.5% | 86 |
| Cre EL > 7.5% | 0 |

As shown in Table 57, in the case where the dose to be administered is 3 mg/kg, for example, when the Cremophor EL concentration is greater than 7.5% by volume, the probability of an AUClast ratio of 0.7 or more is 0%, and when the Cremophor EL concentration is less than 2.0% by volume, the probability of an AUClast ratio of 0.7 or more is 42%, whereas when the Cremophor EL concentration is 2.0 to 7.5% by volume, the proportion of an AUClast ratio of 0.7 or higher is improved to 86%. That is, for cyclic peptide compounds, with the use of 2.0 to 7.5% by volume of Cremophor EL, compounds with low membrane permeability were confirmed to exhibit higher absorbability (AUClast ratio of 0.7 or more) compared to the case where a Cremophor EL concentration of less than 2.0% by volume and greater

than 7.5% by volume was used.

From the above results, by screening oral absorbability at a certain Cremophor EL concentration, high oral absorbability and high plasma concentration (AUC and Cmax) can be obtained. That is, it is possible to enhance the oral absorbability of compounds with low oral absorbability characteristics, which are a barrier to drug discovery, depending on their potential, thus enabling screening of compounds with excellent oral absorbability while reducing the dropout rate of compounds due to false negatives, thereby enabling selection of the optimal compound. Furthermore, by obtaining high oral absorbability and high plasma concentration, it is possible to precisely evaluate oral bioavailability, pharmacological activity during oral administration, safety during oral administration, pharmacokinetics during oral administration, and physical properties of compounds as various characteristics of compounds, which are essential for drug discovery and development, and to determine better pharmaceuticals in drug discovery and development.

## Claims

**1.** A formulation comprising:
a compound and an oily component having a polyoxyethylene structure, wherein a content of the oily component is 2.0% by volume or more and 7.5% by volume or less based on 100% by volume of the formulation.

**2.** An agent for promoting absorption of a compound, comprising:

an oily component having a polyoxyethylene structure as an active component,
wherein the agent is used such that a content of the oily component is 2.0% by volume or more and 7.5% by volume or less based on 100% by volume of a formulation containing the compound.

**3.** An agent for improving solubility of a compound, comprising:

an oily component having a polyoxyethylene structure as an active component,
wherein the agent is used such that a content of the oily component is 2.0% by volume or more and 7.5% by volume or less based on 100% by volume of a formulation containing the compound.

**4.** The formulation or agent according to any one of claims 1 to 3, wherein the oily component having a polyoxyethylene structure is at least one selected from the group consisting of polyoxyethylene castor oil, polyoxyethylene hydrogenated castor oil, and polyoxyethylene sorbitan fatty acid esters.

**5.** The formulation or agent according to any one of claims 1 to 4, wherein the oily component having a polyoxyethylene structure is polyoxyethylene castor oil.

**6.** The formulation or agent according to any one of claims 1 to 5, wherein the compound is a peptide compound.

**7.** The formulation or agent according to any one of claims 1 to 6, wherein the compound is a peptide compound containing 5 or more and 30 or less amino acid residues.

**8.** The formulation or agent according to claim 6 or 7, wherein the peptide compound is a peptide compound having a cyclic moiety.

**9.** The formulation or agent according to any one of claims 1 to 8, wherein the formulation is an oral formulation.

**10.** The formulation or agent according to any one of claims 1 to 9, wherein the formulation is for screening a candidate compound.

**11.** A method for screening a candidate compound, comprising:
administering to a subject the formulation according to any one of claims 1 and 4 to 10.

**12.** The method according to claim 11, comprising selecting the candidate compound using at least one selected from the group consisting of the following (1), (2), and (3) as an indicator:

(1) drug efficacy;
(2) toxicity; and

(3) pharmacokinetic characteristics.

**13.** The method according to claim 11 or 12, wherein the candidate compound is selected from multiple test compounds.

**14.** The method according to any one of claims 11 to 13, wherein the subject is a non-human animal.

**15.** The method according to any one of claims 11 to 14, wherein a dose of the test compounds to be administered is 0.1 mg/kg or more and 1000 mg/kg or less.

*Fig.1*

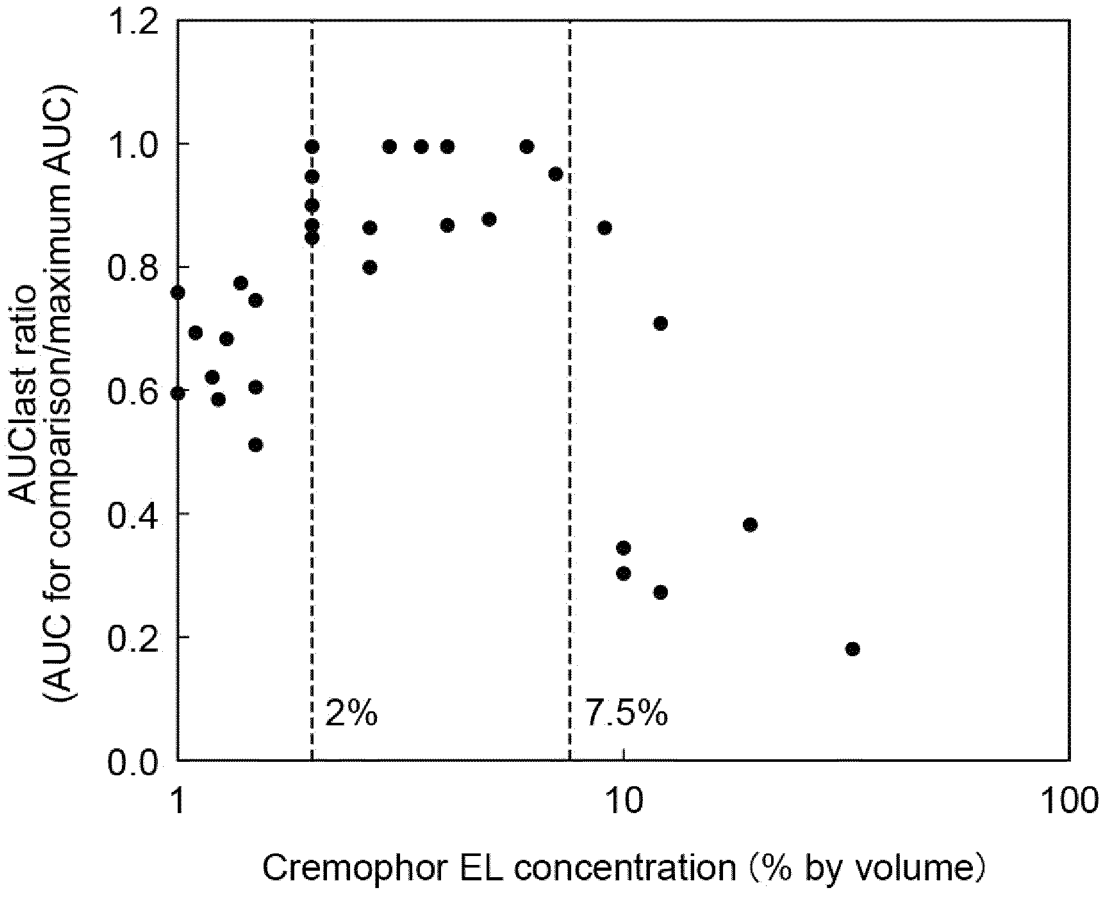
AUClast ratio
(AUC for comparison/maximum AUC)
1.2
1.0
0.8
0.6
0.4
0.2
0.0
2%
7.5%
1
10
100
Cremophor EL concentration (% by volume)

# Fig.2

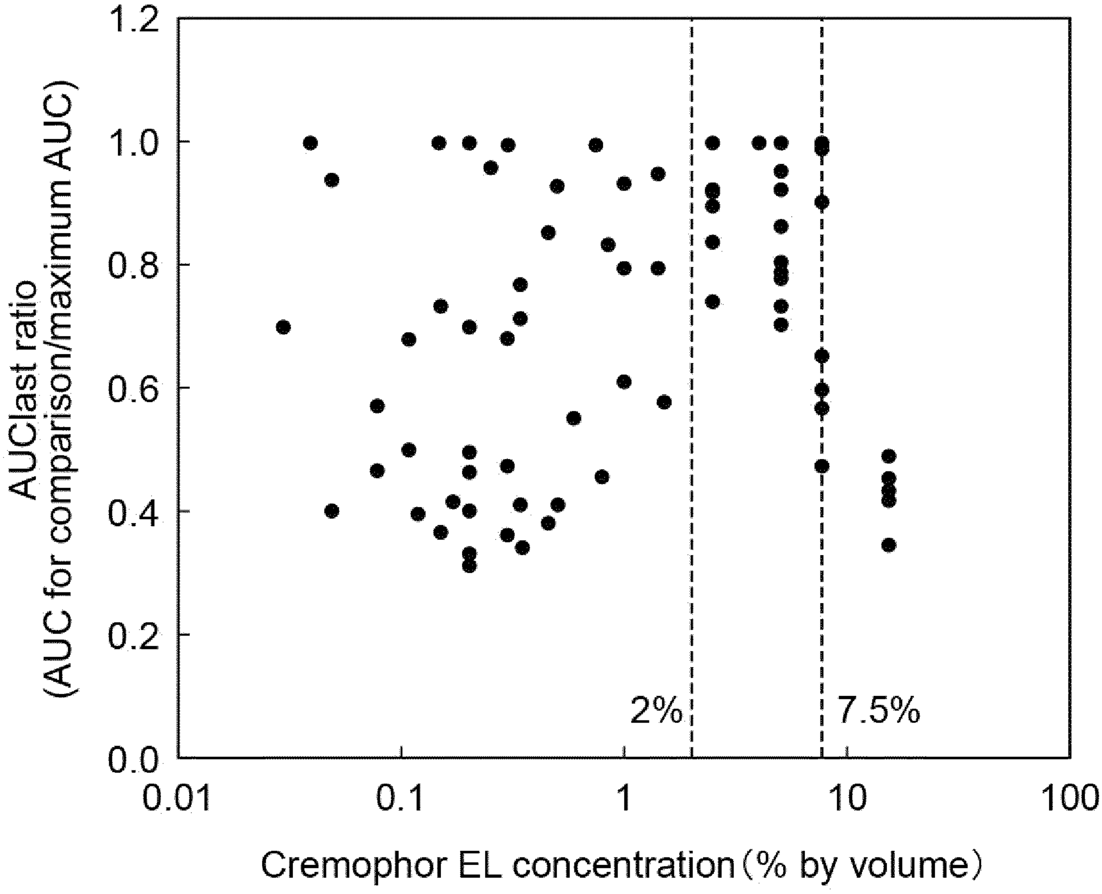
1.2
1.0
0.8
0.6
0.4
0.2
0.0
AUClast ratio
(AUC for comparison/maximum AUC)
2%
7.5%
0.01
0.1
1
10
100
Cremophor EL concentration (% by volume)

**INTERNATIONAL SEARCH REPORT**

International application No. **PCT/JP2023/012087**

**A. CLASSIFICATION OF SUBJECT MATTER**

***A61K 47/34***(2017.01)i; ***A61K 47/44***(2017.01)i; ***A61K 38/08***(2019.01)i; ***G01N 33/15***(2006.01)i; ***G01N 33/50***(2006.01)i
FI: A61K47/34; A61K38/08; A61K47/44; G01N33/15 Z; G01N33/50 Z

According to International Patent Classification (IPC) or to both national classification and IPC

**B. FIELDS SEARCHED**

Minimum documentation searched (classification system followed by classification symbols)

A61K47/34; A61K47/44; A61K38/08; G01N33/15; G01N33/50

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2021/090855 A1 (CHUGAI PHARMACEUTICAL CO LTD) 14 May 2021 (2021-05-14) claims, claim 1, paragraphs [0001], [0003]-[0004], [0343], examples 1-2, compound 558 | 1-15 |
| Y | TAKEUCHI, Koh et al. Conformational Plasticity of Cyclic Ras-Inhibitor Peptides Defines Cell Permeabilization Activity. Angew. Chem. Int. Ed. 2021, vol. 60, pp. 6567-6572 columns "abstract", "Introduction", 1st paragraph | 1-15 |
| Y | JP 2001-515491 A (ABBOTT LABORATORIES) 18 September 2001 (2001-09-18) claims, pp. 9-10, examples 3, 13, 25, table 2 | 1-15 |
| X | | 1-9 |
| Y | JP 11-505257 A (ABBOTT LABORATORIES) 18 May 1999 (1999-05-18) claims, p. 6, lines 6-19, examples 1-4 | 1-15 |
| X | | 1-9 |
| Y | JP 2021-503494 A (ICURE BNP CO LTD) 12 February 2021 (2021-02-12) paragraph [0054] | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 June 2023** | **27 June 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/012087**

**C. DOCUMENTS CONSIDERED TO BE RELEVANT**

| Category* | Citation of document, with indication, where appropriate, of the relevant passages | Relevant to claim No. |
|---|---|---|
| Y<br>X | JP 2015-514800 A (UNIV YONSEI IACF) 21 May 2015 (2015-05-21)<br>paragraph [0009], examples 1-4 | 1-15<br>1-8 |
| X | WO 2021/049825 A1 (TAEJOON PHARMACEUTICAL CO., LTD.) 18 March 2021 (2021-03-18)<br>example 6 | 1-8 |
| A | WO 2018/225864 A1 (CHUGAI PHARMACEUTICAL CO LTD) 13 December 2018 (2018-12-13)<br>entire text | 1-15 |
| A | JP 2021-169476 A (CHUGAI PHARMACEUTICAL CO LTD) 28 October 2021 (2021-10-28)<br>entire text | 1-15 |

Form PCT/ISA/210 (second sheet) (January 2015)

**INTERNATIONAL SEARCH REPORT**
**Information on patent family members**

International application No.
**PCT/JP2023/012087**

| Patent document cited in search report | Publication date (day/month/year) | Patent family member(s) | Publication date (day/month/year) |
|---|---|---|---|
| WO 2021/090855 A1 | 14 May 2021 | EP 4043478 A1<br>claims, paragraphs [0001], [0003]-[0004] , [0366], examples 1-2, compound no. 558<br>KR 10-2021-0064234 A<br>CN 114729006 A | |
| JP 2001-515491 A | 18 September 2001 | WO 1998/040051 A1<br>claims, pp. 4-5, examples 3, 13, 25, table 2 | |
| JP 11-505257 A | 18 May 1999 | WO 1996/036316 A1<br>claims, p. 2, lines 27-33, examples 1-4 | |
| JP 2021-503494 A | 12 February 2021 | US 2021/0170036 A1<br>paragraph [0064]<br>EP 3725297 A1<br>KR 10-2019-0070653 A<br>CN 111356445 A | |
| JP 2015-514800 A | 21 May 2015 | US 2015/0125494 A1<br>paragraph [0009], examples 1-4<br>EP 2845602 A1<br>KR 10-1211902 B1<br>CN 104302308 A | |
| WO 2021/049825 A1 | 18 March 2021 | JP 2022-548221 A<br>example 6<br>EP 4029493 A1<br>example 6 | |
| WO 2018/225864 A1 | 13 December 2018 | US 2020/0131669 A1<br>whole document<br>EP 3636807 A1<br>CN 110869544 A | |
| JP 2021-169476 A | 28 October 2021 | US 2019/0380958 A1<br>whole document<br>EP 3563833 A1<br>CN 110869544 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2018225864 A


### Non-patent literature cited in the description


- **DONOVAN, M. D. et al.** *Pharm. Res.,* 1990, vol. 7, 863-868
- **LIPINSKI, C. A.** *Adv. Drug Del. Rev.,* 1997, vol. 23, 3-25
- **GHADI, R. et al.** *J. Control. Release,* 2017, vol. 248, 71-95
- *CHEMICAL ABSTRACTS,* 146982-24-3
- *Letters in Peptide Science,* 2002, vol. 9, 203
- *CHEMICAL ABSTRACTS,* 30924-93-7
- *CHEMICAL ABSTRACTS,* 72914-19-3
- *CHEMICAL ABSTRACTS,* 467435-07-0
- *CHEMICAL ABSTRACTS,* 156243-64-0